# EUROPEAN PATENT SPECIFICATION

(11) **EP 2 526 898 B1**
(45) Date of publication and mention of the grant of the patent: **17.04.2013**
(21) Application number: 12179049.7
(22) Date of filing: 22.12.2004
(51) Int. Cl.: A61F 2/24, A61F 2/01

(54) **Repositionable heart valve**
Umpositionierbares Herzventil
Valvule cardiaque repositionnable

(30) Priority: 23.12.2003 US 746280; 23.12.2003 US 746942; 23.12.2003 US 746240; 23.12.2003 US 746872; 23.12.2003 US 746887; 23.12.2003 US 746120; 23.12.2003 US 746285; 15.07.2004 US 893151; 15.07.2004 US 893131; 15.07.2004 US 893143; 15.07.2004 US 893142; 21.10.2004 US 972287; 21.10.2004 US 971535; 05.11.2004 US 982692; 05.11.2004 US 982388
(43) Date of publication of application: 28.11.2012
(62) Divisional of application: 04815634.3
(73) Proprietor: Sadra Medical, Inc., Campbell, CA 95008 (US)
(72) Inventor: Salahieh, Amr, Saratoga, CA California 95070 (US); Brandt, Brian, D., San Jose, CA California 95119 (US); Morejohn, Dwight, P., Davis, CA California 95616 (US); Haug, Ulrich, R., Campbell, CA California 95008 (US); Dueri, Jean-Pierre, Stockton, CA California 95219 (US); Valencia, Hans, F., San Jose, CA California 95125 (US); Geshlider, Robert, A., San Francisco, CA California 94131 (US); Krolik, Jeff, Campbell, CA California 95008 (US); Saul, Tom, Moss Beach, CA California 94038 (US); Argento, Claudio, Los Gatos, CA California 95033 (US); Hildebrand, Daniel, Menlo Park, CA California 94025 (US)
(74) Representative: Peterreins, Frank

(56) References cited:
- US-A1- 2002 032 481

## Description

### BACKGROUND OF THE INVENTION

The present invention relates to methods and apparatus for endovascularly replacing a heart valve. More particularly, the present invention relates to methods and apparatus for percutaneously replacing a heart valve with a replacement valve using an expandable and retrievable anchor.

Heart valve surgery is used to repair or replace diseased heart valves. Valve surgery is an open-heart procedure conducted under general anesthesia. An incision is made through the patient's sternum (sternotomy), and the patient's heart is stopped while blood flow is rerouted through a heart-lung bypass machine.

Valve replacement may be indicated when there is a narrowing of the native heart valve, commonly referred to as stenosis, or when the native valve leaks or regurgitates.

When replacing the valve, the native valve is excised and replaced with either a biologic or a mechanical valve. Mechanical valves require lifelong anticoagulant medication to prevent blood clot formation, and clicking of the valve often may be heard through the chest. Biologic tissue valves typically do not require such medication. Tissue valves may be obtained from cadavers or may be porcine or bovine, and are commonly attached to synthetic rings that are secured to the patient's heart.

Valve replacement surgery is a highly invasive operation with significant concomitant risk. Risks include bleeding, infection, stroke, heart attack, arrhythmia, renal failure, adverse reactions to the anesthesia medications, as well as sudden death. 2-5% of patients die during surgery.

Post-surgery, patients temporarily may be confused due to emboli and other factors associated with the heart-lung machine. The first 2-3 days following surgery are spent in an intensive care unit where heart functions can be closely monitored. The average hospital stay is between 1 to 2 weeks, with several more weeks to months required for complete recovery.

In recent years, advancements in minimally invasive surgery and interventional cardiology have encouraged some investigators to pursue percutaneous replacement of the aortic heart valve. Percutaneous Valve Technologies ("PVT") of Fort Lee, New Jersey, has developed a balloon-expandable stent integrated with a bioprosthetic valve. The stent/valve device is deployed across the native diseased valve to permanently hold the valve open, thereby alleviating a need to excise the native valve and to position the bioprosthetic valve in place of the native valve. PVT's device is designed for delivery in a cardiac catheterization laboratory under local anesthesia using fluoroscopic guidance, thereby avoiding general anesthesia and open-heart surgery. The device was first implanted in a patient in April of 2002.

PVT's device suffers from several drawbacks. Deployment of PVT's stent is not reversible, and the stent is not retrievable. This is a critical drawback because improper positioning too far up towards the aorta risks blocking the coronary ostia of the patient. Furthermore, a misplaced stent/valve in the other direction (away from the aorta, closer to the ventricle) will impinge on the mitral apparatus and eventually wear through the leaflet as the leaflet continuously rubs against the edge of the stent/valve.

Another drawback of the PVT device is its relatively large cross-sectional delivery profile. The PVT system's stent/valve combination is mounted onto a delivery balloon, making retrograde delivery through the aorta challenging. An antegrade transseptal approach may therefore be needed, requiring puncture of the septum and routing through the mitral valve, which significantly increases complexity and risk of the procedure. Very few cardiologists are currently trained in performing a transseptal puncture, which is a challenging procedure by itself.

Other prior art replacement heart valves use self-expanding stents as anchors. In the endovascular aortic valve replacement procedure, accurate placement of aortic valves relative to coronary ostia and the mitral valve is critical. Standard self-expanding systems have very poor accuracy in deployment, however. Often the proximal end of the stent is not released from the delivery system until accurate placement is verified by fluoroscopy, and the stent typically jumps once released. It is therefore often impossible to know where the ends of the stent will be with respect to the native valve, the coronary ostia and the mitral valve.

Also, visualization of the way the new valve is functioning prior to final deployment is very desirable. Visualization prior to final and irreversible deployment cannot be done with standard self-expanding systems, however, and the replacement valve is often not fully functional before final deployment.

Another drawback of prior art self-expanding replacement heart valve systems is their lack of radial strength. In order for self-expanding systems to be easily delivered through a delivery sheath, the metal needs to flex and bend inside the delivery catheter without being plastically deformed. In arterial stents, this is not a challenge, and there are many commercial arterial stent systems that apply adequate radial force against the vessel wall and yet can collapse to a small enough of a diameter to fit inside a delivery catheter without plastically deforming.

However when the stent has a valve fastened inside it, as is the case in aortic valve replacement, the anchoring of the stent to vessel walls is significantly challenged during diastole. The force to hold back arterial pressure and prevent blood from going back inside the ventricle during diastole will be directly transferred to the stent/vessel wall interface. Therefore the amount of radial force required to keep the self expanding stent/valve in contact with the vessel wall and not sliding will be much higher than in stents that do not have valves inside of them. Moreover, a self-expanding stent without sufficient radial force will end up dilating and contracting with each heartbeat, thereby distorting the valve, affecting its function and possibly migrating and dislodging completely. Simply increasing strut thickness of the self-expanding stent is not a practical solution as it runs the risk of larger profile and/or plastic deformation of the self-expanding stent.

U.S. patent application Serial No. 2002/0151970 to Garrison et al. describes a two-piece device for replacement of the aortic valve that is adapted for delivery through a patient's aorta. A stent is percutaneously placed across the native valve, then a replacement valve is positioned within the lumen of the stent. By separating the stent and the valve during delivery, a profile of the device's delivery system may be sufficiently reduced to allow aortic delivery without requiring a transseptal approach. Both the stent and a frame of the replacement valve may be balloon-expandable or self-expanding.

While providing for an aortic approach, devices described in the Garrison patent application suffer from several drawbacks. First, the stent portion of the device is delivered across the native valve as a single piece in a single step, which precludes dynamic repositioning of the stent during delivery. Stent foreshortening or migration during expansion may lead to improper alignment.

Additionally, Garrison's stent simply crushes the native valve leaflets against the heart wall and does not engage the leaflets in a manner that would provide positive registration of the device relative to the native position of the valve. This increases an immediate risk of blocking the coronary ostia, as well as a longer-term risk of migration of the device post-implantation. FurtherstiU, the stent comprises openings or gaps in which the replacement valve is seated post-delivery. Tissue may protrude through these gaps, thereby increasing a risk of improper seating of the valve within the stent.

US 2002/032481 discloses an apparatus for replacing a native aortic valve, comprising an anchor with an expandable braid with closed ends, and a replacement valve configured to be secured within the anchor.

In view of drawbacks associated with previously known techniques for percutaneously replacing a heart valve, it would be desirable to provide methods and apparatus that overcome those drawbacks.

### SUMMARY OF THE INVENTION

The present invention provides an apparatus for replacing a native aortic valve as set forth in the claims.

The apparatus comprises an anchor comprising an expandable braid with closed ends and a replacement valve configured to be secured within the anchor. The anchor is adapted to be endovascularly delivered and secured at an anchor site within the native aortic valve. The anchor includes a proximal deployment system interface at a proximal end of the anchor, the proximal deployment system interface being adapted to permit a deployment system to apply a distally directed force on the proximal end of the anchor. The proximal deployment system interface is further adapted to expand radially during application of a distally directed force on the proximal end of the anchor.

The proximal deployment system interface may further be adapted to permit a deployment system to apply a distally directed force on the proximal end of the anchor through a plurality of deployment system fingers. The apparatus may include a distal deployment system interface disposed at a distal end of the anchor, the distal deployment system interface being adapted to permit a deployment system to apply a proximally directed force on the distal end of the anchor. The distal deployment system interface may further be adapted to expand radially during application of a proximally directed force on the distal end of the anchor. The distal deployment system interface may further be adapted to permit a deployment system to apply a proximally directed force on the distal end of the anchor without passing any portion of a deployment system through a center opening of the replacement valve. The delivery length of the apparatus can be between about 15 mm and about 150 mm and the deployed length can be between 5 mm and about 40 mm.

The present invention also provides a deployment system and an apparatus for replacing a native aortic valve, the apparatus comprising the features described above.

### BRIEF DESCRIPTION OF THE DRAWINGS

Figures 1 A-B are elevational views of a replacement heart valve and anchor according to one embodiment of the invention.
Figures 2 A-B are sectional views of the anchor and valve of Figures 1.
Figures 3 A-B show delivery and deployment of a replacement heart valve and anchor, such as the anchor and valve of Figures 1 and 2.
Figures 4A-F also show delivery and deployment of a replacement heart valve and
anchor, such as the anchor and valve of Figures 1 and 2.
Figures 5 A-I show the use of a replacement heart valve and anchor to replace an aortic valve.
Figures 6A-F show the use of a replacement heart valve and anchor with a positive registration feature to replace an aortic valve.
Figure 7 shows the the use of a replacement heart valve and anchor with an alternative positive registration feature to replace an aortic valve.
Figures 8 A-C show another embodiment of a replacement heart valve and anchor according to the invention.
Figures 9A-H show delivery and deployment of the replacement heart valve and anchor of Figures 8.
Figure 10 is a cross-sectional drawing of the delivery system used with the method and apparatus of Figures 8 and 9.
Figures 11 A-C show alternative locks for use with replacement heart valves and anchors of this invention.
Figures 12 A-C show a vessel wall engaging lock for use with replacement heart valves and anchors of this invention.
Figures 13 A-B show an alternative anchor lock embodiment in an unlocked configuration.
Figures 14 A-B show the anchor lock of Figure 13 in a locked configuration.
Figure 15 shows an alternative anchor deployment tool attachment and release mechanism for use with the invention.
Figure 16 shows the attachment and release mechanism of Figure 15 in the process of being released.
Figure 17 shows the attachment and release mechanism of Figures 15 and 16 in a released condition.
Figure 18 shows an alternative embodiment of a replacement heart valve and anchor and a deployment tool according to the invention in an undeployed configuration.
Figure 19 shows the replacement heart valve and anchor of Figure 18 in a partially deployed configuration.
Figure 20 shows the replacement heart valve and anchor of Figures 18 and 19 in a more fully deployed configuration but with the deployment tool still attached.
Figure 21 shows yet another embodiment of the delivery and deployment apparatus of the invention in use with a replacement heart valve and anchor.
Figure 22 shows the delivery and deployment apparatus of Figure 21 in the process of deploying a replacement heart valve and anchor.
Figures 23A-H show yet another embodiment of a replacement heart valve, anchor and deployment system according to this invention.
Figures 24A-E show more detail of the anchor of the embodiment shown in Figures 23A-H.
Figures 25A-B show further details of the embodiment of Figures 23A-H.
Figures 26 A-C illustrate a method for percutaneously replacing a patient's diseased heart valve.
Figures 27A and 27B show replacement valve apparatus in accordance with the present invention. Figure 27A illustrates the apparatus in a collapsed delivery configuration within a delivery system. Figure 27B illustrates the apparatus in an expanded configuration partially deployed from the delivery system.
Figures 28A-28F show an anchor of the apparatus of Figures 27 in the collapsed delivery configuration and the expanded deployed configuration, as well as the full apparatus in the deployed configuration, and optional locking mechanisms for use with the apparatus.
Figure 29 shows a detail view of a variation of an anchor post.
Figures 30A and 30B show an alternative variation of the post having a lock alignment feature.
Figures 31A and 31B show a variation of the post having an alternative lock alignment feature.
Figure 32 shows a variation of the post having an expansile element.
Figure 33 shows a variation of the post with an alternative expansile or cable element.
Figures 34A-34C show a variation of the post having an alternative lock alignment feature.
Figure 35 shows the post variation of Figure 29 in combination with an illustrative actuator and release actuator.
Figures 36A-36C show a variation of the post, actuator and release actuator that form an alternative releasable attachment mechanism.
Figures 37A-37C show another variation of the releasable attachment mechanism.
Figures 38A-38C show yet another variation of the releasable attachment mechanism.
Figures 39 A and 39 B show still another variation of the releasable attachment element.
Figure 40 shows a variation of the post, actuator and anchor lock element having a reversible lock.
Figures 41A-41C show a variation of the actuator, lock actuator and release actuator.
Figure 42 shows a variation of the anchor lock element having a lock alignment feature.
Figures 43 A and 43B show expansion, locking and actuation of the releasable attachment mechanism of the apparatus of Figure 42.
Figure 44 shows another variation of the apparatus having an actuable lock prevention mechanism.
Figures 45 A and 45B show a variation of the post that is configured to lock against the braid of the anchor.
Figures 46A-46C show actuation and release of a variation of the anchor lock element.
Figures 47A and 47B show another variation of a releasable actuation mechanism having a lock alignment mechanism which can be cut from a tube.
Figures 48A-48D show actuation of a variation of the anchor lock element that may be formed from a cut tube.
Figures 49A-49F show a variation of the post having an unlock actuator.
Figures 50 A and 50B show another buckle variation of the anchor lock element.
Figure 51 shows attachment of a variation of the anchor lock element to the anchor.
Figure 52 shows a variation of the post and anchor lock element having a ratcheting lock.
Figures 53 A and 53B show variations of the ratcheting lock.
Figures 54A-54H show actuation of another variation of the ratcheting lock.
Figures 55A-55C show a tubular variation of the ratcheting lock element.
Figures 56A-56C show a variation of the anchor lock element of Figures 55.
Figures 57 A and 57B show a variation of the apparatus of Figures 56 comprising a lock alignment feature.
Figures 58A-58F show a method of actuating and adjusting the ratcheting lock of the apparatus of Figures 56.
Figures 59 A and 59 B show a variation of an anchor/actuator.
Figures 60A-60C show detail views of the releasable attachment mechanism of the actuator of Figures 59.
Figures 61A-61C show a variation of the releasable attachment mechanism of Figures 60.
Figures 62A-62C show another variation of the releasable attachment mechanism.
Figures 63A-63C show yet another variation of the releasable attachment mechanism.
Figures 64A-64N show variations of a release actuator used in conjunction with the releasable attachment mechanism of Figures 60.
Figures 65A and 65B show detail views of an embodiment of the delivery system/deployment tool.
Figures 66A and 66B show the delivery system/deployment tool of Figures 65 releaseably attached to apparatus 10, and detached from the apparatus.
Figures 67 A and 67B show a variation of the delivery system deployment tool of

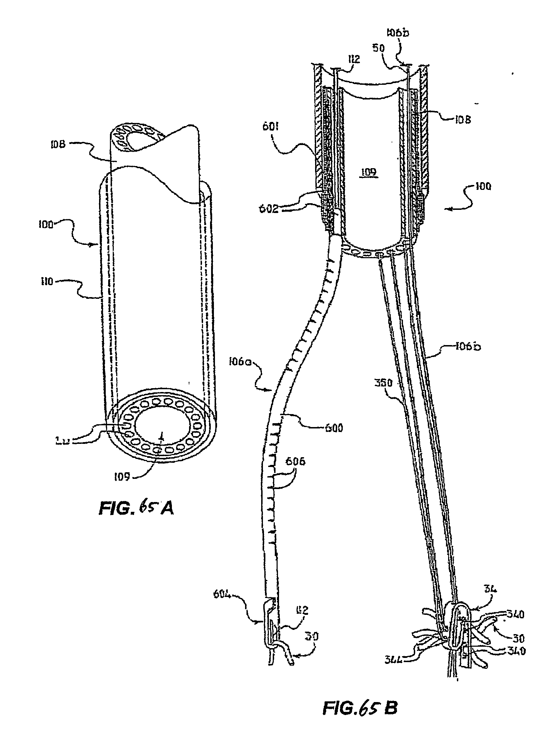

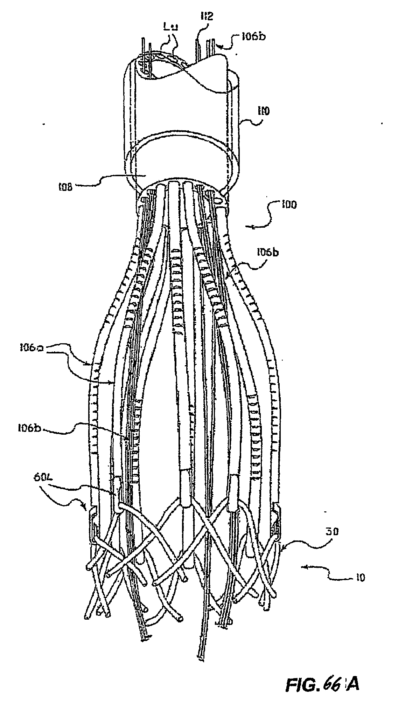

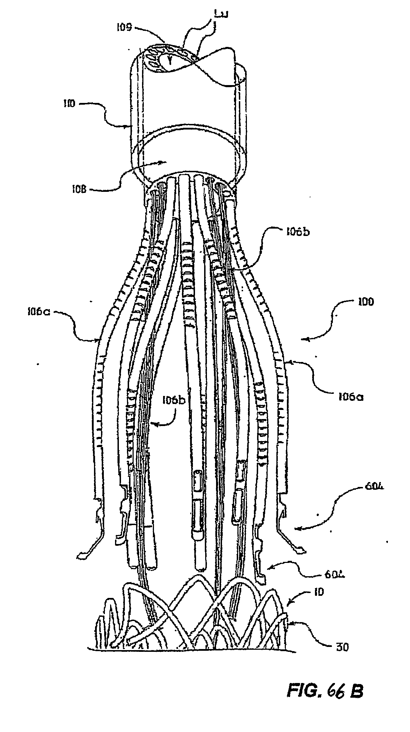

Figures 65 and 66 wherein the actuators extend from a unitary structure.

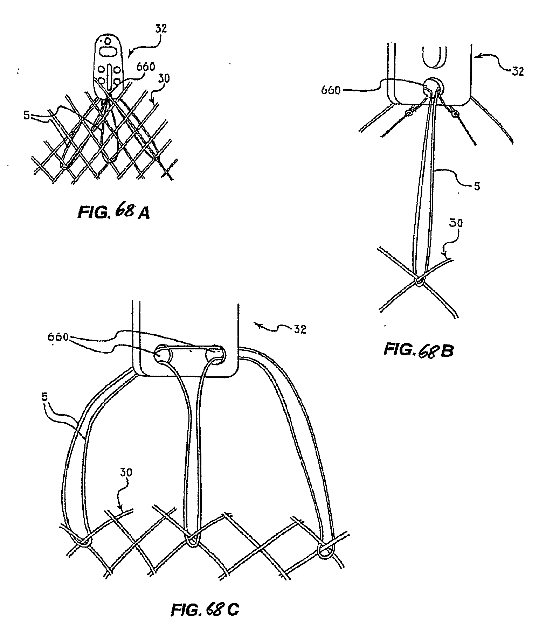

Figures 68A-68C show various ways to connect elements to the anchor of the replacement valve apparatus.

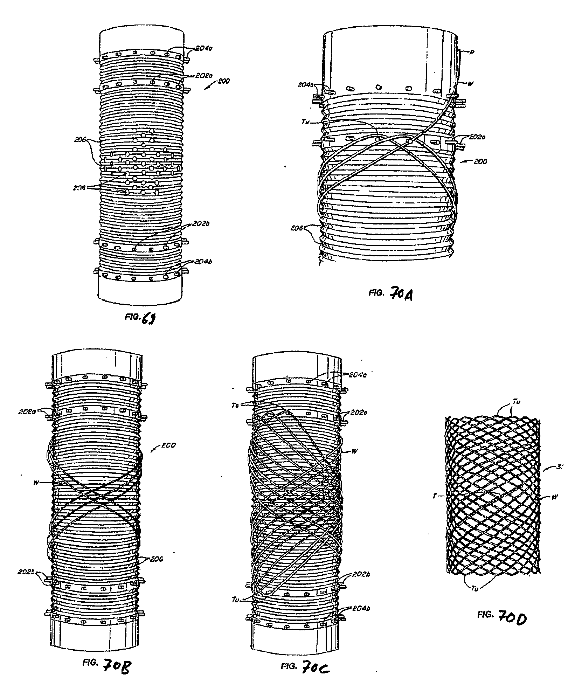

Figure 69 is a schematic top view of an apparatus for fabricating braided anchors in accordance with the present invention.
Figures 70A-70D are schematic top views illustrating a method of using the apparatus of Figure 91 to fabricate a braided anchor of the present invention.

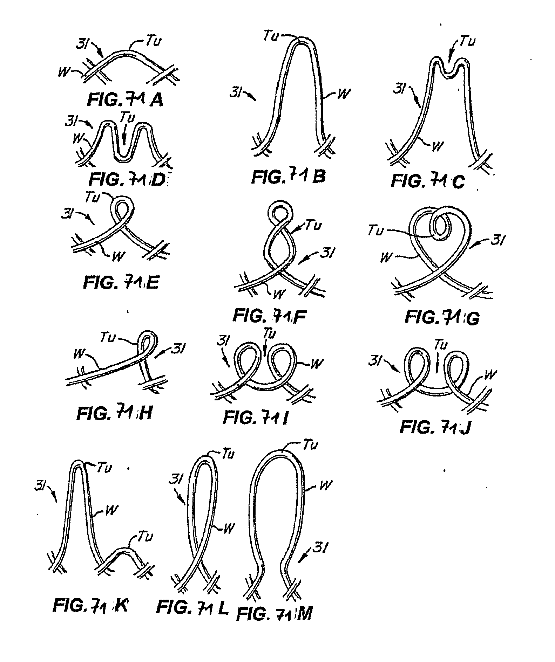

Figures 71A-71O are schematic detail views illustrating features of braid cells at an anchor edge.

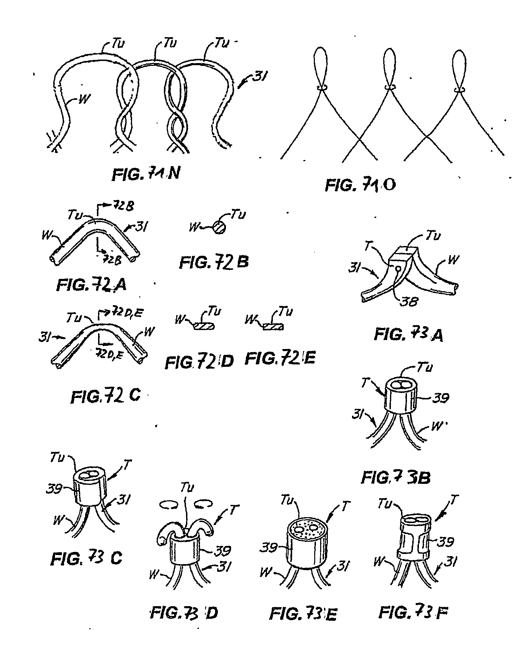

Figures 72A-72E illustrate further features of braid cells at an anchor edge.

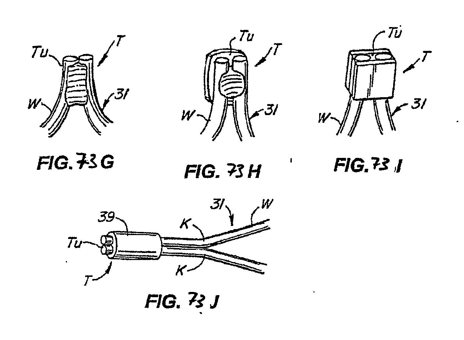

Figures 73A-73 J are schematic detail views terminations for one or more wire strands forming anchors of the present invention.

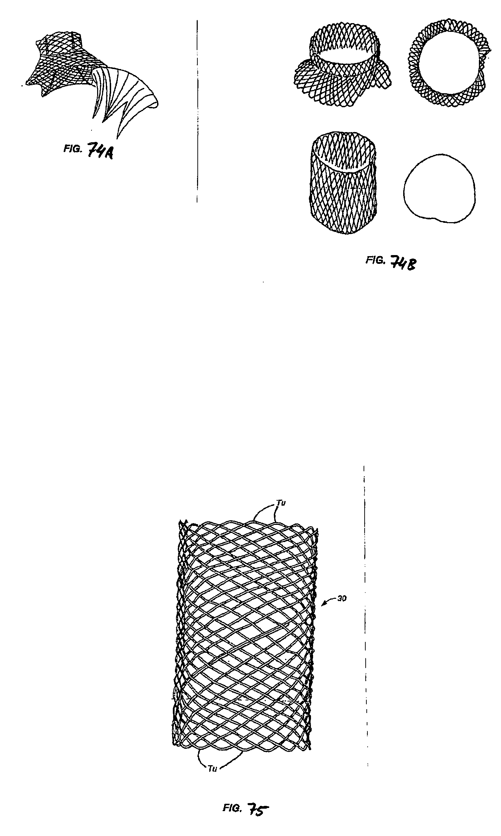

Figures 74A and 74B illustrate a bilaterally symmetrical anchor and an asymmetric anchor, respectively.
Figure 75 illustrates a braided anchor of the present invention with closed end turns Tu.

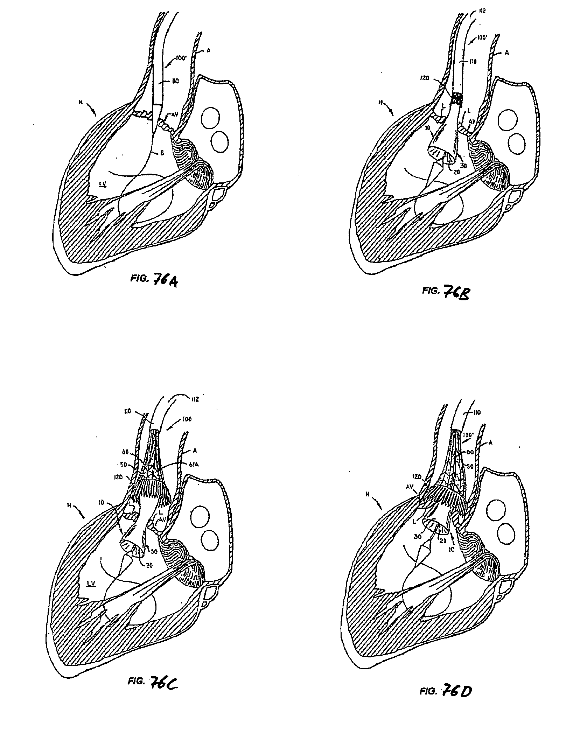

Figures 76A-76F illustrate deployment of an anchor with leaflet engagement elements on the deployment system.

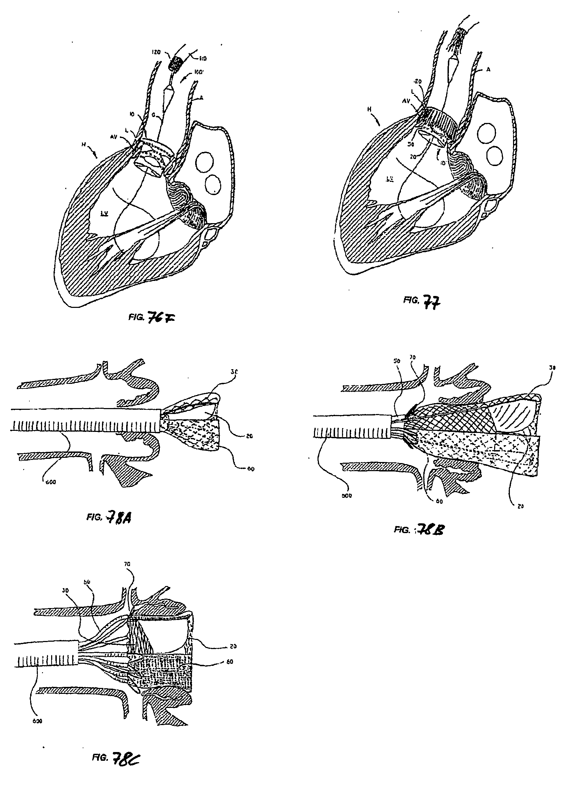

Figure 77 illustrates a deployed anchor with leaflet engagement elements on the proximal end of the anchor.
Figures 78A-78C illustrate deployment of an anchor with anchor registration elements and a seal.

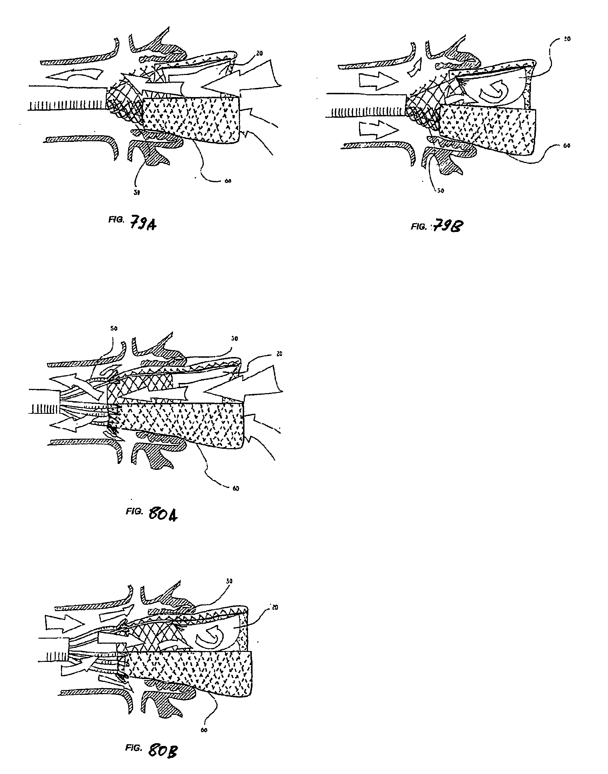

Figures 79A-79B illustrate an embodiment of the apparatus with a seal that does not reach the proximal end of the anchor during both systole and diastole.
Figures 80A-80B illustrate an embodiment of the apparatus with a seal that reaches the proximal end of the anchor during both systole and diastole.

### DETAILED DESCRIPTION

The present invention relates to apparatus and methods for endovascularly or percutaneously delivering and deploying a prosthesis, e.g., an aortic prosthesis, within and/or across a patient's native heart valve, referred to hereinafter as replacing the patient's heart valve. A delivery system and/or deployment tool is provided including a sheath assembly and a guidewire for placing the prosthetic apparatus endovascularly within the patient and a user control allowing manipulation of the prosthetic apparatus from external to the patient through the application of a non-hydraulically expanding or non-pneumatically expanding force on the anchor. A hydraulically or pneumatically expanding force would be, for example, a force applied to the anchor by a balloon expanded within the anchor. In certain embodiments, the application of a non-hydraulically expanding or non-pneumatically expanding force could include the use of a hydraulic component transmitting a proximally or distally directed force on an anchor.

The apparatus includes an anchor and a replacement valve. The anchor includes an expandable braid. The expandable braid includes closed ends. The replacement valve is adapted to be secured within the anchor, and as such, be delivered endovascularly to the patient's heart to replace one of the patient's native heart valves. More preferably, the apparatus and methods of the present invention contemplate replacement of the patient's aortic valve.

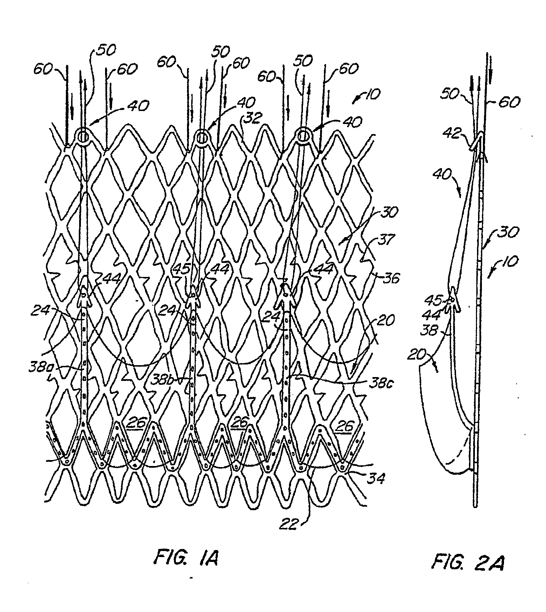

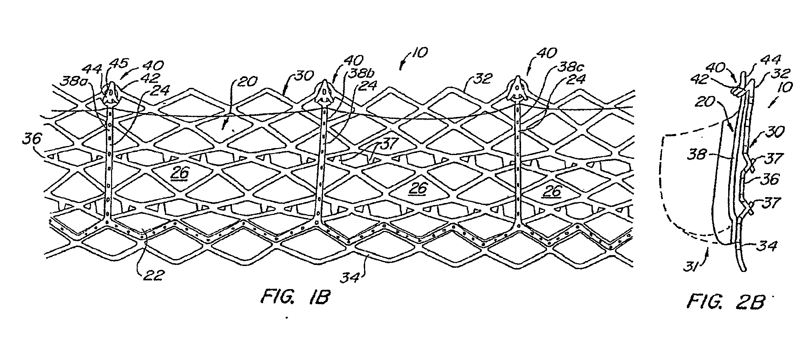

With reference now to Figures 1-4, a first embodiment of replacement heart valve apparatus in accordance with the present invention is described, including a method of actively foreshortening and expanding the apparatus from a delivery configuration and to a deployed configuration. Apparatus 10 comprises replacement valve 20 disposed within and coupled to anchor 30. Figures 1 schematically illustrate individual cells of anchor 30 of apparatus 10, and should be viewed as if the cylindrical anchor has been cut open and laid flat. Figures 2 schematically illustrate a detail portion of apparatus 10 in side-section.

Anchor 30 has a lip region 32, a skirt region 34 and a body region 36. First, second and third posts 38a, 38b and 38c, respectively, are coupled to skirt region 34 and extend within lumen 31 of anchor 30. Posts 38 preferably are spaced 120° apart from one another about the circumference of anchor 30.

Anchor 30 preferably is fabricated by using self-expanding patterns (laser cut or chemically milled), braids, and materials, such as a stainless steel, nickel-titanium ("Nitinol") or cobalt chromium but alternatively may be fabricated using balloon-expandable patterns where the anchor is designed to plastically deform to it's final shape by means of balloon expansion. Replacement valve 20 is preferably from biologic tissues, e.g. porcine valve leaflets or bovine or equine pericardium tissues, alternatively it can be made from tissue engineered materials (such as extracellular matrix material from Small Intestinal Submucosa (SIS)) but alternatively may be prosthetic from an elastomeric polymer or silicone, Nitinol or stainless steel mesh or pattern (sputtered, chemically milled or laser cut). The leaflet may also be made of a composite of the elastomeric or silicone materials and metal alloys or other fibers such Kevlar or carbon. Annular base 22 of replacement valve 20 preferably is coupled to skirt region 34 of anchor 30, while commissures 24 of replacement valve leaflets 26 are coupled to posts 38.

Anchor 30 may be actuated using external non-hydraulic or non-pneumatic force to actively foreshorten in order to increase its radial strength. As shown below, the proximal and distal end regions of anchor 30 may be actuated independently. The anchor and valve may be placed and expanded in order to visualize their location with respect to the native valve and other anatomical features and to visualize operation of the valve. The anchor and valve may thereafter be repositioned and even retrieved into the delivery sheath or catheter. The apparatus may be delivered to the vicinity of the patient's aortic valve in a retrograde approach in a catheter having a diameter no more than 23 french, preferably no more than 21 french, more preferably no more than 19 french, or more preferably no more than 17 french. Upon deployment the anchor and replacement valve capture the native valve leaflets and positively lock to maintain configuration and position.

A deployment tool is used to actuate, reposition, lock and/or retrieve anchor 30. In order to avoid delivery of anchor 30 on a balloon for balloon expansion, a non-hydraulic or non-pneumatic anchor actuator is used. In this embodiment, the actuator is a deployment tool that includes distal region control actuators 50, control actuators 60 (embodied here as rods or tubes) and proximal region control actuators 62. Locks 40 include posts or arms 38 preferably with male interlocking elements 44 extending from skirt region 34 and mating female interlocking elements 42 in lip region 32. Male interlocking elements 44 have eyelets 45. Control actuators 50 pass from a delivery system for apparatus 10 through female interlocking elements 42, through eyelets 45 of male interlocking elements 44, and back through female interlocking elements 42, such that a double strand of wire 50 passes through each female interlocking element 42 for manipulation by a medical practitioner external to the patient to actuate and control the anchor by changing the anchor's shape. Control actuators 50 may comprise, for example, strands of suture or wire.

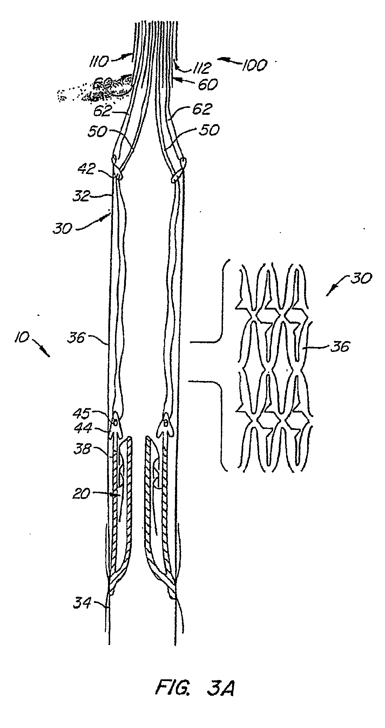

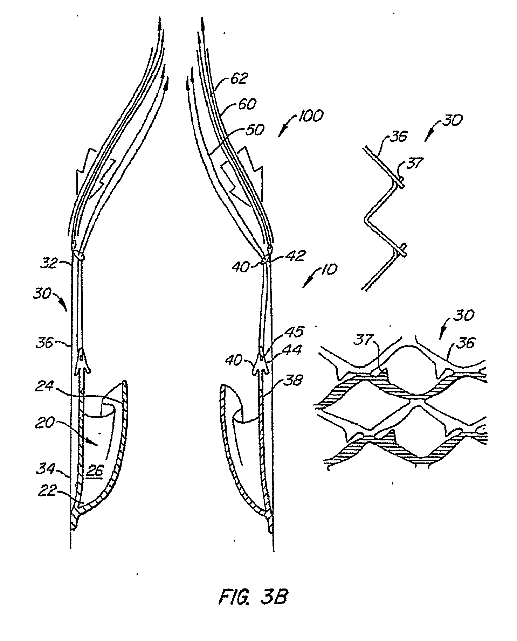

Actuators 60 are reversibly coupled to apparatus 10 and may be used in conjunction with actuators 50 to actuate anchor 30, e.g., to foreshorten and lock apparatus 10 in the fully deployed configuration. Actuators 60 also facilitate repositioning and retrieval of apparatus 10, as described hereinafter. For example, anchor 30 may be foreshortened and radially expanded by applying a distally directed force on actuators 60 while proximally retracting actuators 50. As seen in Figures 3, control actuators 62 pass through interior lumens 61 of actuators 60. This ensures that actuators 60 are aligned properly with apparatus 10 during deployment and foreshortening. Control actuators 62 can also actuate anchor 60; proximally directed forces on control actuators 62 contacts the proximal lip region 32 of anchor 30. Actuators 62 also act to couple and decouple actuators 60 from apparatus 10. Actuators 62 may comprise, for example, strands of suture or wire.

Figures 1 A and 2A illustrate anchor 30 in a delivery configuration or in a partially deployed configuration (e.g., after dynamic self-expansion expansion from a constrained delivery configuration within a delivery sheath). Anchor 30 has a relatively long length and a relatively small width in the delivery or partially deployed configuration, as compared to the foreshortened and fully deployed configuration of Figures 1B and 2B.

In Figures 1A and 2A, replacement valve 20 is collapsed within lumen 31 of anchor 30. Retraction of actuators 50 relative to actuators 60 foreshortens anchor 30, which increases the anchor's width while decreasing its length. Such foreshortening also properly seats replacement valve 20 within lumen 31 of anchor 30. Imposed foreshortening will enhance radial force applied by apparatus 10 to surrounding tissue over at least a portion of anchor 30. In some embodiments, the anchor exerts an outward force on surrounding tissue to engage the tissue in such way to prevent migration of anchor caused by force of blood against closed leaflet during diastole. This anchoring force is preferably 0,45 kg to 0,91 kg [1 to 2 lbs], more preferably 0,91 kg to 1,81 kg [2 to 4 lbs], or more preferably 1,81 to 4,54 kg [4 to 10 lbs]. In some embodiments, the anchoring force is preferably greater than 0,45 kg [1 pound], more preferably greater than 0,91 kg [2 pounds], or more preferably greater than 1,81 kg [4 pounds]. Enhanced radial force of the anchor is also important for enhanced crush resistance of the anchor against the surrounding tissue due to the healing response (fibrosis and contraction of annulus over a longer period of time) or to dynamic changes of pressure and flow at each heart beat In an alternative embodiment, the anchor pattern or braid is designed to have gaps or areas where the native tissue is allowed to protrude through the anchor slightly (not shown) and as the foreshortening is applied, the tissue is trapped in the anchor. This feature would provide additional means to prevent anchor migration and enhance long term stability of the device.

Deployment of apparatus 10 is fully reversible until lock 40 has been locked via mating of male interlocking elements 44 with female interlocking elements 42. Deployment is then completed by decoupling actuators 60 from lip section 32 of anchor 30 by retracting one end of each actuator 62 relative to the other end of the actuator, and by retracting one end of each actuator 50 relative to the other end of the actuator until each actuator has been removed from eyelet 45 of its corresponding male interlocking element 44.

As best seen in Figure 2B, body region 36 of anchor 30 optionally may comprise barb elements 37 that protrude from anchor 30 in the fully deployed configuration, for example, for engagement of a patient's native valve leaflets and to preclude migration of the apparatus.

With reference now to Figures 3, a delivery and deployment system for a self-expanding embodiment of apparatus 10 including a sheath 110 having a lumen 112. Self-expanding anchor 30 is collapsible to a delivery configuration within lumen 112 of sheath 110, such that apparatus 10 may be delivered via delivery system 100. As seen in Figure 3 A, apparatus 10 may be deployed from lumen 112 by retracting sheath 110 relative to apparatus 10, control actuators 50 and actuators 60, which causes anchor 30 to dynamically self-expand to a partially deployed configuration. Control actuators 50 then are retracted relative to apparatus 10 and actuators 60 to impose foreshortening upon anchor 30, as seen in Figure 3B.

During foreshortening, actuators 60 push against lip region 32 of anchor 30, while actuators 50 pull on posts 38 of the anchor. Actuators 62 may be retracted along with actuators 50 to enhance the distally-directed pushing force applied by actuators 60 to lip region 32. Continued retraction of actuators 50 relative to actuators 60 would lock locks 40 and fully deploy apparatus 10 with replacement valve 20 properly seated within anchor 30, as in Figures 1B and 2B. Apparatus 10 comprises enhanced radial strength in the fully deployed configuration as compared to the partially deployed configuration of Figure 3 A. Once apparatus 10 has been fully deployed, actuators 50 and 62 may be removed from apparatus 10, thereby separating delivery system 100 including actuators 60 from the apparatus.

Deployment of apparatus 10 is fully reversible until locks 40 have been actuated. For example, just prior to locking the position of the anchor and valve and the operation of the valve may be observed under fluoroscopy. If the position needs to be changed, by alternately relaxing and reapplying the proximally directed forces exerted by control actuators 50 and/or control actuators 62 and the distally directed forces exerted by actuators 60, expansion and contraction of the lip and skirt regions of anchor 30 may be independently controlled so that the anchor and valve can be moved to, e.g., avoid blocking the coronary ostia or impinging on the mitral valve. Apparatus 10 may also be completely retrieved within lumen 112 of sheath 110 by simultaneously proximally retracting actuators 50 and actuators 60/actuators 62 relative to sheath 110. Apparatus 10 then may be removed from the patient or repositioned for subsequent redeployment.

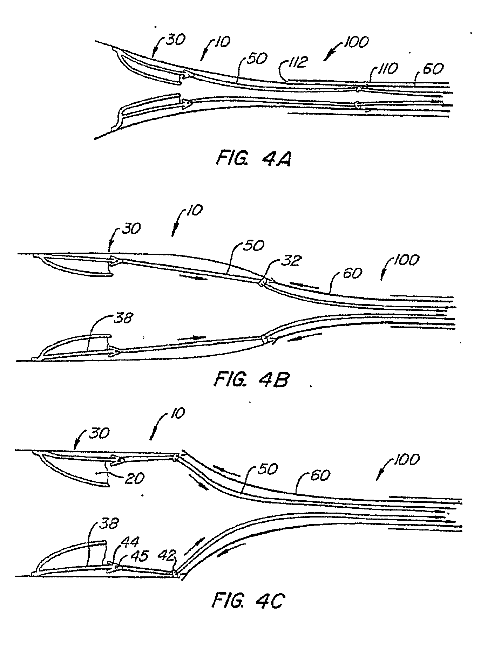

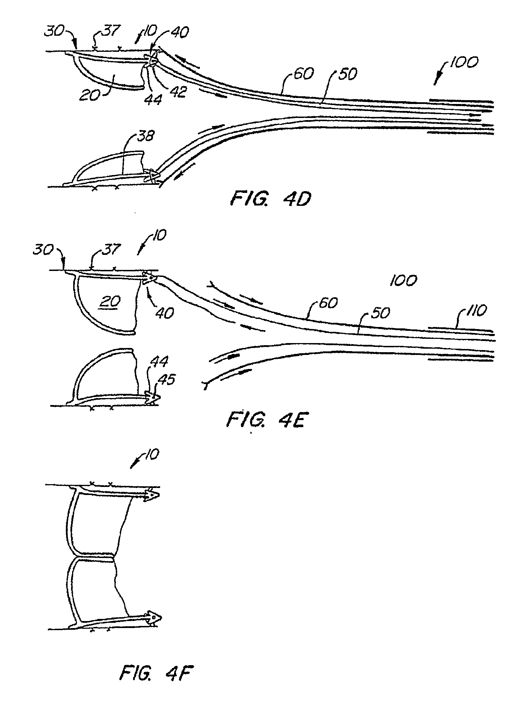

Referring now to Figures 4, step-by-step deployment of apparatus 10 via delivery system 100 is described. In Figure 4A, sheath 110 is retracted relative to apparatus 10, actuators 50 and actuators 60, thereby causing self-expandable anchor 30 to dynamically self-expand apparatus 10 from the collapsed delivery configuration within lumen 112 of sheath 110 to the partially deployed configuration. Apparatus 10 may then be dynamically repositioned via actuators 60 to properly orient the apparatus, e.g. relative to a patient's native valve leaflets.

In Figure 4B, control actuators 50 are retracted while actuators 60 are advanced, thereby urging lip region 32 of anchor 30 in a distal direction while urging posts 38 of the anchor in a proximal direction. This foreshortens apparatus 10, as seen in Figure 4C. Deployment of apparatus 10 is fully reversible even after foreshortening has been initiated and has advanced to the point illustrated in Figure 4C.

In Figure 4D, continued foreshortening causes male interlocking elements 44 of locks 40 to engage female interlocking elements 42. The male elements mate with the female elements, thereby locking apparatus 10 in the foreshortened configuration, as seen in Figure 4E. Actuators 50 are then pulled through eyelets 45 of male elements 44 to remove the actuators from apparatus 10, and actuators 62 are pulled through the proximal end of anchor 30 to uncouple actuators 60 from the apparatus, thereby separating delivery system 100 from apparatus 10. Fully deployed apparatus 10 is shown in Figure 4F.

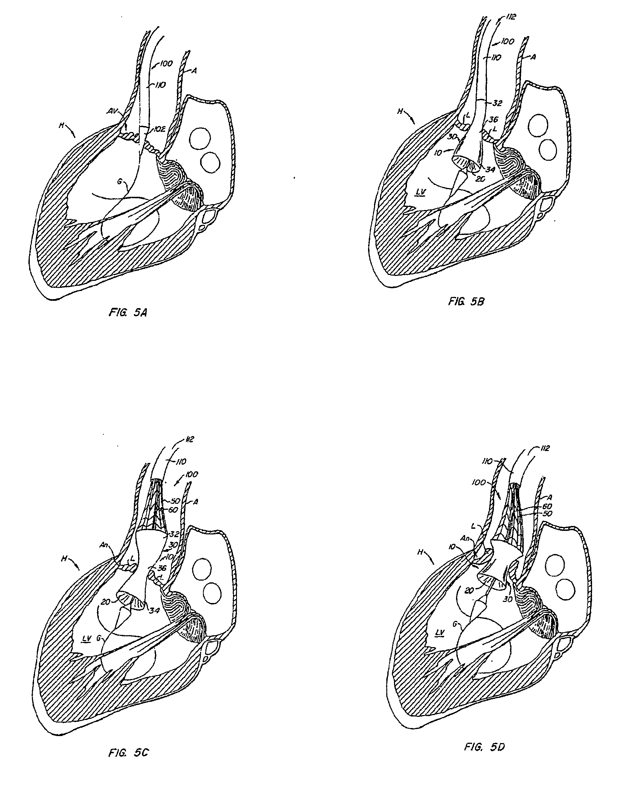

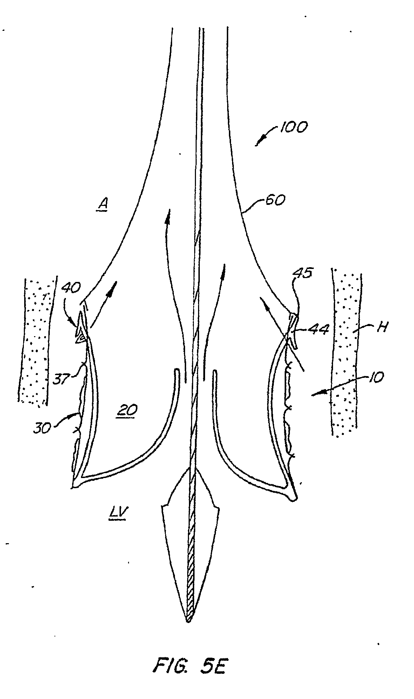

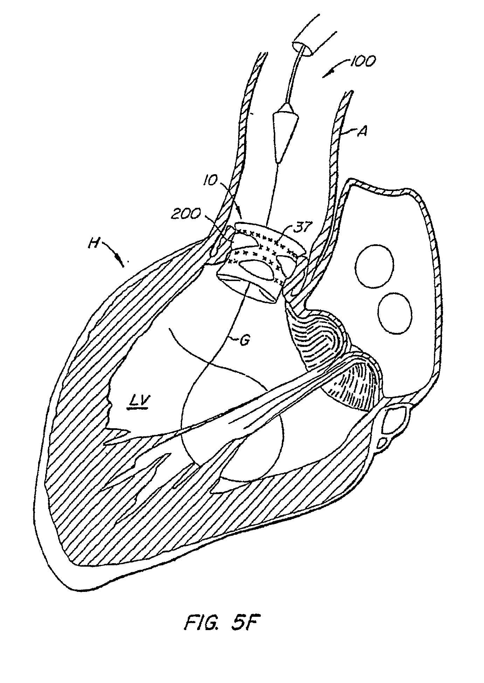

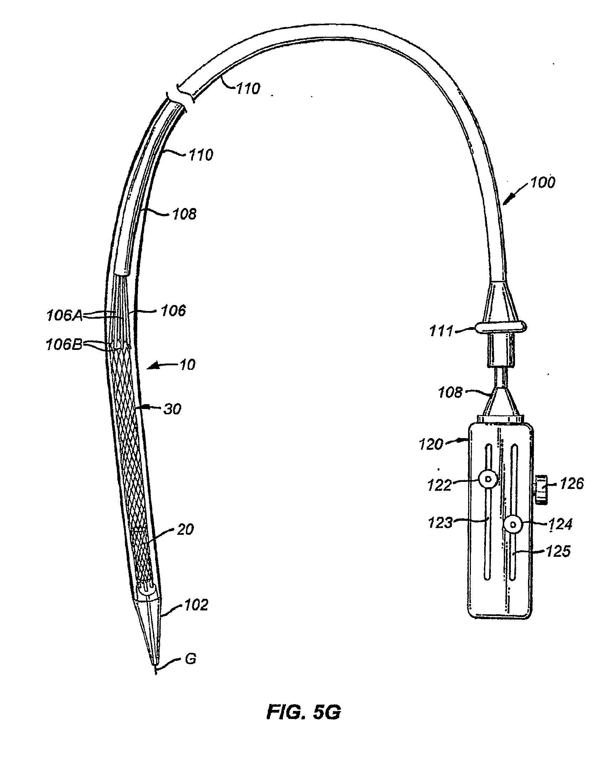

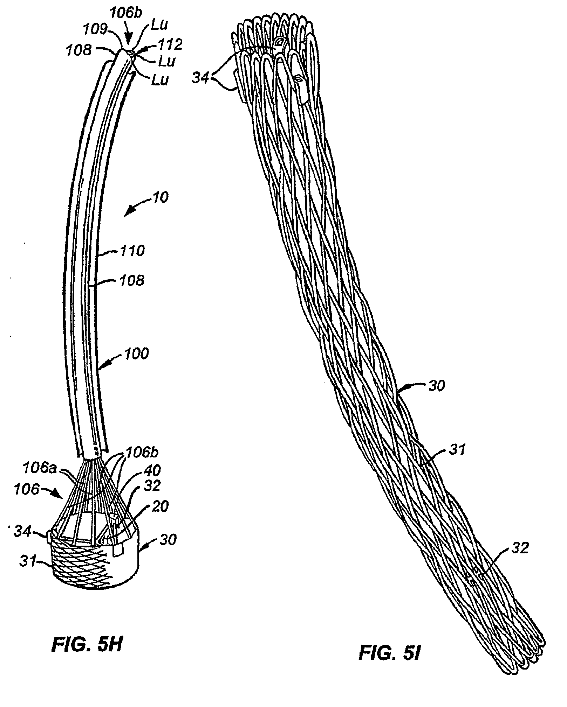

Referring to Figures 5, a method of percutaneously replacing a patient's diseased aortic valve with apparatus 10 and delivery system 100 is described. As seen in Figure 5 A, sheath 110 of delivery system 100, having apparatus 10 disposed therein, is percutaneously advanced over guide wire G, preferably in a retrograde fashion (although an antegrade or hybrid approach alternatively may be used), through a patient's aorta A to the patient's diseased aortic valve AV. A nosecone 102 precedes sheath 110 in a known manner. In Figure 5B, sheath 110 is positioned such that its distal region is disposed within left ventricle LV of the patient's heart H.

Apparatus 10 is deployed from lumen 112 of sheath 110, for example, under fluoroscopic guidance, such that anchor 30 of apparatus 10 dynamically self-expands to a partially deployed configuration, as in Figure 5C. Advantageously, apparatus 10 may be retracted within lumen 112 of sheath 110 via actuators 50 - even after anchor 30 has dynamically expanded to the partially deployed configuration, for example, to abort the procedure or to reposition apparatus 10 or delivery system 100. As yet another advantage, apparatus 10 may be dynamically repositioned, e.g. via sheath 110 and/or actuators 60, in order to properly align the apparatus relative to anatomical landmarks, such as the patient's coronary ostia or the patient's native valve leaflets L. When properly aligned, skirt region 34 of anchor 30 preferably is disposed distal of the leaflets, while body region 36 is disposed across the leaflets and lip region 32 is disposed proximal of the leaflets.

Once properly aligned, actuators 50 are retracted relative to actuators 60 to impose foreshortening upon anchor 30 and expand apparatus 10 to the fully deployed configuration, as in Figure 5D. Foreshortening increases the radial strength of anchor 30 to ensure prolonged patency of valve annulus An, as well as to provide a better seal for apparatus 10 that reduces paravalvular regurgitation. As seen in Figure 5E, locks 40 maintain imposed foreshortening. Replacement valve 20 is properly seated within anchor 30, and normal blood flow between left ventricle LV and aorta A is thereafter regulated by apparatus 10. Deployment of apparatus 10 advantageously is fully reversible until locks 40 have been actuated.

As seen in Figure 5F, actuators 50 have been pulled from eyelets 45 of male elements 44 of locks 40, actuators 60 are decoupled from anchor 30, e.g. via actuators 62, and delivery system 100 is removed from the patient, thereby completing deployment of apparatus 10. Optional barb elements 37 engage the patient's native valve leaflets, e.g. to further preclude migration of the apparatus and/or reduce paravalvular regurgitation.

Figures 5G and 5H show further details of deployment using a deployment apparatus. Apparatus 10 is deployed from lumen Lu of sheath 110, for example, under fluoroscopic guidance by proximally retracting proximal handle 111 of sheath 110 relative to shaft 108, such that anchor 30 of apparatus 10 dynamically self-expands to the partially deployed configuration of Figure 5C. Advantageously, apparatus 10 may be retracted within lumen Lu of sheath 110 by retracting shaft 108 relative to the sheath, and thereby retracting actuators 106a coupled to anchor 30 relative to sheath 110. In this manner, anchor 30 may be retrieved even after the anchor has dynamically expanded to the partially deployed configuration, for example, to abort the procedure or to reposition apparatus 10 or delivery system 100. As yet another advantage, apparatus 10 may be dynamically repositioned, in order to properly align the apparatus relative to anatomical landmarks, such as the patient's coronary ostia or the patient's native valve leaflets L. When properly aligned, a distal region of anchor 30 preferably is disposed distal of the leaflets, while a central region of the anchor is disposed across the leaflets and a proximal region is disposed proximal of the leaflets.

Once properly aligned, actuators 106b are proximally retracted relative to actuators 106a, e.g., via knob 126 of handle 120, to impose foreshortening upon anchor 30 and further expand apparatus 10 to the fully deployed configuration, as in Figure 5D. Foreshortening increases the radial strength of anchor 30 to ensure prolonged patency of valve annulus An, as well as to provide a better seal for apparatus 10 that reduces paravalvular regurgitation. Lock 40 formed by engaging post lock elements 44 of posts 32 with anchor lock elements 34 of anchor 30 maintains imposed foreshortening. Replacement valve 20 is properly seated within anchor 30, and normal blood flow between left ventricle LV and aorta A is thereafter completely regulated by apparatus 10, although valve 20 is functional during deployment as well. Deployment of apparatus 10 advantageously is fully reversible until the locks have been actuated. Releasable lock prevention mechanisms may be provided to ensure that the locks are not actuated prematurely. Furthermore, the locks may be reversible, such that apparatus 10 may be retrieved or repositioned even after actuation of the locks.

Once apparatus 10 is fully expanded and locked in the expanded configuration, actuators 106a are decoupled from anchor 30 by actuating releasable attachment mechanisms, e.g., by retracting release actuators 112 relative to the actuators 106a via knob 122 of handle 120. Likewise, actuators 106b are decoupled from posts 32 by actuating releasable attachment mechanisms, e.g., by retracting release actuators 112 relative to the actuators 106b via knob 124 of handle 120. As seen in Figure 5E, delivery system 100 then may be removed from the patient, thereby completing deployment of apparatus 10. Optional barb elements 37 engage the patient's native valve leaflets, e.g. to preclude migration of the apparatus and/or to reduce paravalvular regurgitation.

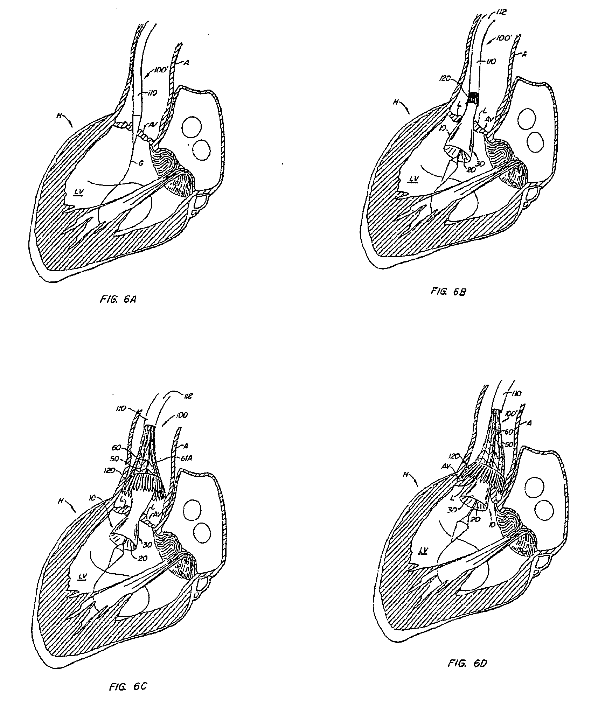

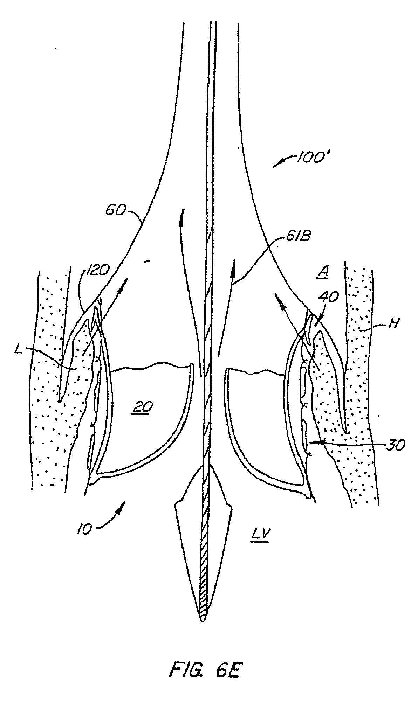

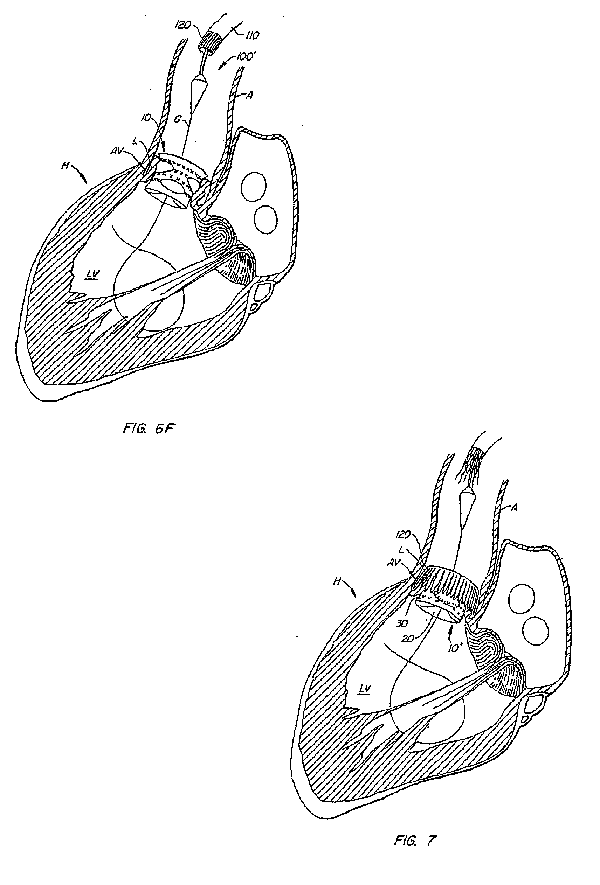

With reference now to Figures 6, a method of percutaneously replacing a patient's diseased aortic valve with apparatus 10 is provided, wherein proper positioning of the apparatus is ensured via positive registration of a modified delivery system to the patient's native valve leaflets. In Figure 6A, modified delivery system 100' delivers apparatus 10 to diseased aortic valve AV within sheath 110. As seen in Figures 6B and 6C, apparatus 10 is deployed from lumen 112 of sheath 110, for example, under fluoroscopic guidance, such that anchor 30 of apparatus 10 dynamically self-expands to a partially deployed configuration. As when deployed via delivery system 100, deployment of apparatus 10 via delivery system 100' is fully reversible until locks 40 have been actuated.

Delivery system 100' comprises leaflet engagement element 120, which preferably self-expands along with anchor 30. Engagement element 120 is disposed between actuators 60 of delivery system 100' and lip region 32 of anchor 30. Element 120 releasably engages the anchor. As seen in Figure 6C, the element is initially deployed proximal of the patient's native valve leaflets L. Apparatus 10 and element 120 then may be advanced/dynamically repositioned until engagement element positively registers against the leaflets, thereby ensuring proper positioning of apparatus 10. Also delivery system 100' includes filter structure 61 A (e.g., filter membrane or braid) as part of push actuators 60 to act as an embolic protection element. Emboli can be generated during manipulation and placement of anchor from either diseased native leaflet or surrounding aortic tissue and can cause blockage.
Arrows 61B in Figure 6E show blood flow through filter structure 61 A where blood is allowed to flow but emboli is trapped in the delivery system and removed with it at the end of the procedure.

Alternatively, foreshortening may be imposed upon anchor 30 while element 120 is disposed proximal of the leaflets, as in Figure 6D. Upon positive registration of element 120 against leaflets L, element 120 precludes further distal migration of apparatus 10 during additional foreshortening, thereby reducing a risk of improperly positioning the apparatus. Figure 6E details engagement of element 120 against the native leaflets. As seen in Figure 6F, once apparatus 10 is fully deployed, element 120, actuators 50 and actuators 60 are decoupled from the apparatus, and delivery system 100' is removed from the patient, thereby completing the procedure.

With reference to Figure 7, an alternative embodiment of the apparatus of Figures 6 is described, wherein leaflet engagement element 120 is coupled to anchor 30 of apparatus 10', rather than to delivery system 100. Engagement element 120 remains implanted in the patient post-deployment of apparatus 10'. Leaflets L are sandwiched between lip region 32 of anchor 30 and element 120 in the fully deployed configuration. In this manner, element 120 positively registers apparatus 10' relative to the leaflets and precludes distal migration of the apparatus over time.

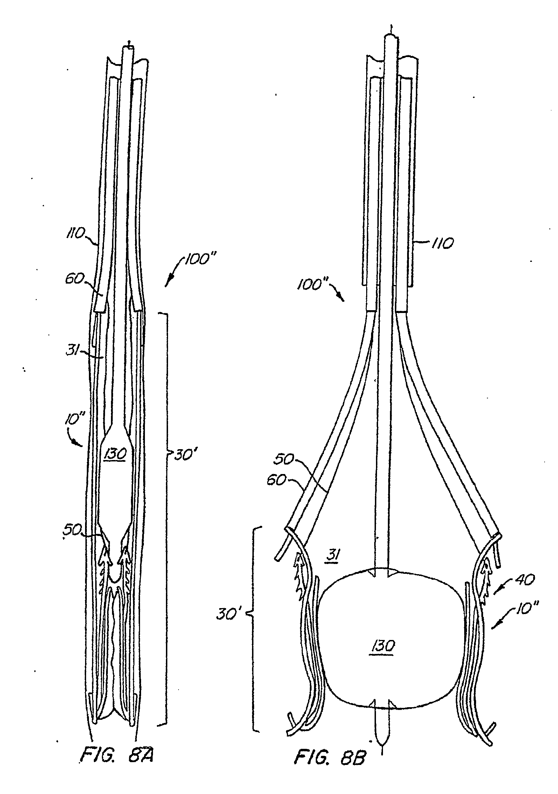

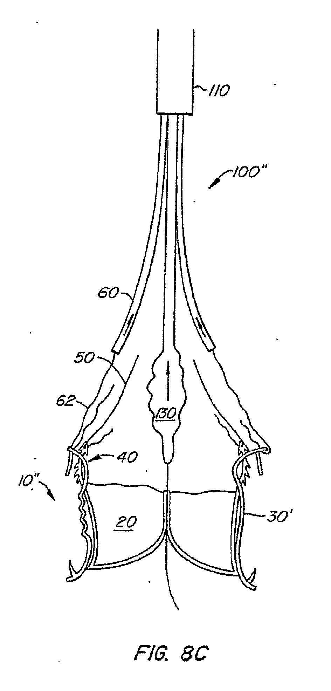

Referring now to Figures 8, an alternative delivery system adapted for use with a balloon expandable embodiment of the present invention is described. In Figure 8 A, apparatus 10" comprises anchor 30' that maybe fabricated from balloon-expandable materials. Delivery system 100" comprises inflatable member 130 disposed in a deflated configuration within lumen 31 of anchor 30'. In Figure 8B, optional outer sheath 110 is retracted, and inflatable member 130 is inflated to expand anchor 30' to the fully deployed configuration. As inflatable member 130 is being deflated, as in earlier embodiments, actuators 50 and 62 and actuators 60 maybe used to assist deployment of anchor 30' and actuation of locks 40, as well as to provide reversibility and retrievability of apparatus 10" prior to actuation of locks 40. Next, actuators 50 and 62 and actuators 60 are removed from apparatus 10", and delivery system 100" is removed, as seen in Figure 8C.

As an alternative delivery method, anchor 30' may be partially deployed via partial expansion of inflatable member 130. The inflatable member would then be advanced within replacement valve 20 prior to inflation of inflatable member 130 and full deployment of apparatus 10". Inflation pressures used will range from about 3,0 to 6,1 bar [3 to 6 atm], or more preferably from about 4,1 to 5,1 bar [4 to 5 atm], though higher and lower atm pressures may also be used (e.g., greater than 3,1 bar [3 atm], more preferably greater than 4,1 bar [4 atm], more preferably greater than 5,1 bar [5 atm], or more preferably greater than 6,1 bar [6 atm]). Advantageously, separation of inflatable member 130 from replacement valve 20, until partial deployment of apparatus 10" at a treatment site, is expected to reduce a delivery profile of the apparatus, as compared to previously known apparatus. This profile reduction may facilitate retrograde delivery and deployment of apparatus 10", even when anchor 30' is balloon-expandable.

Although anchor 30' has illustratively been described as fabricated from balloon-expandable materials, it should be understood that anchor 30' alternatively may be fabricated from self-expanding materials whose expansion optionally may be balloon-assisted. In such a configuration, anchor 30' would expand to a partially deployed configuration upon removal of outer sheath 110. If required, inflatable member 130 then would be advanced within replacement valve 20 prior to inflation. Inflatable member 130 would assist full deployment of apparatus 10", for example, when the radial force required to overcome resistance from impinging tissue were too great to be overcome simply by manipulation of actuators 50 and actuators 60. Advantageously, optional placement of inflatable member 130 within replacement valve 20, only after dynamic self-expansion of apparatus 10" to the partially deployed configuration at a treatment site, is expected to reduce a delivery profile of the apparatus, as compared to previously known apparatus. This reduction may facilitate retrograde delivery and deployment of apparatus 10".

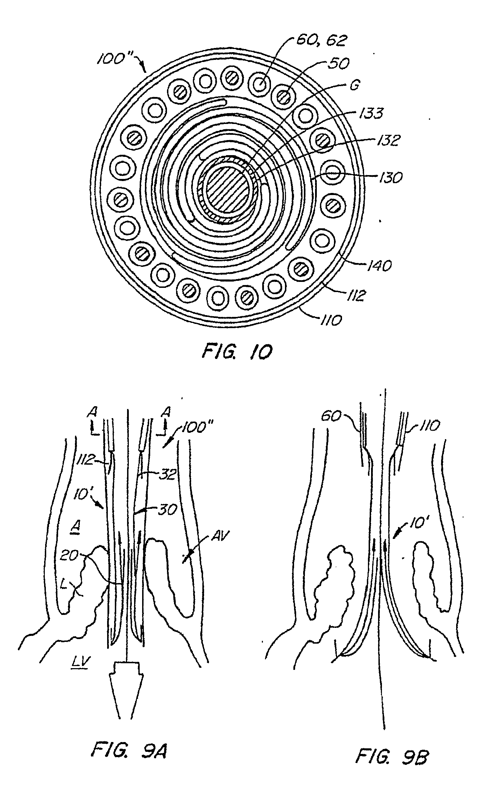

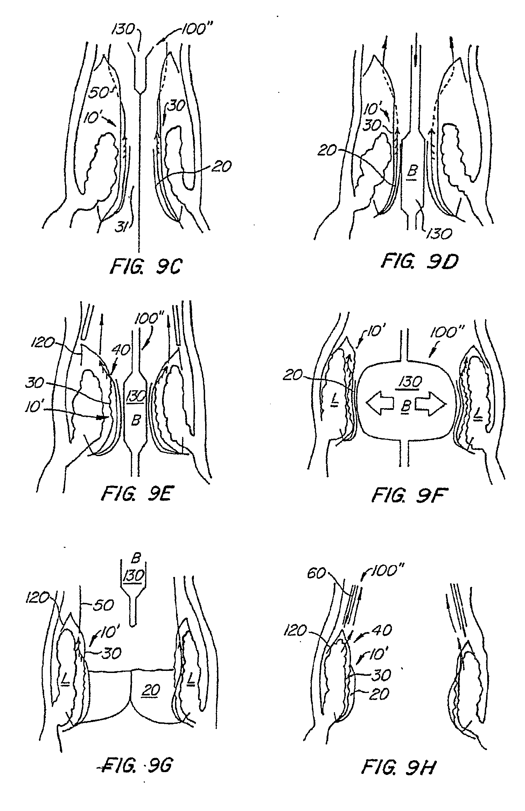

With reference to Figures 9 and 10, methods and apparatus for a balloon-assisted embodiment of the present invention are described in greater detail. Figures 9 and 10 illustratively show apparatus 10' of Figures 7 used in combination with delivery system 100" of Figures 8. Figure 10 illustrates a sectional view of delivery system 100". Inner shaft 132 of inflatable member 130 preferably is about 4 Fr in diameter, and comprises lumen 133 configured for passage of guidewire G, having a diameter of about 0.0889 cm [0.035"], therethrough. Push actuators 60 and pull actuators 50 pass through guidetube 140, which preferably has a diameter of about 15 Fr or smaller. Guide tube 140 is disposed within lumen 112 of outer sheath 110, which preferably has a diameter of about 17 Fr or smaller.

In Figure 9A, apparatus 10' is delivered to diseased aortic valve AV within lumen 112 of sheath 110. In Figure 9B, sheath 110 is retracted relative to apparatus 10' to dynamically self-expand the apparatus to the partially deployed configuration. Also retracted and removed is nosecone 102 which is attached to a pre-slit lumen (not shown) that facilitates its removal prior to loading and advancing of a regular angioplasty balloon catheter over guidewire and inside delivery system 110.

In Figure 9C, pull actuators 50 and push actuators 60 are manipulated from external to the patient to foreshorten anchor 30 and sufficiently expand lumen 31 of the anchor to facilitate advancement of inflatable member 130 within replacement valve 20. Also shown is the tip of an angioplasty catheter 130 being advanced through delivery system 110.

The angioplasty balloon catheter or inflatable member 130 then is advanced within the replacement valve, as in Figure 9D, and additional foreshortening is imposed upon anchor 30 to actuate locks 40, as in Figure 9E. The inflatable member is inflated to further displace the patient's native valve leaflets L and ensure adequate blood flow through, and long-term patency of, replacement valve 20, as in Figure 9F. Inflatable member 130 then is deflated and removed from the patient, as in Figure 9G. A different size angioplasty balloon catheter could be used repeat the same step if deemed necessary by the user. Push actuators 60 optionally may be used to further set leaflet engagement element 120, or optional barbs B along posts 38, more deeply within leaflets L, as in Figure 9H. Then, delivery system 100" is removed from the patient, thereby completing percutaneous heart valve replacement.

As will be apparent to those of skill in the art, the order of imposed foreshortening and balloon expansion described in Figures 9 and 10 is only provided for the sake of illustration. The actual order may vary according to the needs of a given patient and/or the preferences of a given medical practitioner. Furthermore, balloon-assist may not be required in all instances, and the inflatable member may act merely as a safety precaution employed selectively in challenging clinical cases.

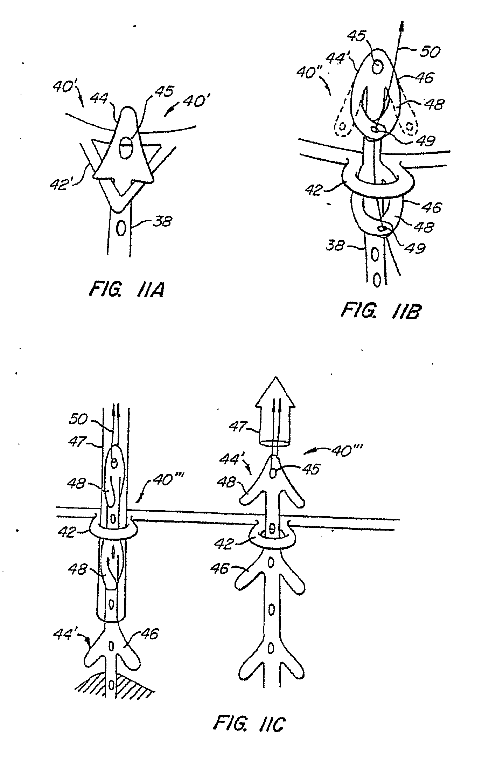

Referring now to Figures 11, alternative locks for use with apparatus of the present invention are described. In Figure 11 A, lock 40' comprises male interlocking element 44 as described previously. However, female interlocking element 42' illustratively comprises a triangular shape, as compared to the round shape of interlocking element 42 described previously. The triangular shape of female interlocking element 42' may facilitate mating of male interlocking element 44 with the female interlocking element without necessitating deformation of the male interlocking element.

In Figure 11 B, lock 40" comprises alternative male interlocking element 44' having multiple in-line arrowheads 46 along posts 38. Each arrowhead comprises resiliently deformable appendages 48 to facilitate passage through female interlocking element 42. Appendages 48 optionally comprise eyelets 49, such that control actuator 50 or a secondary wire may pass therethrough to constrain the appendages in the deformed configuration. To actuate lock 40", one or more arrowheads 46 of male interlocking element 44' are drawn through female interlocking element 42, and the wire is removed from eyelets 49, thereby causing appendages 48 to resiliently expand and actuate lock 40".

Advantageously, providing multiple arrowheads 46 along posts 38 yields a ratchet that facilitates in-vivo determination of a degree of foreshortening imposed upon apparatus of the present invention. Furthermore, optionally constraining appendages 48 of arrowheads 46 via eyelets 49 prevents actuation of lock 40" (and thus deployment of apparatus of the present invention) even after male element 44' has been advanced through female element 42. Only after a medical practitioner has removed the wire constraining appendages 48 is lock 40" fully engaged and deployment no longer reversible.

Lock 40"' of Figure 11 C is similar to lock 40" of Figure 11 B, except that optional eyelets 49 on appendages 48 have been replaced by optional overtube 47. Overtube 47 serves a similar function to eyelets 49 by constraining appendages 48 to prevent locking until a medical practitioner has determined that apparatus of the present invention has been foreshortened and positioned adequately at a treatment site. Overtube 47 is then removed, which causes the appendages to resiliently expand, thereby fully actuating lock 40"'.

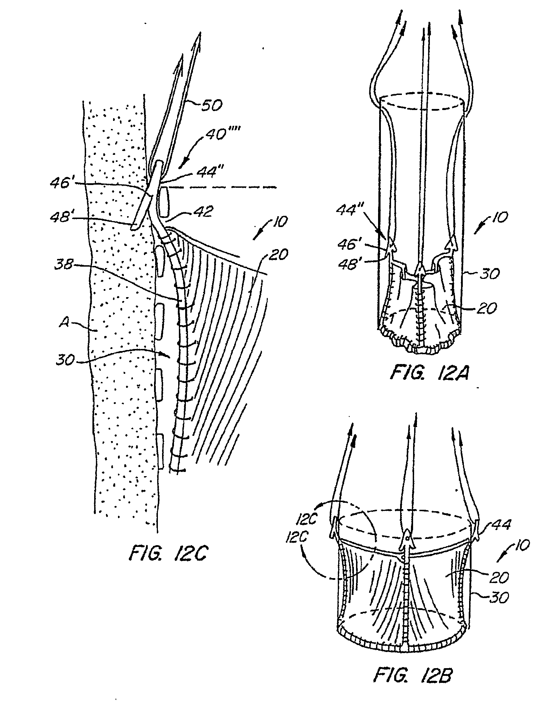

With reference to Figures 12, an alternative locking mechanism is described that is configured to engage the patient's aorta. Male interlocking elements 44" of locks 40"" comprise arrowheads 46' having sharpened appendages 48'. Upon expansion from the delivery configuration of Figure 12A to the foreshortened configuration of Figure 12B, apparatus 10 positions sharpened appendages 48' adjacent the patient's aorta A. Appendages 48' engage the aortic wall and reduce a risk of device migration over time.

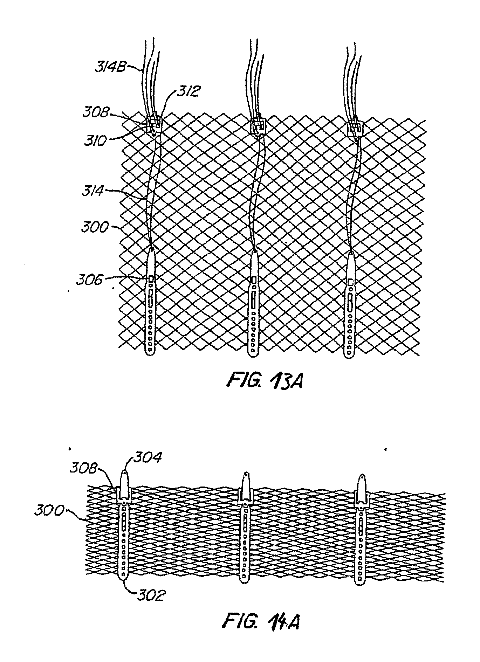

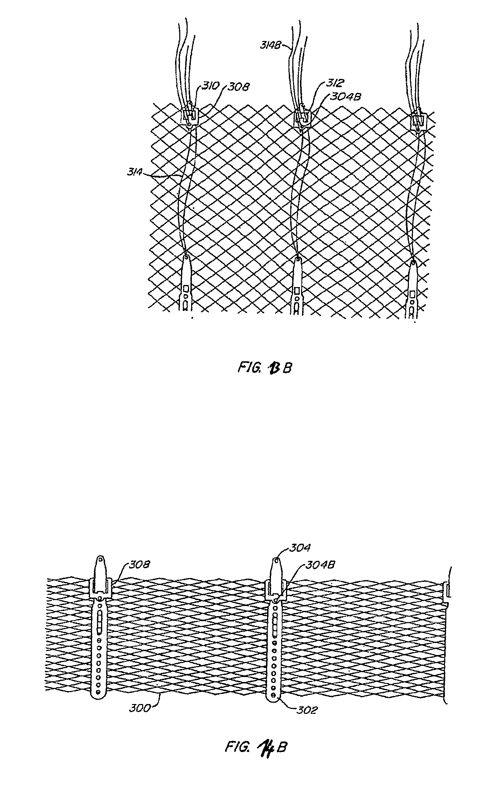

Figures 13 and 14 show yet another alternative embodiment of the anchor lock. Anchor 300 has a plurality of male interlocking elements 302 having eyelets 304 formed therein. Male interlocking elements are connected to braided structure 300 by inter-weaving elements 302 (and 308) or alternatively suturing, soldering, welding, or connecting with adhesive. Valve commissures 24 are connected to male interlocking elements 302 along their length. Replacement valve 20 annular base 22 is connected to the distal end 34 of anchor 300 (or 30) as is illustrated in figures 1A and 1B. Male interlocking elements 302 also include holes 306 that mate with tabs 310 extending into holes 312 in female interlocking elements 308. To lock, control actuators 314 passing through eyelets 304 and holes 312 arc pulled proximally with respect to the proximal end of braided anchor 300 to draw the male interlocking elements through holes 312 so that tabs 310 engage holes 306 in male interlocking elements 302. Also shown is release actuators 314B that passes through eylet 304B in female interlocking element 308. If needed, during the procedure, the user may pull on release actuators 314B reversing orientation of tabs 310 releasing the anchor and allowing for repositioning of the device or it's removal from the patient. After the desired final position has been attained, release actuator 314B and control actuator 314 be removed from the patient with the delivery system.

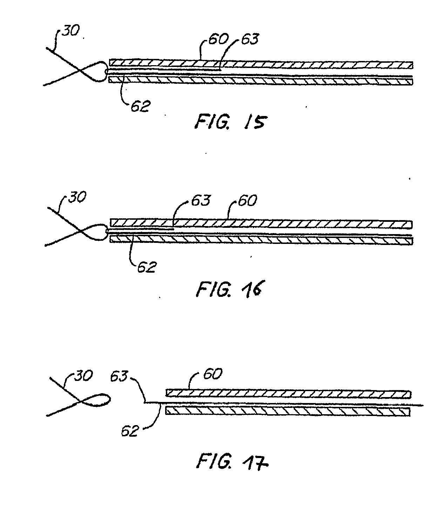

Figures 15-17 show an alternative way of releasing the connection between the anchor and its actuating actuators and control actuators. Control actuators 62 (i.e., release actuators) extend through actuators 60 from outside the patient, loop through the proximal region of anchor 30 and extend partially back into tube 60 (i.e., an anchor actuator). The doubled up portion of control actuator 62 creates a force fit within tube 60 that maintains the control actuators's position with respect to tube 60 when all control actuators 62 are pulled proximally to place a proximally directed force on anchor 30. When a single half of control actuator 62 is pulled proximally, however, the frictional fit between that control wire and the tube in which it is disposed is overcome, enabling the end 63 of control actuator 62 to pull free of the tube, as shown in Figure 17, thereby releasing anchor 30.

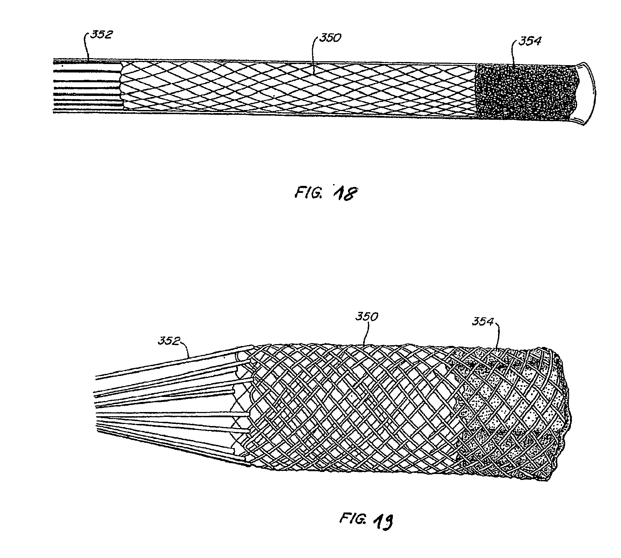

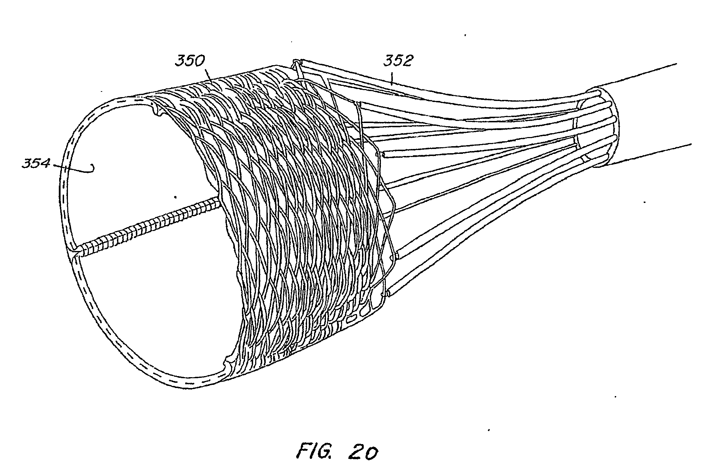

Figures 18-20 show an alternative embodiment of the anchor. Anchor 350 is made of a metal braid, such as Nitinol or stainless steel. A replacement valve 354 is disposed within anchor 350. Anchor 350 is actuated in substantially the same way as anchor 30 of Figures 1-4 through the application of proximally and distally directed forces from control actuators (not shown) and actuators 352.

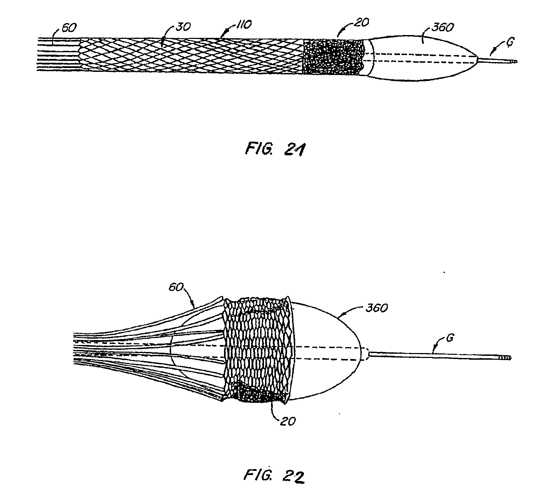

Figures 21 and 22 show yet another embodiment of the delivery and deployment apparatus of the invention. As an alternative to the balloon expansion method described with respect to Figures 8, in this embodiment the nosecone (e.g., element 102 of Figures 5) is replaced by an angioplasty balloon catheter 360. Thus, expandable balloon cathether 360 precedes sheath 110 on guidewire G. When anchor 30 and valve 20 are expanded through the operation of actuators 60 and the control actuators (not shown) as described above, balloon cathether 360 is retracted proximally within the expanded anchor and valve and expanded further as described above with respect to Figures 8.

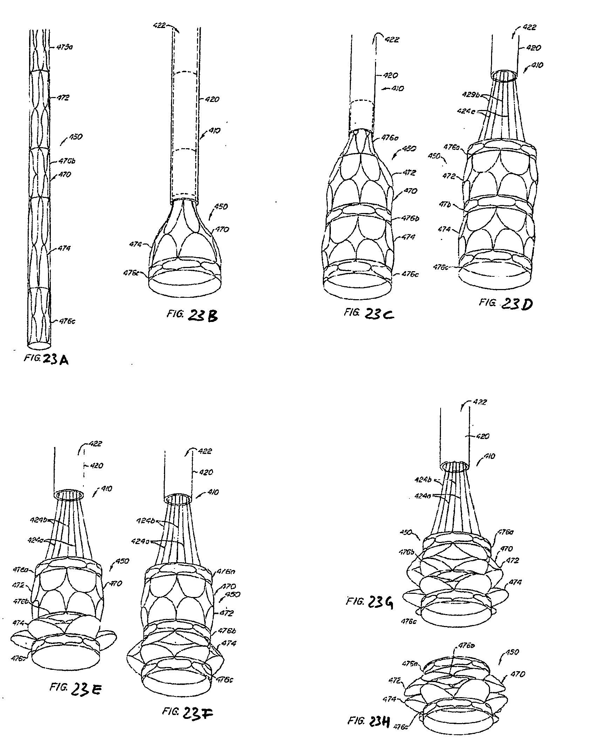

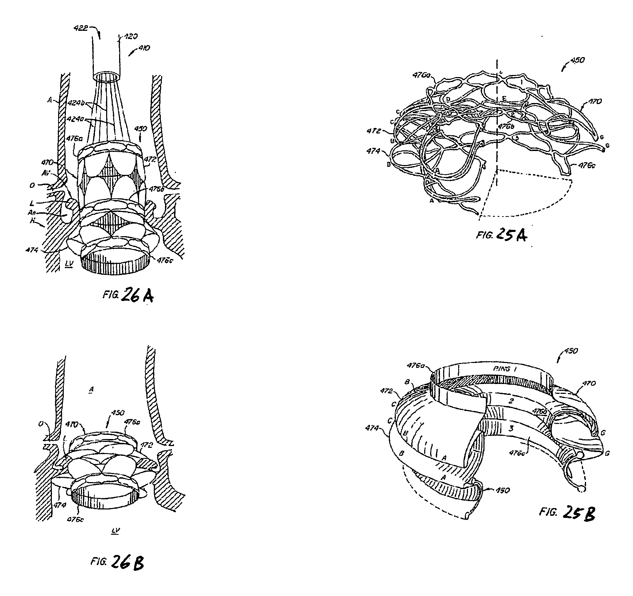

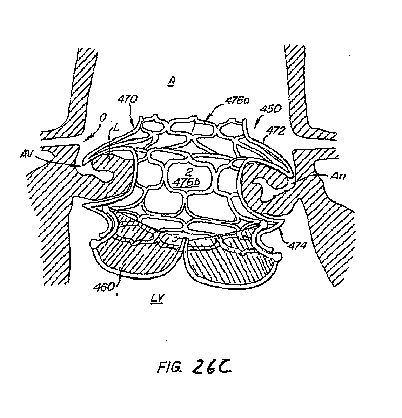

Figures 23A-H show another embodiment of a replacement heart valve apparatus in accordance with the present invention. Apparatus 450 comprises replacement valve 460 (see Figures 25B and 26C) disposed within and coupled to anchor 470. Replacement valve 460 is preferably biologic, e.g. porcine, but alternatively may be synthetic. Anchor 470 preferably is fabricated from self-expanding materials, such as a stainless steel wire mesh or a nickel-titanium alloy ("Nitinol"), and comprises lip region 472, skirt region 474, and body regions 476a, 476b and 476c. Replacement valve 460 preferably is coupled to skirt region 474, but alternatively may be coupled to other regions of the anchor. As described herein below, lip region 472 and skirt region 474 are configured to expand and engage/capture a patient's native valve leaflets, thereby providing positive registration, reducing paravalvular regurgitation, reducing device migration, etc.

As seen in Figure 23A, apparatus 450 is collapsible to a delivery configuration, wherein the apparatus may be delivered via delivery system 410. Delivery system 410 comprises sheath 420 having lumen 422, as well as actuators 424a and 424b seen in Figures 23D-23G. Actuators 424a are configured to expand skirt region 474 of anchor 470, as well as replacement valve 460 coupled thereto, while actuators 424b are configured to expand lip region 472.

As seen in Figure 23B, apparatus 450 may be delivered and deployed from lumen 422 of catheter 420 while the apparatus is disposed in the collapsed delivery configuration. As seen in Figures 23B-23D, catheter 420 is retracted relative to apparatus 450, which causes anchor 470 to dynamically self-expand to a partially deployed configuration. Actuators 424a are then retracted to expand skirt region 474, as seen in Figures 23E and 23F. Preferably, such expansion may be maintained via locking features described hereinafter.

In Figure 23G, actuators 424b are retracted to expand lip region 472 and fully deploy apparatus 450. As with skirt region 474, expansion of lip region 472 preferably may be maintained via locking features. After both lip region 472 and skirt region 474 have been expanded, actuators 424 may be removed from apparatus 450, thereby separating delivery system 410 from the apparatus. Delivery system 410 then may be removed, as seen in Figure 23H.

As will be apparent to those of skill in the art, lip region 472 optionally may be expanded prior to expansion of skirt region 474. As yet another alternative, lip region 472 and skirt region 474 optionally may be expanded simultaneously, in parallel, in a step-wise fashion or sequentially. Advantageously, delivery of apparatus 450 is fully reversible until lip region 472 or skirt region 474 has been locked in the expanded configuration.

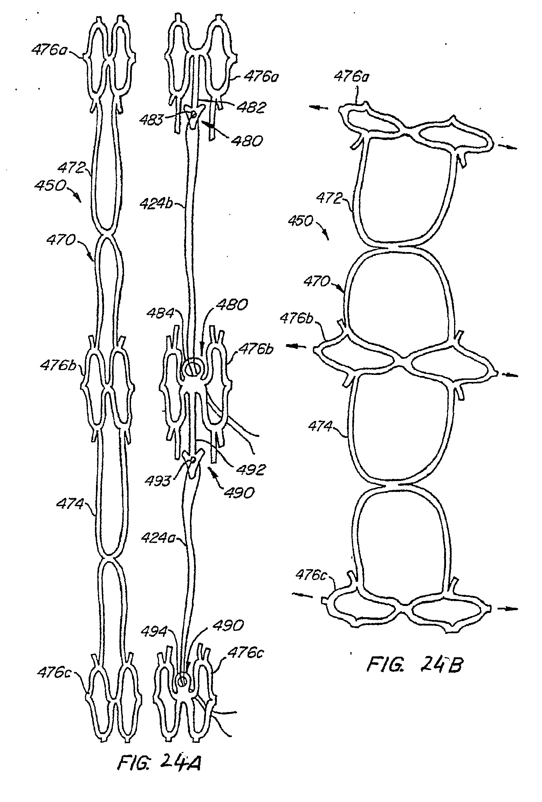

With reference now to Figures 24A-E, individual cells of anchor 470 of apparatus 450 are described to detail deployment and expansion of the apparatus. In Figure 24A, individual cells of lip region 472, skirt region 474 and body regions 476a, 476b and 476c are shown in the collapsed delivery configuration, as they would appear while disposed within lumen 422 of sheath 420 of delivery system 410 of Figures 23. A portion of the cells forming body regions 476, for example, every 'nth' row of cells, comprises locking features.

Body region 476a comprises male interlocking element 482 of lip lock 480, while body region 476b comprises female interlocking element 484 of lip lock 480. Male element 482 comprises eyelet 483. Wire 424b passes from female interlocking element 484 through eyelet 483 and back through female interlocking element 484, such that there is a double strand of wire 424b that passes through lumen 422 of catheter 420 for manipulation by a medical practitioner external to the patient. Body region 476b further comprises male interlocking element 492 of skirt lock 490, while body region 476c comprises female interlocking element 494 of the skirt lock. Wire 424a passes from female interlocking element 494 through eyelet 493 of male interlocking element 492, and back through female interlocking element 494. Lip lock 480 is configured to maintain expansion of lip region 472, while skirt lock 490 is configured to maintain expansion of skirt region 474.

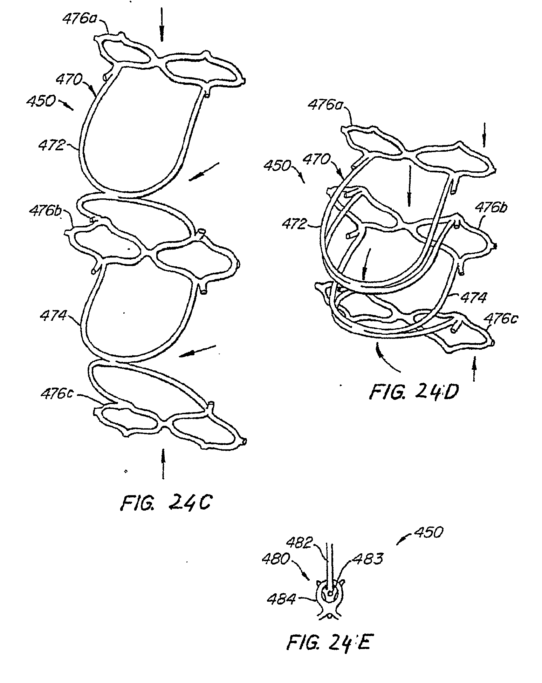

In Figure 24B, anchor 470 is shown in the partially deployed configuration, e.g., after deployment from lumen 422 of sheath 420. Body regions 476, as well as lip region 472 and skirt region 474, self-expand to the partially deployed configuration. Full deployment is then achieved by refracting actuators 424 relative to anchor 470, and expanding lip region 472 and skirt region 474 outward, as seen in Figures 24C and 24D. As seen in Figure 24E, expansion continues until the male elements engage the female interlocking elements of lip lock 480 and skirt lock 490, thereby maintaining such expansion (lip lock 480 shown in Figure 24E). Advantageously, deployment of apparatus 450 is fully reversible until lip lock 480 and/or skirt lock 490 has been actuated.

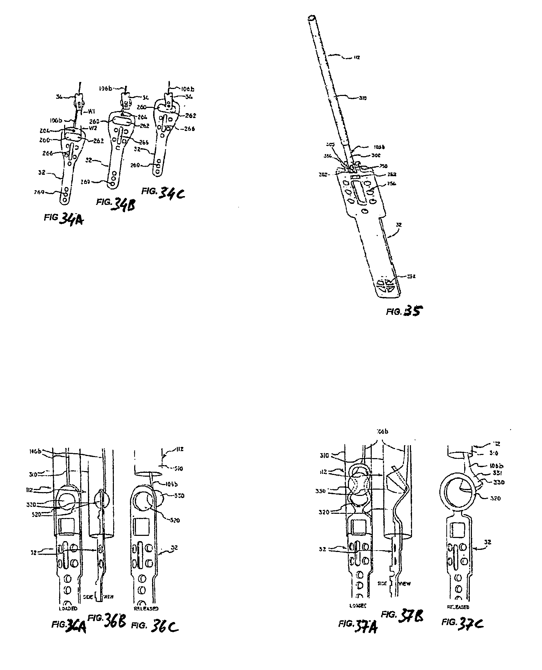

With reference to Figures 25A-B, isometric views, partially in section, further illustrate apparatus 450 in the fully deployed and expanded configuration. Figure 25A illustrates the wireframe structure of anchor 470, while Figure 25B illustrates an embodiment of anchor 470 covered in a biocompatible material B. Placement of replacement valve 460 within apparatus 450 may be seen in Figure 37B. The patient's native valve is captured between lip region 472 and skirt region 474 of anchor 470 in the fully deployed configuration (see Figure 26B).

Referring to Figures 26 A-C, in conjunction with Figures 23 and 24, a method for percutaneously replacing a patient's diseased aortic valve with apparatus 450 is described. Delivery system 410, having apparatus 450 disposed therein, is percutaneously advanced, preferably in a retrograde fashion, through a patient's aorta A to the patient's diseased aortic valve AV. Sheath 420 is positioned such that its distal end is disposed within left ventricle LV of the patient's heart H. As described with respect to Figures 23, apparatus 450 is deployed from lumen 422 of sheath 420, for example, under fluoroscopic guidance, such that skirt section 474 is disposed within left ventricle LV, body section 476b is disposed across the patient's native valve leaflets L, and lip section 472 is disposed within the patient's aorta A. Advantageously, apparatus 450 may be dynamically repositioned to obtain proper alignment with the anatomical landmarks. Furthermore, apparatus 450 may be refracted within lumen 422 of sheath 420 via actuators 424, even after anchor 470 has dynamically expanded to the partially deployed configuration, for example, to abort the procedure or to reposition sheath 420.

Once properly positioned, elements 424a are retracted to expand skirt region 474 of anchor 470 within left ventricle LV. Skirt region 474 is locked in the expanded configuration via skirt lock 490, as previously described with respect to Figures 24. In Figure 26A, skirt region 474 is maneuvered such that it engages the patient's valve annulus An and/or native valve leaflets L, thereby providing positive registration of apparatus 450 relative to the anatomical landmarks.

Elements 424b are then actuated external to the patient in order to expand lip region 472, as previously described in Figures 23. Lip region 472 is locked in the expanded configuration via lip lock 480. Advantageously, deployment of apparatus 450 is fully reversible until lip lock 480 and/or skirt lock 490 has been actuated. Elements 424 are pulled from eyelets 483 and 493, and delivery system 410 is removed from the patient. As will be apparent, the order of expansion of lip region 472 and skirt region 474 may be reversed, concurrent, etc.

As seen in Figure 26B, lip region 472 engages the patient's native valve leaflets L, thereby providing additional positive registration and reducing a risk of lip region 472 blocking the patient's coronary ostia O. Figure 26C illustrates the same in cross-sectional view, while also showing the position of replacement valve 460. The patient's native leaflets are engaged and/or captured between lip region 472 and skirt region 474. Advantageously, lip region 472 precludes distal migration of apparatus 450, while skirt region 474 precludes proximal migration. It is expected that lip region 472 and skirt region 474 also will reduce paravalvular regurgitation.

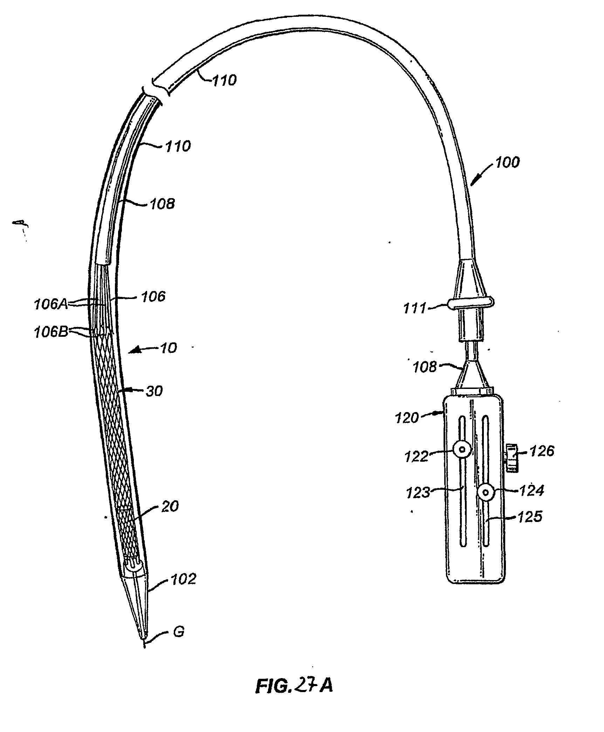

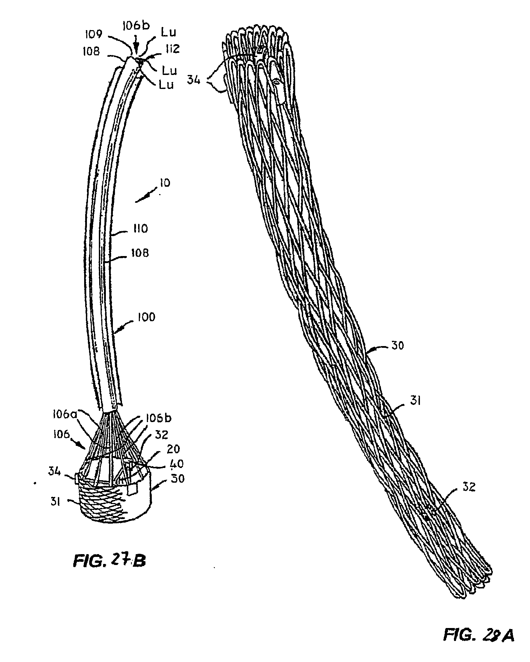

Figures 27A and 27B illustrate one embodiment of a delivery system/deployment tool and apparatus in accordance with the present invention. As seen in Figure 27A, apparatus 10 may be collapsed for delivery within delivery system/deployment tool 100. Delivery system 100 includes guidewire G, nosecone 102, anchor actuation elements 106, multi-lumen shaft or catheter 108 having optional central lumen 109 and a plurality of circumferentially disposed lumens Lu, external sheath 110 having optional proximal handle 111, and control handle 120. Nosecone 102 may, for example, be manipulated via a shaft extending through central lumen 109 of multi-lumen catheter 108.

Anchor actuation elements 106 preferably comprise both proximal anchor actuation elements and distal anchor actuation elements. The proximal anchor actuation elements may, for example, comprise actuators 106a that are releasably coupled to a proximal region of anchor 30 of apparatus 10 via releasable attachment mechanisms for manipulating a proximal region of apparatus 10. The distal anchor actuation elements may comprise actuators 106b that are releasably coupled to a distal region of anchor 30 via releasable attachment mechanisms for manipulating the distal region of apparatus 10. In some embodiments, the distal anchor actuation elements may comprise posts or anchor attachment elements 32 of anchor 30 and the releasable attachment mechanisms connecting actuators 106b to posts 32. In an alternative configuration, the proximal anchor actuation elements may be releasably coupled to a proximal region of apparatus 10 through posts and releasable attachment mechanisms for manipulation of a proximal region of the apparatus, while the distal anchor actuation elements may connect to a distal region of anchor 30 via releasable attachment mechanisms to manipulate a distal region of the apparatus. As another alternative, both proximal and distal anchor actuation element may connect to anchor 30 via releasable attachment mechanisms.

In the embodiment shown in Figures 27, actuators 106a may, for example, include stiff finger elements extending from a distal region of multi-lumen shaft 108, while actuators 106b may include control elements (e.g., strands of suture, or metal or polymer wires) which pass through one or more lumens Lu of shaft 108. Release actuators 112 for the releasable attachment mechanisms for both sets of actuators also may pass through one or more lumens Lu of shaft 108. The release actuators may comprise, for example, control elements (e.g., strands of suture, or metal or polymer wires), covers, mandrels, elongated elements, friction surfaces, wrap portions, interference shapes, etc. The release actuators preferably are movable relative to anchor actuation elements 106, e.g., via control handle 120.

Control handle 120 is coupled to multi-lumen shaft 108. Knob 122 disposed in slot 123 may actuate release actuators 112 that couple actuators 106a of anchor actuation elements 106 to apparatus 10. Likewise, knob 124 disposed in slot 125 may actuate release actuators 112 that couple actuators 106b of anchor actuation elements 106 to posts 32 of anchor 30 of apparatus 10. Handle 120 also comprises knob 126 for, e.g., manipulating the actuators 106b to control movement of the distal region of apparatus 10 relative to its proximal region. Conversely, controlled movement of the proximal region of apparatus 10 relative to its distal region may be achieved by holding knob 126 stationary while advancing or retracting handle 120. Knob 126 optionally may move actuators 106b in unison with their concomitant release actuators 112.

Apparatus 10 comprises anchor 30 and replacement valve 20. Anchor 30 comprises a braid, which has closed ends at either or both its ends. Replacement valve 20 is preferably coupled to the anchor along posts 32, e.g., along a valve attachment structure, such as a tab and/or a plurality of holes. Posts 32, therefore, may function as valve supports and may be adapted to support the replacement valve within the anchor. In the embodiment shown, there are three posts, corresponding to the valve's three commissural attachment points. The posts can be attached to the braid portion of anchor 30. The posts can be attached to the braid's distal end, as shown in Figure 28A, central region, or proximal end. Replacement valve 20 can be composed of a synthetic material and/or may be derived from animal tissue. Replacement valve 20 is preferably configured to be secured within anchor 30.

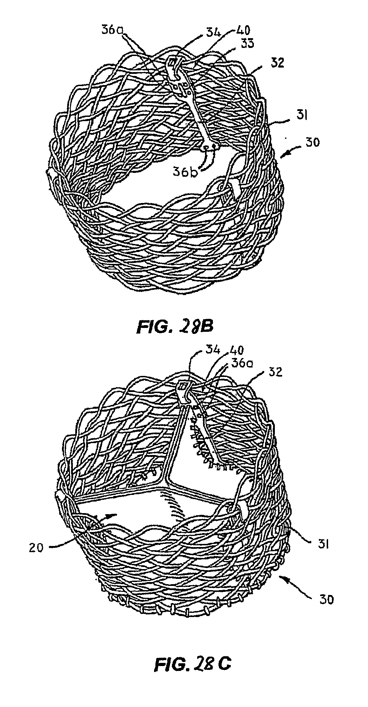

Anchor 30 comprises a plurality of anchor lock elements 34, e.g., buckles 34, attached to its proximal region, one for each post 32. Posts 32 may comprise a lock element that forms a two-part locking mechanism with anchor lock elements 34 for maintaining anchor 30 in a deployed or expanded configuration (e.g., as illustrated in Figures 27B, 28B and 28C).

Anchor 30 is formed from a collapsible and expandable wire braid. Anchor braid 30 is preferably self-expanding and is preferably formed from a material such as Nitinol, cobalt-chromium steel or stainless steel wire using one or more strands of wire. Delivery and deployment of braided anchor 30 is similar to the delivery and deployment of the anchors described in U.S. Patent Appl. Ser. No. 10/746,120. Specifically, in one embodiment described below, during deployment braided anchor 30 is actively foreshortened by proximally retracting the actuators 106b relative to the actuators 106a to expand and lock the anchor in place. In some embodiments, foreshortening may expand anchor 30 to a radially symmetrical, bilaterally symmetrical, or asymmetrical expanded shape. The foreshortening step can include expanding a first region of the anchor to a first diameter and a second region of the anchor to a second diameter larger than the first diameter. A third region may also be expanded to a diameter larger than the first diameter. The expansion of various regions of the anchor (e.g., the distal region) can be especially useful in locating the aortic valve and centering the anchor within it. Preferably, the secured anchor does not interfere with the mitral valve or the ostia. In some embodiments, the anchor is allowed to self-expand prior to the foreshortening step.

As seen in Figures 27, after endovascular delivery through sheath 110 to the vicinity of the patient's native valve (such as the aortic valve), apparatus 10 may be expanded from the collapsed delivery configuration of Figure 27A to the expanded deployed configuration of Figure 27B using delivery system/deployment tool 100. To deploy apparatus 10, external sheath 110 may be retracted relative to apparatus 10 by proximally retracting sheath handle 111 relative to control handle 120. Sheath 110 is thereby removed from the exterior of apparatus 10, permitting the anchor 30 to self-expand. For example, if anchor braid 30 is composed of a shape memory material, it may self-expand to or toward its "at-rest" configuration. This at-rest configuration of the braid can be, for example its expanded configuration, a collapsed configuration, or a partially expanded configuration between the collapsed configuration and the expanded configuration, or some combination. In preferred embodiments, the anchor's at-rest configuration is between the collapsed configuration and the expanded configuration. Depending on the at-rest diameter of the braid and the diameter of the patient's anatomy at the chosen deployment location, the anchor may or may not self-expand to come into contact with the diameter of the patient's anatomy at that location.

In its collapsed configuration, anchor 30 preferably has a collapsed delivery diameter between about 3 to 30 Fr, or more preferably 6 to 28 Fr, or more preferably 12 to 24 Fr. In some embodiments, anchor 30 in its collapsed configuration will have a length ranging from about 5 to about 170 mm, more preferably from about 10 to about 160 mm, more preferably from about 15 to about 150 mm, more preferably from about 20 to about 140 mm, or more preferably from about 25 mm to about 130 mm.

Similarly, in its expanded configuration, anchor 30 preferable has a diameter ranging between about 10 to about 36 mm, or more preferably from about 24 to about 33 mm, or more preferably from about 24 to about 30 mm. In some embodiments, anchor 30 in its expanded configuration will have a length ranging from about 1 to about 50 mm, more preferably from about 2 to about 40 mm, more preferably from about 5 to about 30 mm, or more preferably from about 7 to about 20 mm.

Overall, the ratio of deployed to collapsed/sheathed lengths is preferably between about 0.05 and 0.5, more preferably about 0.1 to 0.35, or more preferably about 0.15 to 0.25. In any of the embodiments herein, anchor 30 in its expanded configuration preferably has a radial crush strength that maintains the anchor substantially un-deformed in response to a pressure of up to about 0,5 bar [0.5 atm] directed substantially radially inward toward the central axis, or more preferably up to about 2,0 bar [2 atm] directed substantially radially inward toward the central axis. In addition, in any of the embodiments herein, the anchor preferably has an axial spring constant of between about 10 to 250 g/cm, more preferably between about 20 to 200 g/cm, or more preferably between about 40 to 160 g/cm. In addition, in any of the embodiments herein, the anchor is preferably adapted to support the replacement valve at the anchor site in response to a differential pressure of up to about 120 mm Hg, more preferably up to about 240 mm Hg, or more preferably up to about 320 mm Hg.

These parameters are not intended to be limiting. Additional parameters within the scope of the present invention will be apparent to those of skill in the art.

As seen in Figure 27B, anchor 30 may be expanded to a fully deployed configuration from a partial deployed configuration (e.g., self-expanded configuration) by actively foreshortening anchor 30 during endovascular deployment. Foreshortening of the apparatus involves applying a distally directed force on the proximal end of the anchor by one or more anchor actuation elements to move the proximal end of the anchor distally while maintaining the position of the distal end of the anchor. For example, the proximal region of anchor 30 may be pushed distally by certain anchor actuation elements 106, e.g., actuators 106a. In addition, foreshortening of the apparatus can involve applying a proximally directed force on the distal end of the anchor by one or more anchor actuation elements to move the distal end of the anchor proximally while maintaining the position of the proximal end of the anchor. For example, the distal region of anchor 30 may be pulled proximally via a proximally directed force applied by post actuation elements 106b, this force opposed by anchor actuators 106a.

Anchor actuation elements 106 preferably are adapted to expand radially as the anchor expands radially and to contract radially as the anchor contracts radially. Furthermore, proximally or distally directed forces by the anchor actuation elements on one end of the anchor do not diametrically constrain the opposite end of the anchor. In addition, when a proximally or distally directed force is applied on the anchor by the anchor actuation elements, it is preferably applied without passing any portion of a deployment system through a center opening of the replacement valve. This arrangement enables the replacement valve to operate during deployment and before removal of the deployment system.

The distal anchor actuation elements may include, for example, actuators 106b and/or release actuators 112 that are controlled, e.g., by control knobs 124 and 126 of control handle 120. Similarly, the proximal regions of anchor 30 may be pushed distally via proximal anchor actuation elements, e.g., actuators 106a, at the proximal region of the anchor. The proximal anchor actuation elements facilitate application of a distally directed force to the proximal end of anchor 30 to move or constrain the proximal end of the anchor distally and are controlled through motion of shaft 108 relative to the distal anchor actuation elements. Control knob 122 of control handle 120 may control release actuators 112 for releasing the proximal anchor actuation elements from the braid. The proximal anchor actuation elements may be further adapted to expand as the proximal end of the anchor expands radially during application of a distally directed force on the proximal end of the anchor. Preferably, the proximal anchor actuation elements apply a distally directed force on the proximal end of the anchor system through a plurality of actuators 106a in order to expand the braid of anchor 30. Such braid expansion optionally may be assisted via inflation of a balloon catheter (see Figures 21 and 22) reversibly disposed within apparatus 10, as described in U.S. Patent Appl. Ser. No. 10/746,120.

In the fully deployed configuration, lock elements of posts 32 and anchor lock elements or buckles 34 of anchor 30 may be used to lock and maintain the anchor in the deployed configuration. Apparatus 10 may be repositioned or retrieved from the patient until the lock elements of posts 32 have been interlocked with anchor lock elements 34 of anchor 30 to form lock 40. In one embodiment, actuators 106b and attendant release actuators 112 comprise control elements attached to posts 32 that are threaded through buckles 34 so that the proximally directed force exerted on posts 32 by the control elements during deployment pulls a lock element of posts 32 toward and through buckles 34 to form lock 40. In this manner, the control elements may act as both anchor actuators and lock actuators.

Such lock optionally may be selectively reversible to allow for repositioning and/or retrieval of apparatus 10 during or post-deployment. When the lock is selectively reversible, the apparatus may be repositioned and or retrieved as desired, i.e., even after actuation of lock 40.

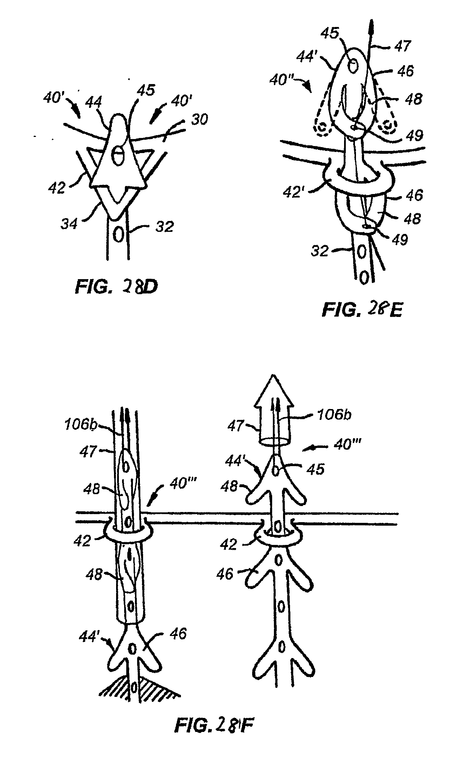

Locks used herein may also include a plurality of levels of locking wherein each level of locking results in a different amount of expansion. For example, the anchor lock elements at the proximal end of the post can have multiple configurations for locking within the buckle wherein each configuration results in a different amount of anchor expansion (see, e.g., Figure 28F). Such locking mechanisms may, for example, comprise ratchets having multiple lock locations. Furthermore, lock alignment features may be provided to facilitate alignment of the post and anchor lock elements, such as a hinge or an oversized width of the post or anchor lock elements. Furtherstill, lock prevention mechanisms may be provided to preclude locking until desired by a medical practitioner.

When apparatus 10 is placed across a patient's diseased heart valve, anchor 30 may be used to displace the patient's native valve leaflets, and replacement valve 20 will thereafter serve in place of the native valve. After final positioning and expansion, apparatus 10 may be decoupled from delivery system 100 by decoupling the proximal and distal anchor actuation elements 106 from the apparatus via releasable attachment mechanisms, e.g., by decoupling proximal actuators 106a from braided anchor 30 and distal actuators 106b from posts 32 of the anchor via the releasable attachment mechanisms. Moving release actuators 112, e.g., using knobs 122 and 124 of handle 120, may, for example, actuate the releasable attachment mechanisms. Preferably, the releasable attachment mechanisms may be actuated by moving the release actuator(s) less than about 2,54 cm [1 inch]. After decoupling, delivery system/deployment tool 100 may be removed from the patient, thereby completing endovascular replacement of a patient's heart valve.

Prior to implantation of replacement valve apparatus described herein, it may be desirable to perform a valvuloplasty on the patient's diseased valve by inserting a balloon into the valve and expanding it using, e.g., saline mixed with a contrast agent. In addition to preparing the valve site for implant, fluoroscopic viewing of the valvuloplasty will help determine the appropriate size of replacement valve implant to use.

Figures 28A-28C show further details of anchor 30 of apparatus 10. Figure 28A shows the apparatus in a collapsed configuration, such as for delivery within a sheath or other lumen or for retrieval and recapture into a sheath or other lumen. Figures 28B and 28C show the anchor and valve in an expanded and locked configuration.

As shown in Figure 28B, anchor 30 illustratively has three posts and three buckles. As seen in Figure 28C, the three leaflets of replacement valve 20 may be, coupled to the three posts 32 along valve support structures. Thus, posts 32 act as valve supports. The posts, unlike the braid, do not collapse or expand. In some embodiments, a post 32 has one or more proximal slots 33, at least one proximal hole 36a and at least one distal hole 36b. Leaflet tissue may, for example, be passed through slot 33 and sutured in place via suture routed through one or more proximal holes 36a. In this manner, slot(s) 33 and hole(s) 36a may form a valve support structure. Alternative valve support structures known in the art for fixing valve leaflets to posts may also be employed.

Posts 32 may be coupled to anchor braid 30 via one or more distal holes 36b. For example, anchor braid 30 may be woven through holes 36b, or a suture or wire may be routed through holes 36b and tied to the braid. Yet another proximal hole (not shown) in post 32 serves as an anchor lock element that interfaces with the anchor lock element provided by buckle 34 to form lock 40. Buckles 34 may likewise be attached to anchor braid 30 via weaving or suturing.

Alternative locks may be used to lock the anchor of the present invention in the foreshortened configuration, as shown, e.g., in Figures 28D-28F. Preferably, a lock of the present invention can have multiple locking options such that locking can confer a plurality of amounts of expansion. Furthermore, the locking option can be employed asymmetrically to confer non-cylindrical shapes to the anchor. In Figure 28D, lock 40' comprises male lock element 44 disposed on post 32 and anchor lock element 34 disposed on braided anchor 30. Anchor lock element 34 illustratively comprises triangular protrusion or eyelet 42 of anchor 30. The triangular shape of female lock element 42 may facilitate mating of male lock element 44 with the female lock element without necessitating deformation of the male lock element. One or more holes 45 may be provided through post 32, e.g., for releasably attaching an actuator 106b to the post.

In Figure 28E, lock 40" comprises alternative male lock element 44' having multiple in-line arrowheads 46 along posts 32. Each arrowhead comprises resiliently deformable appendages 48 to facilitate passage through female lock element 42', which illustratively comprises a rounded eyelet. Appendages 48 optionally comprise holes 49, such that releasable lock prevention mechanism 47, illustratively a control wire, may pass through the holes to constrain the appendages in the deformed configuration. To actuate lock 40", one or more arrowheads 46 of male lock element 44' are drawn through female lock element 42', e.g., via a post/lock actuator, and the lock prevention mechanism is removed from holes 49, thereby causing appendages 48 to resiliently expand and actuate lock 40".

Advantageously, providing multiple arrowheads 46 along posts 32 yields a ratchet that facilitates in-vivo determination of a degree of foreshortening and expansion imposed upon anchor 30. Furthermore, optionally constraining appendages 48 of arrowheads 46 via mechanism 47 prevents actuation of lock 40" (and thereby deployment of apparatus 10) even after male element 44' has been advanced through female element 42'. Only after a medical practitioner has removed lock prevention mechanism 47, which constrains appendages 48, is lock 40" fully engaged and is deployment no longer reversible.

Lock 40"' of Figure 28F is similar to lock 40" of Figure 28E, except that holes 49 on appendages 48 have been eliminated, and the lock prevention mechanism comprises overtube or cover 47. Overtube 47 constrains appendages 48 to prevent locking until a medical practitioner has determined that apparatus of the present invention has been foreshortened and positioned adequately at a treatment site. Lock 40'" may, for example, be actuated by applying a proximally-directed force to actuator 106b. Actuator 106b illustratively comprises a control wire releasably disposed through hole 45 in post 32. Lock prevention mechanism 47 then is withdrawn proximally relative to anchor 30, which causes the appendages to resiliently expand, thereby fully actuating lock 40"'.

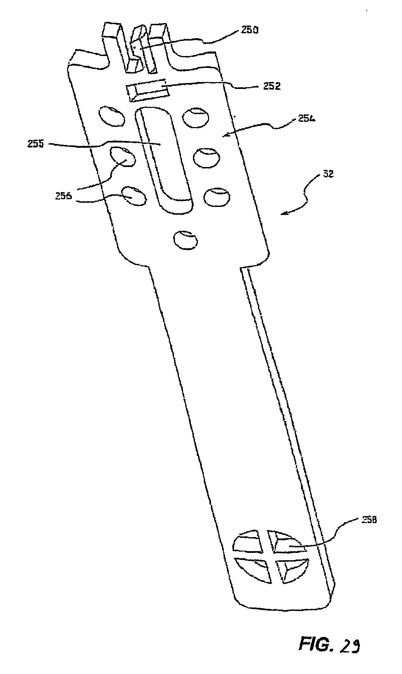

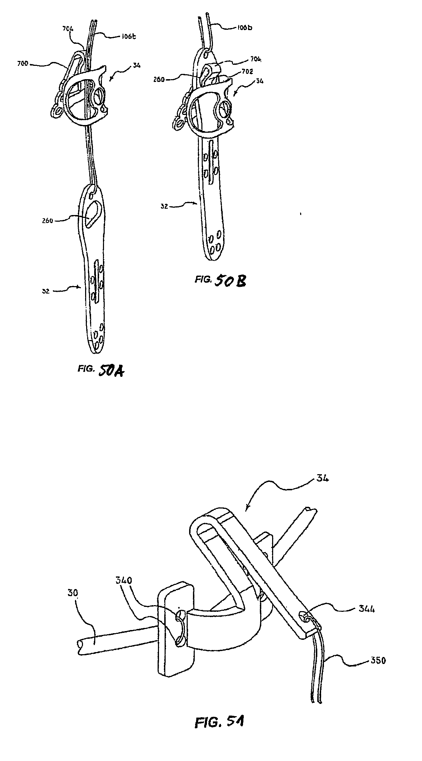

Referring now to Figure 29, a detail view of a variation of post 32 is described. In Figure 51, post 32 illustratively comprises actuator attachment element 250 for attaching the post to an actuator 106b; post lock element 252, illustratively a slot, for interlocking post 32 with an anchor lock element 34; valve attachment structure 254, comprising slot 255 and a plurality of holes 256, for attaching replacement valve 20 to the post (a tab of the valve may be passed through slot 255, then sewn to the back of the post through holes 256); and braid attachment element 258 for attaching the post to a distal region of anchor 30. The braid of anchor 30 may, for example, be interwoven through braid attachment element 258. Post 32 may be fabricated from a variety of materials, e.g., metallic materials such as stainless steel, and maybe laser cut, die cast, etc. In this variation of post 32, valve 20 is disposed distal of lock element 252. In alternative variations, the valve may be attached to the post proximal of the lock element or in-line with the lock element (i.e., neither proximal nor distal to the lock).

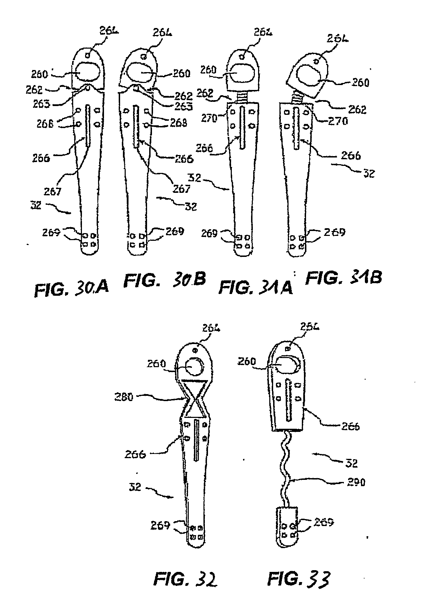

Figures 30 provide an alternative variation of post 32. In Figures 30, post 32 comprises lock element 260 having lock alignment feature 262, illustratively hinge 263. Hinge 263 allows lock element 260 to rotate from a position in line with post 32, as in Figure 30A, to a position out of alignment with the post, as in Figure 30B, thereby facilitating alignment with an anchor lock element 34. As shown, post 32 further comprises actuator attachment element 264, illustratively an eyelet, valve support structure 266 having slot 267 and a plurality of holes 268, and braid attachment element 269.

Figures 31 illustrate an alternative variation of lock alignment feature 262 comprising spring 270. As with hinge 263, spring 270 facilitates alignment of post lock element 260 with an anchor lock element 34 by allowing the post lock element to rotate from a position in line with post 32, as in Figure 31A, to a position out of alignment with the post, as in Figure 31B. Spring 270 also applies a restoring force that urges post lock element 260 back into alignment with post 32. Furthermore, spring 270 may facilitate dynamic elongation of post 32 in response to axial tension. This elongation may facilitate axial lengthening of anchor 30 in response to radially inward compression applied to the anchor.

With reference to Figure 32, another variation of post 32 is provided comprising expansion zone 280, which may, for example, comprise a laser cut feature along post 32. Expansion zone 280 facilitates dynamic elongation of post 32 in response to axial tension applied to the post, which facilitates axial lengthening of anchor 30 in response to radially inward compression applied to the anchor.

Figure 33 illustrates an alternative expansile element 290 comprising a curved wire or rod that may be elongated and straightened through application of axial tension to facilitate axial lengthening of the anchor in response to radially inward compression applied to the anchor (and thereby axial tension applied to post 32 via interaction between post lock element 260 and an anchor lock element 34). Element 290 additionally or alternatively may serve as a lock alignment feature. In such a configuration, element 290 optionally may not be expansile. More generally, post 32 may comprise proximal and distal ends connected by a tensile member.

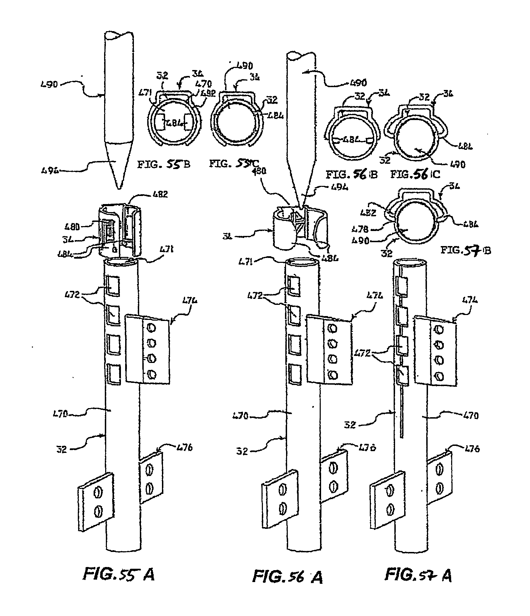

Figures 34 illustrate another variation of post 32 having another alternative lock alignment feature 262. In Figures 34, actuator 106b applies a proximally-directed force which brings post lock element 260 and anchor lock element 34 proximate to one another allowing the system to lock. Anchor lock element 34 defines a lock width Wi. In this embodiment, lock alignment feature 262 comprises post lock element lock area or width W2 that is substantially wider than the lock width Wi, for example, at least about twice as wide. This increased width enhances the probability of interlocking the post and anchor lock elements, even at sharply misaligned angles. In Figures 56, post 32 and anchor lock element 34 are disposed at an illustrative misalignment angle of about 10°.

Referring now to Figure 35, the variation of post 32 of Figure 29 is shown in combination with an illustrative actuator 106b and release actuator 112. In Figure 35, actuator 106b illustratively comprises rod 300 having post attachment element 302 that mates with actuator attachment element 250 of post 32. Angled camming surfaces 304 and 305 of post attachment element 302 and actuator attachment element 250, respectively, form an interface between post attachment element 302 and actuator attachment element 250. Proximal movement of actuator 106b with respect to post 32 is translated by the camming surfaces into a lateral force between the two elements that acts to separate and release post 32 from actuator 106b. Release actuator 112, illustratively tube 310, may be advanced over actuator 300 to cover the camming surface interface of the post and the actuator 106b, thereby forming a releasable attachment mechanism for securing the post to the actuator even during application of axial tension to the actuator. To separate post 32 from actuator 106b, e.g., after expansion and locking of anchor 30, release actuator 112 may be retracted relative to actuator 106b to the position shown in Figure 35, thereby removing a constraint from camming surfaces 304 and 305 and allowing the post and actuator to be pulled apart. Release actuator 112 preferably is retracted less than about 2,54 cm [1 inch] relative to the actuator 106b in order to actuate the releasable attachment mechanism, e.g., to remove constraint from camming surfaces 304 and 305.

Referring now to Figures 36, an alternative releasable attachment mechanism for attaching a variation of post 32 to a variation of actuator 106b is described.In Figures 36A and 36B, post 32 having actuator attachment element 320, illustratively an enlarged proximal opening within the post, is interference fit with post attachment element 330 of actuator 106b, illustratively an enlarged bulb, knob or other distal protrusion of the actuator. The slope of element 330 provides a camming surface that interfaces with an inside surface of opening 320. The angle of the camming interface between element 330 and opening 320 translates proximal movement of actuator 106b with respect to post 32 into a lateral movement between actuator 106b and post 32, thereby separating these elements. Release actuator 112, illustratively tube 310, covers the interference fit releasable attachment mechanism to preclude lateral movement of the post attachment element relative to the actuator attachment element, thereby releasably attaching the post to the actuator 106b. In Figure 36C, tube 310 is retracted relative to the post and actuator, which permits lateral movement between the post and actuator attachment elements, thereby separating actuator 106b from post 32. If tube 310 has not been refracted, of course, proximal movement of actuator 106b moves post 32 and the distal portion of the anchor proximally.

Figures 37 illustrate a variation of the releasable attachment mechanism of Figures 36. In the variation of Figures 37, actuator attachment element 320 of post 32 is deformable from a substantially round profile to an oval or "figure eight" profile by advancement of release actuator 112 over the attachment element. This forms a releasable attachment mechanism.In the deformed profile of Figures 37A and 37B, post attachment element 330 of actuator 106b is interference fit with the deformed actuator attachment element of post 32. In Figure 37C, retraction of release actuator 112 relative to the post and actuator allows actuator attachment element 320 to resiliently resume its un-deformed or at-rest configuration, thereby permitting separation of post 32 from actuator 106b. Actuator attachment element 320 may, for example, be fabricated from a shape memory material, such as Nitinol. A camming surface 331 on post attachment element 330 and a corresponding surface on the inner portion of element 320 translate proximal movement of actuator 106b with respect to post 32 into lateral movement of element 330 with respect to element 320 when release actuator 112 has been retracted.

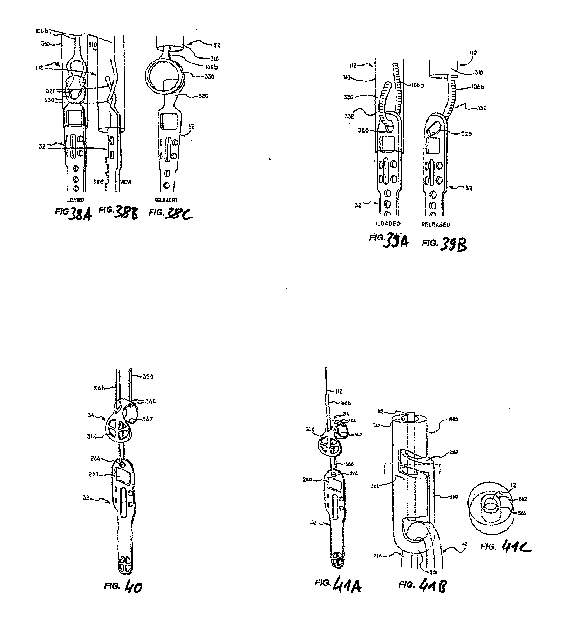

In the variation of Figures 38, post attachment element 330 is deformable (as in Figures 37A and 37B), and anchor attachment element 320 may be interference fit with the post attachment element. Figure 38C shows the post attachment element 330 in its at-rest configuration after tube 310 has been retracted, thereby releasing anchor attachment element 320. As will be apparent, for many or all of the two-part locking or attachment element elements described herein, the position of the elements may be reversed.

In Figures 39, post attachment element 330 comprises wrap portion 332 that may be inserted through anchor attachment element 320, illustratively an eyelet, wrapped backwards, then covered with release actuator tube 3 10 to constrain the wrap portion 332 in the wrapped configuration, as in Figure 39A. Release actuator tube 310 may be retracted relative to the wrap portion to resiliently or dynamically (e.g., by retracting actuator 106b relative to post 32) reshape the wrap portion to a substantially straight configuration for releasing the attachment between the post and the actuator, as in Figure 39B. Wrap portion 332 preferably is fabricated from a shape memory material, such as Nitinol, or a resilient material, such as spring steel.

Figure 40 shows another variation of the post, actuator and anchor lock element. In Figure 40, post 32 comprises post lock element 260 and actuator attachment element 264, illustratively an eyelet, through which actuator 106b is reversibly disposed. Anchor lock element 34 illustratively comprises a buckle, which may, for example, be formed from a cut tube or a bent resilient material. Anchor lock element 34 comprises anchor or braid attachment element 340 for attaching the buckle to anchor 30, and tab 342 for interlocking the buckle with post lock element 260, which illustratively is a slot formed through post 32. Actuator 106b therefore actuates the post (and therefore the distal end of the anchor to which the post is attached) as well as the anchor lock. Actuator 106b may be released from the post (and therefore from the anchor) by pulling one end of the control wire proximally to draw the control wire through and out of opening 264.

Anchor lock element 34 also comprises optional unlock actuator attachment 344, illustratively a pair of eyelets, through which unlock actuator 350 is releasably coupled to anchor lock element 34. Unlock actuator 350 illustratively comprises a control wire. Upon locking of tab 342 of buckle 34 within slot 260 of post 32, a proximally-directed force applied to unlock actuator 350 may remove the tab from the slot, thereby unlocking buckle 34 and post 32 and permitting the anchor to contract and elongate. Unlocking may be utilized, for example, to reposition or retrieve the anchor and valve apparatus even after the apparatus has been locked in the fully deployed configuration, as described previously with respect to Figures 5.

Figures 41 show another variation of the actuator, the lock actuator and the release actuator. As with other anchor lock elements, anchor lock element 34 in this embodiment is attached to a proximal end of the anchor, and the distal end of post 32 is attached to a distal end of the anchor. The anchor is not shown in Figures 41 for ease of illustration. For the purposes of illustration, the unlock actuator also is not shown in Figures 41.

As shown, actuator 106b actuates both post 32 (and therefore the distal end of the anchor to which the post is attached) and the lock formed between post lock element 260 and anchor lock element 34. In Figure 41A, release actuator 112 passes through actuator 106b to actuate the releasable attachment mechanism between post 32 and actuator 106b. Figure 41B provides a detail view of the releasable attachment mechanism. Actuator 106b comprises wrap portion 360 that passes through actuator attachment element 264 and wraps around the end of post 32. Wrap portion 360 may comprise a shape memory material, such as Nitinol, or a deformable material, e.g., a resiliently deformable material.

Wrap portion 360 further comprises first opening 362 for engaging release actuator 112, illustratively a wire or rod that passes through lumen Lu of actuator 106b. The walls of the lumen act a linear bearing and/or motion guide during advancement and retraction of the release actuator relative to the actuator. Actuator 106b also comprises second opening 364, which may be aligned with first opening 362 to engage release actuator 112, as shown. As seen in the cross-sectional view of Figure 41C, wrap portion 360, and especially the curved portion 361 of the wrap portion, acts as a spring element that urges the first opening out of alignment with the second opening. In this manner, release actuator 112 may be interference or friction fit through first opening 362 and second opening 364. Retraction of the release actuator proximal of the first and second openings may actuate the releasable attachment mechanism to resiliently or dynamically unwrap portion 360 and release actuator 106b from post 32. Wrap and/or curved portion 360/361 of actuator 106b illustratively is disposed at a distal end of the actuator.

As will be apparent to those of skill in the art, the releasable attachment mechanism of Figures 41 may also be utilized to attach a actuator 106a to a braided anchor 30. More generally, wrap portion 360 provides an illustrative first shape on an anchor actuation element 106 that is adapted to mate with a second shape on a post or anchor actuator attachment element (such as element 264 in Figures 41, or a wire of the braid of anchor 30) to substantially prevent relative distal or proximal movement between the anchor actuation element and the anchor. The apparatus further comprises a release actuator adapted to actuate the releasable attachment mechanism. The release actuator is adapted to be moved to permit relative movement between the first shape and the second shape. This relative movement may change the first shape and/or the second shape to a third shape that permits relative distal or proximal movement between the anchor actuation element and the anchor or post. Furthermore, this relative movement may separate the anchor actuation element from the anchor or actuator attachment element.

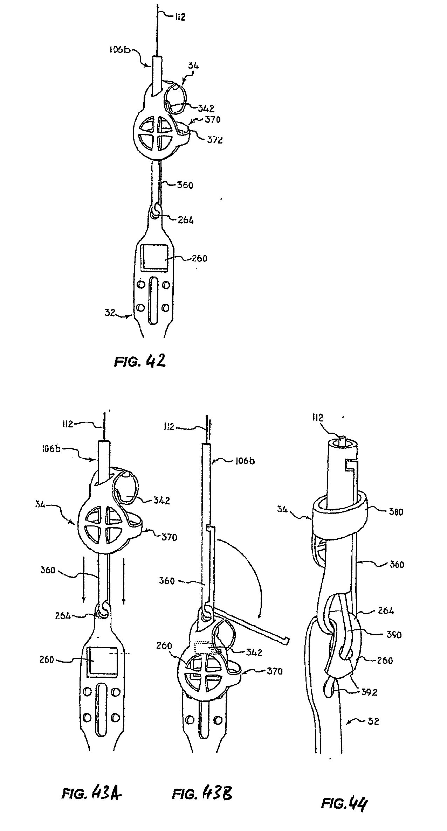

Figure 42 illustrates a variation of the anchor lock element of Figures 41. In Figure 42, anchor lock element 34 comprises lock alignment feature 370. Feature 370 comprises engagement portion 372, illustratively a loop, that is adapted to engage post 32 before engagement of anchor lock element 34 (i.e., before engagement of tab 342 of the anchor lock element) with post lock element 260. Feature 370 ensures alignment of the post and buckle prior to locking. Furthermore, feature 370 adds additional strength to anchor lock element 34 and opposes inwardly-directed forces applied to element 34 when valve 20 of apparatus 10 closes during diastole.

Referring now to Figures 43, actuation of the apparatus of Figure 42 is described. As seen in Figure 43A, anchor lock element 34 is advanced distally relative to post 32, for example, by applying a distally-directed force to the anchor via anchor actuator 106a to move the proximal portion of the anchor distally while maintaining the position of post 32 via actuator 106b. Alternatively or additionally, a proximally-directed force may be applied to post 32 via actuator 106b while maintaining the position of the proximal end of the anchor to move the distal portion of the anchor proximally. Lock alignment feature 370 engages the proximal end of the post prior to interlocking of tab 342 of anchor lock element 34 with post lock element 260, thereby ensuring proper alignment. Continued retraction of post 32 relative to buckle 34 locks the post into the buckle, as shown in Figure 43B. This also expands apparatus 10 to the fully deployed configuration of, e.g., Figures 27B and 28C. Next, release actuator 112 is retracted proximally relative to actuator 106b, which causes wrap portion 360 of the actuator to resiliently or dynamically swing outwards, thereby bringing first opening 362 and second opening 364 out of alignment. Proximal retraction of actuator 106b relative to post 32 removes wrap portion 360 from actuator attachment element 264 of post 32.

Figure 44 shows a variation of the apparatus of Figures 42 and 43. In Figure 44, anchor lock element 34 comprises locking hoop 380, while post lock element 260 comprises a wrapped or curved proximal end of post 32. The curved proximal end also forms actuator attachment element 264. Wrap portion 360 of actuator 106b is wrapped about the curved end of post 32. Release actuator 112, passing through first opening 362 and second opening 364 of actuator 106b, releasably secures this attachment. The release actuator further comprises kink 390 that facilitates passage of the actuator through release actuator attachment elements 392 of post 32, illustratively eyelets. When disposed through elements 392, release actuator 112 further acts as a lock prevention mechanism that precludes locking of the curved proximal end of post 32 with hoop 380 of anchor lock element 34.

In use, the proximal end of post 32 may be retracted through hoop 380 of anchor lock element 34. Release actuator 112 then may be refracted relative to anchor actuator 106b and post 32, such that the release actuator is disposed proximal of attachment elements 392 of the post. Next, post 32 may be allowed to distally advance until its curved proximal end catches and locks against hoop 380 of element 34. Continued retraction of release actuator 112 relative to actuator 106b facilitates separation of the actuator from the post, as described previously.

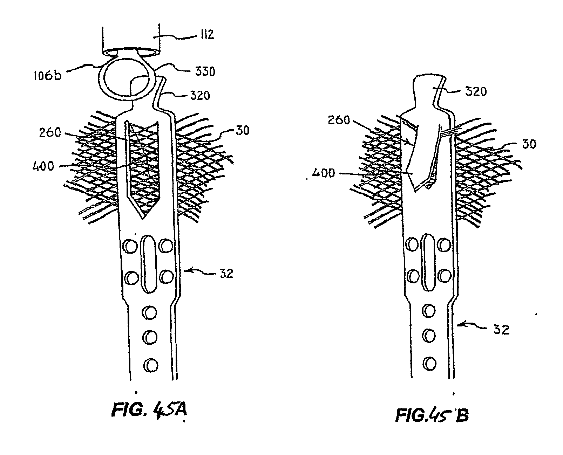

Referring now to Figure 45, an embodiment of post 32 is described that is configured to lock against the braid of anchor 30, as opposed to a separate anchor lock element 34. Post lock element 260 illustratively comprises bent tab 400 that catches against the anchor braid to lock the anchor in a deployed configuration.

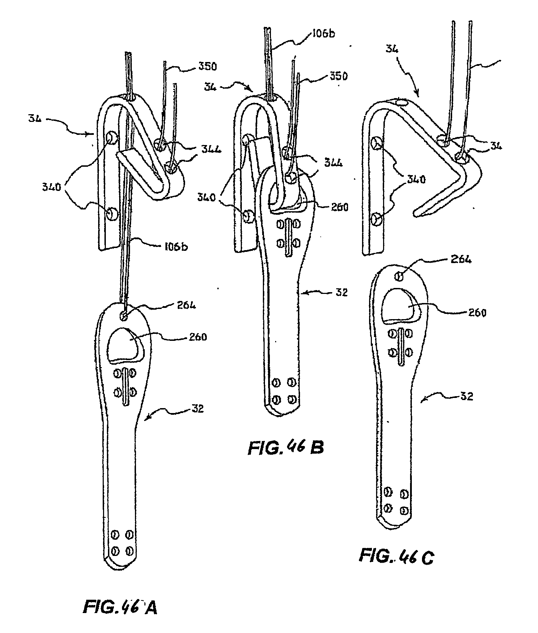

Figures 46 illustrate locking and unlocking of a variation of anchor lock element 34. Anchor lock element 34 of Figures 46 is similar to the buckle variation of element 34 described previously with respect to Figures 40 and 41. However, the variation of Figures 46 is fabricated from a strip of material that is bent to form a wrapped or curved portion. Figure 46A illustrates the apparatus prior to locking, Figure 46B illustrates the locked configuration, and Figure 46C illustrates unlocking through application of a proximally-directed unlocking force to unlock actuator 350.

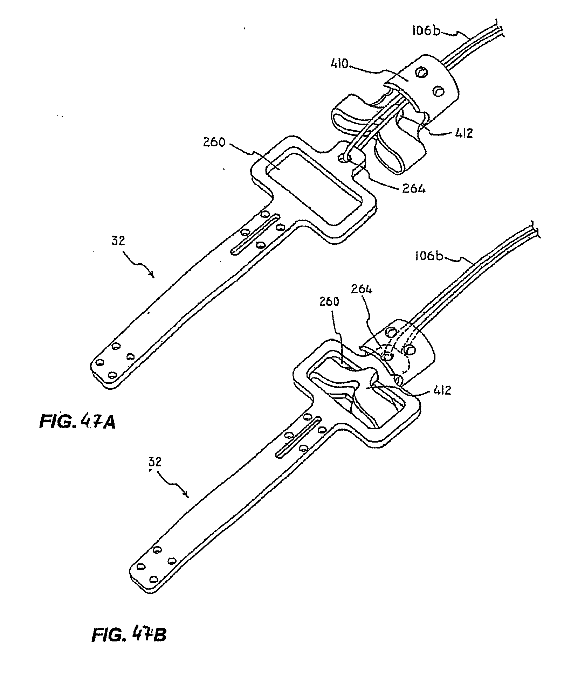

Figures 47 show yet another embodiment of a releasable actuation mechanism. Anchor lock element 34 comprises lock alignment mechanism 410 disposed proximal of locking tab 412. As shown, lock alignment mechanism 410 engages the distal end of post 32 to align the post and the anchor lock element prior to locking of post lock element 260 with tab 412 of anchor lock element 34. Lock alignment mechanism 410 adds additional strength to anchor lock element 34 and opposes inwardly-directed forces applied to element 34 when valve 20 of apparatus 10 closes during diastole. Advantageously, the inwardly-directed forces act to maintain apparatus 10 in the locked configuration. Mechanism 410 optionally may be formed from a cut tube.

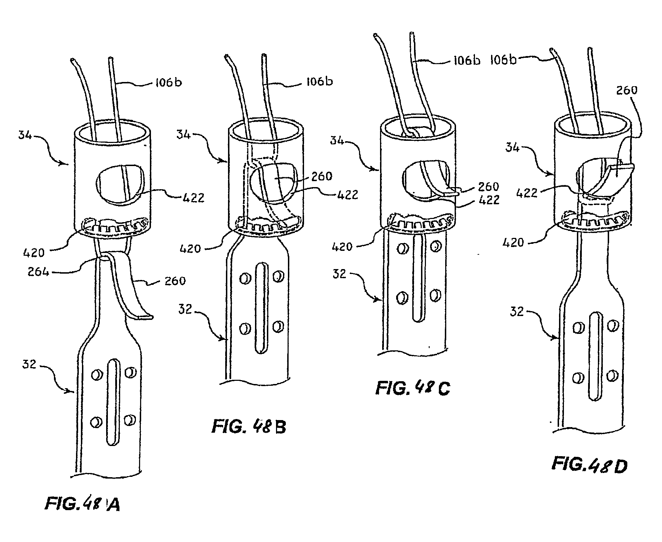

Figures 48 illustrate a variation of anchor lock element 34 that may be formed from a cut tube. As seen in Figures 48A and 48B, element 34 comprises tabs 420 for engaging the curved proximal end of post 32 that forms post locking element 260. In order to lock the post to element 34, the curved distal end of the post is retracted proximally of tabs 420 by the action of proximal tension on post 32 by actuator 106b while element 34 is held stationary, as described above. As it enters anchor lock element 34, the curved end of the post is cammed inward by the engagement of the distal edge of element 34 with the outer surface of the curved end. Once proximal of tabs 420, the curved end of the post moves outward, thereby locking the apparatus and preventing subsequent distal movement of post 32 with respect to element 34. To unlock the apparatus, the curved portion of the post is drawn further proximally by actuator 106b until the tip of the curved portion moves into an opening 422 formed in element 34. As seen in Figures 48C and 48D, resilient distal advancement of the post relative to element 34, e.g., via resilient expansion of the braid of anchor 30, deforms and straightens the curved proximal end of post 32 through a camming engagement of the underside of the curved portion of the post with the inner surface of opening 422, thereby allowing actuator 106b to slide off of post 32, unlocking apparatus 10. The curved portion of post 32 optionally may be formed from a shape memory material, such that the post resumes its curved profile for subsequent relocking after unlocking.

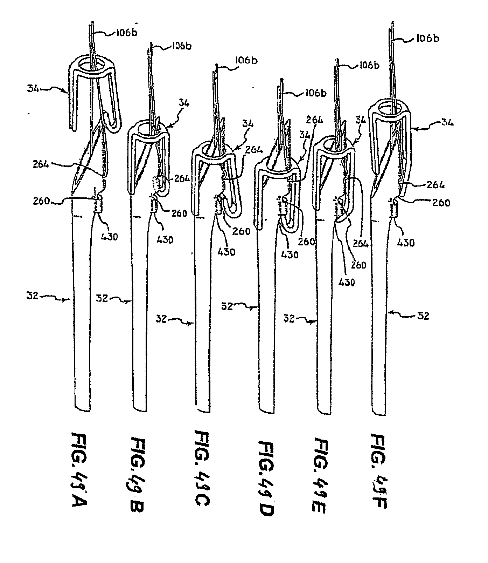

Figures 49 illustrate a variation of post 32 and anchor lock element 32. Anchor lock element 34 illustratively comprises a curved portion 35 that engages and enters the slot of post lock element 260 to lock the anchor as post 32 is drawn proximally into element 34 by actuator 106b. After locking, continued proximal retraction of post 32 by actuator 106b engages the distal end of the curved portion of element 34 with a camming surface 430 of post 32. Resilient distal advancement of post 32 (such as by the resilient contraction and elongation of the anchor to its at-rest configuration) then deforms and straightens the wrapped end of element 34, thereby permitting anchor lock element 34 to separate from post 32, unlocking the apparatus.

Figures 50 and 51 illustrate additional buckle variations of anchor lock element 34. Proximal movement of post 32 into anchor lock element 34 (by, e.g., actuator 106b) engages a bottom surface 702 of a curved portion 700 of element 34 with the proximal end of post 32. Further proximal movement of post 32 with respect to element 34 cams curved portion 700 forward until the curved end 704 of curved portion 700 meets and resiliently moves into opening 260 in post 32, locking the apparatus. The variation of Figures 51 illustrates attachment to the braid of anchor 30 via sutures or the like passed through openings 340 in element 34. The lock is unlockable via unlock actuator 350.

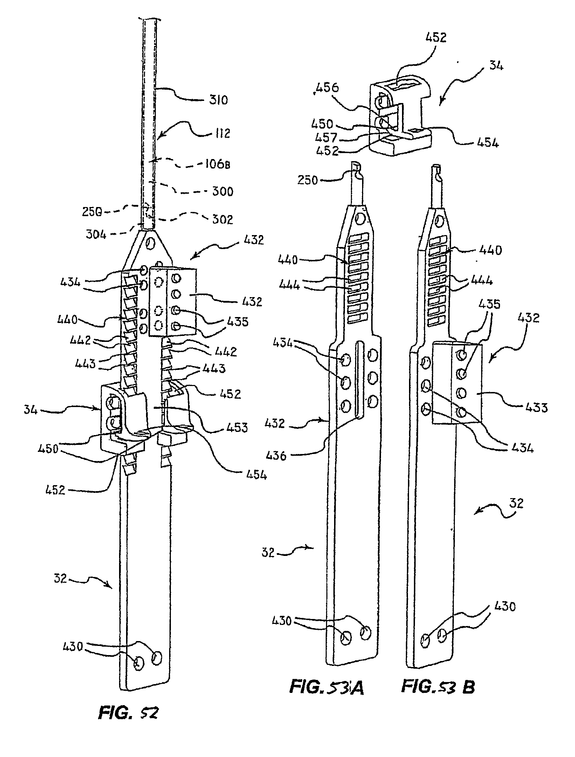

Referring now to Figure 52, an embodiment of a post 32 and anchor lock element 34 with a ratcheting lock is described. Post 32 comprises previously described actuator attachment element 250 that is releasably secured to post attachment element 302 of actuator 106b. (Other releasable attachment mechanisms may alternatively be used.) Post 32 also comprises braid attachment element 430 and valve attachment structure 432. In the variation of Figure 52, valve attachment structure 432 comprises tab 433 that extends from post 32, as well as a plurality of holes 434 through post 32 and a plurality of holes 435 through tab 433. Replacement valve 20 may be attached to post 32 by sewing the valve to the valve attachment structure through holes 434 and/or 435.

Post 32 further comprises ratcheting locking element 440 having a plurality of inclined planes with camming surfaces 442 and friction surfaces 443. The inclined planes are disposed along either side of tab 433 for ratcheting and locking against ratcheting anchor lock element 34. Anchor lock element 34 comprises ratchet teeth 450 on either side of the valve attachment elements that cam against surface 442 and lock against friction surfaces 443 of element 440 of post 32, as post 32 is proximally retracted through element 34. Advantageously, providing multiple rows of inclined plane ratchets along post 32 facilitates interlocking of the post and the element at multiple discrete locations.

Element 34 comprises proximal and distal slots 452 that receive post 32, as well as central longitudinal slot 453 that facilitate passage of tab 433 (and thereby valve 20) therethrough. Actuator 106b may be disposed through slots 452 prior to approximation and locking of the post to anchor lock element 34 in order to facilitate alignment of the post and the anchor lock element. Element 34 may be ratcheted to any position along ratchet lock element 440 to achieve any desired locking configuration and degree of expansion of apparatus 10. Valve attachment structure 432, and thereby replacement valve 20, may be positioned proximal of the ratchet lock post-deployment or in line with the ratchet lock (i.e., neither proximal nor distal to the ratchet lock). Element 34 further comprises unlock actuator attachment(s) 454 for coupling the element to an unlock actuator, e.g., previously described unlock actuator 350, to unlock element 34 by applying a proximally-directed unlocking force that displaces ratchet teeth 450 from friction surfaces 443.

Figures 53 illustrate variations of the apparatus of Figure 52. Ratchet lock elements 440 of posts 32 in Figures 53 comprise a plurality of ratchet slots 444 in which ratchet tooth 450 of anchor lock element 34 may be locked. Ratchet tooth 450 comprises proximal friction surface 456 and distal camming surface 457 to facilitate proximal retraction of a post 32 through slot 452 for ratcheting of camming surface 457 through ratchet slots 444, but to preclude distal advancement of the post once ratchet tooth 450 is engaged within ratchet slots 444 by locking a ratchet slot against friction surface 456. As with the variation of Figure 52, anchor lock element 34 is unlockable and comprises unlock actuator attachment 454. In contrast to the variation of Figure 52, the ratchet lock is disposed proximally of valve attachment structure 432, and thereby proximally of replacement valve 20. In Figure 53A, valve attachment structure 432 comprises slot 436 instead of tab 433.

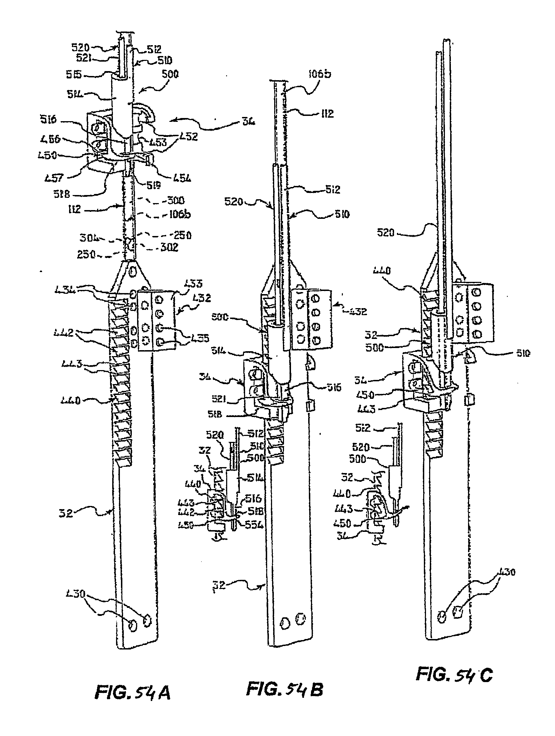

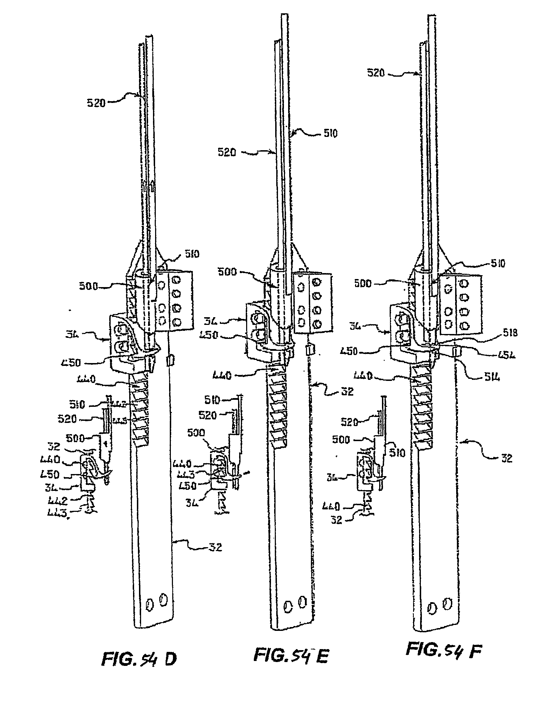

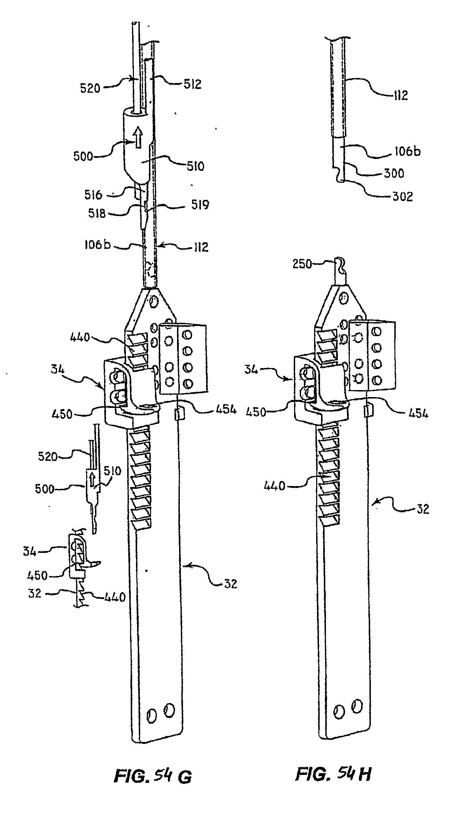

Figures 54 illustrate another variation of the ratchet lock of Figure 52. In Figures 54, ratchet lock elements 440 of post 32 extend along only one edge of the post. Thus, anchor lock element 34 comprises unitary ratchet tooth 450 for camming against surfaces 442 and locking against friction surfaces 443 of elements 440 of post 32, as post 32 is proximally refracted through element 34.

The apparatus of Figures 54 also comprises unlock or adjustment actuator 500 that is releasably attached to anchor lock element 34 along unlock actuator attachment 454. Actuator 500 comprises two independently or concurrently actuable elements: adjustment element 510 and release element 520. Adjustment element 510 comprises elongated member 512 having protrusion 514 with lumen 515, as well as distal extension 516 with notch 518 having optional camming surface 519. Release element 520 comprises elongated member 521, which may, for example, comprise a mandrel, that is configured for passage through lumen 515 of protrusion 514 of adjustment element 510. Elongated members 512 and 521 of actuator 500 preferably extend through delivery system 100 to the exterior of the patient for independent or concurrent advancement and/or retraction by a medical practitioner.

As seen in Figure 54A, notch 518 of adjustment element 510 of actuator 500 maybe positioned within unlock actuator attachment 454 of anchor lock element 34 during deployment of apparatus 10. As seen in Figure 54B, anchor lock element 34 is locked within ratcheting lock elements 440 of post 32 by proximally retracting actuator 106b relative to anchor lock element 34. Release element 520 then may be advanced relative to adjustment element 510 to position elongated member 521 within unlock actuator attachment 454 adjacent distal extension 516 of adjustment element 510. This serves to friction lock or interference fit actuator 500 within attachment 454 along notch 518 of adjustment element 510. Thus, concurrent advancement and/or retraction of the adjustment and release elements of actuator 500 by a medical practitioner causes anchor lock element 34 to move in unison with actuator 500. As will be apparent, actuator 500 alternatively may be friction locked with anchor lock element 34 prior to full deployment of apparatus 10. Furthermore, actuator(s) 500 may assist, or be used in place of, actuators 106a to deploy apparatus 10.

As seen in Figure 54C, the lock formed between anchor lock element 34 and post 32 may be unlocked or adjusted, as desired, by applying a lateral unlocking force to ratchet tooth 450 via actuator 500 that pulls the ratchet tooth away from a friction surface 443 of ratcheting lock elements 440. Actuator 500 then may be distally advanced or, as seen in Figure 54D, proximally retracted relative to ratcheting lock elements 440 and post 32 to further expand or partially collapse anchor 30, respectively (further expansion alternatively may be achieved by further ratcheting ratchet tooth 450 along camming surface 442 of ratcheting lock elements 440, e.g., by further proximally retracting actuator 106b, which is not shown in Figures 54C-54F for the sake of clarity). Anchor actuation elements 106 may assist such controlled expansion or collapse anchor 30.

When (re-)positioned at a desired location and/or when a desired degree of locking has been achieved, the lateral unlocking force may be removed from ratchet tooth 450 to again lock anchor lock element 34 to post 32 along ratcheting lock elements 440, as in Figure 54E. To complete deployment of apparatus 10, adjustment actuator 500 and actuator 106b, as well as actuator 106a (not shown), may be separated from the apparatus. In Figure 54F, release element 520 of actuator 500 is proximally retracted relative to adjustment element 510, thereby removing elongated member 521 of release element 520 from unlock actuator attachment 454 of anchor lock element 34. This removes the interference fit between notch 518 and attachment 454. Proximal retraction of actuator 500 relative to anchor lock element 34 detaches adjustment element 510 of actuator 500 from attachment 454 of anchor lock element 34, as in Figure 54G. Optional camming surface 519 along notch 518 may facilitate such detachment. In Figure 54H, actuator 106b is detached from post 32 by retracting release actuator 112 relative to the actuator, as described previously.

Referring now to Figures 55, another variation of an adjustable ratcheting lock element is described. As seen in Figure 55A, post 32 comprises tube 470 having lumen 471 and ratcheting lock element 472, illustratively a plurality of slots that communicate with lumen 471. Post 32 also comprises valve support structure or attachment element 474 and braid attachment element 476.

Anchor lock element 34, which may be fabricated from a cut tube, comprises a substantially cylindrical structure having braid attachment element 480, lumen 482 and tabs 484. As seen in the top view of Figure 55B, tabs 484 of anchor lock element 34 are configured for locking within the slots of ratcheting lock element 472 of post 32. As seen in the top view of Figure 55C, adjustment actuator 490, illustratively mandrel M having tapered distal end 494 that acts as a camming surface, may be advanced through lumen 481 of anchor lock element 34 and lumen 471 of tube 470 of post 32, to displace tabs 484 from the locking slots of post 32, thereby unlocking the post from the anchor lock element. This facilitates, for example, readjustment of a degree of locking/expansion of apparatus 10, repositioning of apparatus 10, retrieval of apparatus 10, etc.

Figures 56 illustrate a variation of anchor lock element 34 wherein tabs 484 are positioned along a different axis. This may provide a more secure lock between post 32 and anchor lock element 34. Figures 57 illustrate a variation of post 32 configured for use with the variation of anchor lock element 34. In Figures 57, post 32 comprises groove 478 that connects the slots of ratcheting lock element 472. Groove 478 does not communicate with lumen 471 of tube 470 of post 32. Rather, the groove may act as a lock alignment mechanism that guides tabs 484 of anchor lock element 34 along post 32 and ratcheting lock element 472, as seen in the top view of Figure 57B.

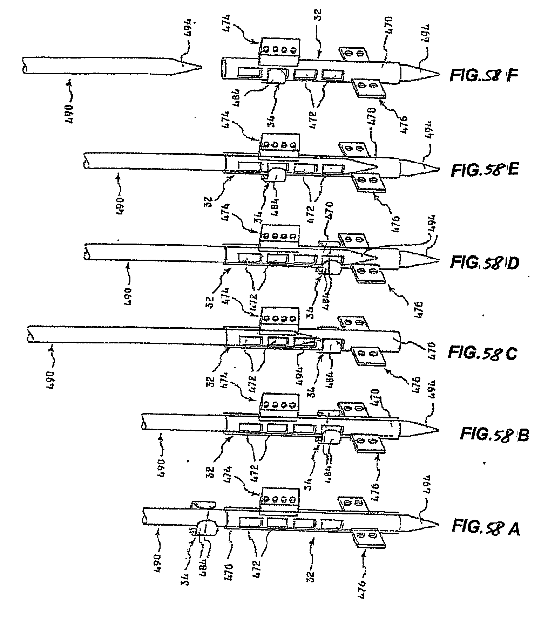

Referring now to Figures 58, a method of actuating the variation of Figures 56 is described. As seen in Figure 58A, adjustment actuator 490 is initially disposed through lumen 482 of anchor lock element 34 and within lumen 471 of post 32. Post 32 then may be proximally retracted relative to anchor lock element 34, e.g., via actuator 106b (not shown). In Figure 58B, actuator 490 serves as a lock prevention mechanism that precludes locking of tabs 484 within ratcheting lock element 472. In Figure 58C, actuator 490 is retracted relative to post 32 and anchor lock element 34, which opens up lumen 471 of tube 470 and allows tabs 484 to pass through the slots of ratcheting lock element 472, thereby locking the post to the anchor lock element. In Figure 58D, actuator 490 is re-advanced within lumen 471, such that tapered distal end 494 of mandrel M serves as a camming surface that urges tabs 484 out of lumen 471 as the actuator is advanced. This unlocks the post from the anchor lock element to facilitate adjustment, repositioning or retrieval of apparatus 10. In Figure 58E, a degree of locking/expansion of the apparatus is adjusted by repositioning anchor lock element 34 relative to post 32, and thereby tabs 484 relative to ratcheting lock element 472. When properly adjusted, actuator 490 may be removed from lumen 471 of tube 470 of post 32, as in Figure 58F. Tabs 484 resiliently return to the locked configuration within the slots of ratcheting lock element 472.

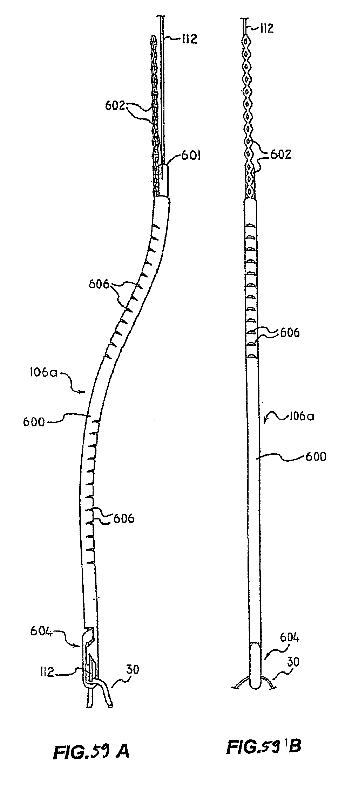

Referring now to Figures 59, an embodiment of anchor actuator 106a is described. Actuator 106a comprises elongated member 600 having proximal extension 602 that may be attached, for example, to previously described multi-lumen shaft or catheter 108 of delivery system/deployment tool 100 (see Figures 27), e.g., via epoxy, UV curing, etc. Lumen 601 extends through elongated member 600 from proximal extension 602 to releasable attachment mechanism 604. Releasable attachment mechanism 604 releasably attached actuator 106a to the braid of anchor 30. The mechanism comprises release actuator 112 and illustratively is similar to the previously described releasable attachment mechanism of Figures 41-43. Release actuator 112, illustratively a mandrel, passes through a lumen Lu of multi-lumen shaft 108 and then through lumen 601 of actuator 106a to mechanism 604.

Actuator 106a further comprises shaping features 606 that affect a shape of the anchor actuator when an anchor actuation force is applied to anchor 30. These features may comprise, for example, reduced diameter portions of the actuator, reduced wall thickness portions of the actuator and/or slits formed in the anchor actuator. Application of an anchor actuation force may, for example, provide actuator 106a with the profile seen in Figure 59A. This profile may facilitate expansion of anchor 30/apparatus 10. As will be apparent, shaping features may be provided with any anchor actuation elements 106, including any of the previously described variations of actuators 106b.

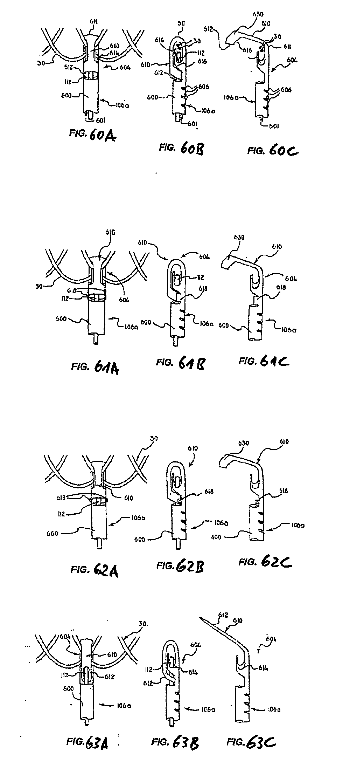

As seen in Figures 60, releasable attachment mechanism 604 comprises wrap portion 610 that may, for example, pass through the braid of anchor 30 and wrap around the proximal end of the anchor. Wrap portion 610 may comprise a shape memory material, such as Nitinol, or a deformable material, e.g., a resiliently deformable material. The wrap portion comprises first opening 612 for engaging release actuator 112. The walls of lumen 601 of elongated member 600 may act as a linear bearing and/or motion guide during advancement and refraction of the release actuator relative to the actuator. Actuator 106a also comprises second opening 614, which may be aligned with first opening 612 to engage release actuator 112, as shown. Wrap portion 610, and especially curved portion 611 of the wrap portion, acts as a spring element that urges the first opening out of alignment with the second opening to engage and hold release actuator 112 in place.

As seen in Figure 60C, when the release actuator is retracted proximally relative to the actuator, wrap portion 610 resiliently or dynamically swings outwards. Thereafter, proximal retraction of anchor actuator 106a relative to anchor 30 detaches wrap portion 610, and thereby actuator 106a, from the anchor. Surface 616 of wrap portion 610 may act as a camming surface as the inner surface of wrap portion 610 slides along the anchor braid 30 to facilitate such detachment.

In this manner, release actuator 112 may be interference or friction fit through first opening 612 and second opening 614. Retraction of the release actuator proximal of the first and second openings actuates releasable attachment mechanism 604 to resiliently or dynamically unwrap portion 610 and release actuator 106a from anchor 30. Wrap portion 610 of actuator 106a illustratively is disposed at a distal end of the actuator.

With reference to Figures 61, a variation of releasable attachment mechanism 604 is described. In Figures 61, wrap portion 610 illustratively comprises tabs 618 that act as an alignment mechanism for aligning the wrap portion of mechanism 604 with elongated member 600. This may facilitate advancement of release actuator 112 through mechanism 604.

Figures 62 illustrate a variation of tabs 618 wherein the tabs are rounded. This may reduce friction, provide an afraumatic surface, etc. Additional shapes for tabs 618 will be apparent. Alternatively, tabs 618 may act as spring elements which are loaded when element 630 is seated, as shown in Figure 62B. In this configuration tabs 618 apply a force directed towards element 630 such that 630 will be ejected when element 112 is refracted. In this way tabs 618 apply a restraining force on element 112 which reduces the risk of an early release.

Figures 63 illustrate a variation of wrap portion 610 that comprises a substantially straight distal region in an at-rest configuration, as seen in Figure 63C. It is expected that providing a substantially straight distal region along wrap portion 610 may facilitate detachment of actuator 106a from anchor 30, i.e., may reduce a risk of snagging the wrap portion along the braid of the anchor. The wrap portion may be resiliently deformed for passage of release actuator 112 through first opening 612, as in Figures 63A and 63B.

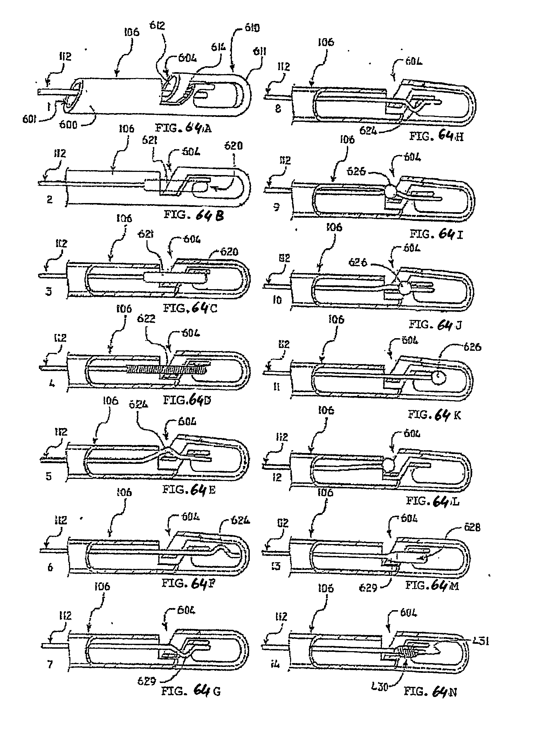

Referring now to Figures 64, variations of release actuator 112 for use with releasable attachment mechanism 604 are described. In Figure 64A, the release actuator comprises a simple mandrel. In Figures 64B and 64C, the release actuator comprises protrusion 620 having friction surface 621. In Figure 64D, actuator 112 comprises coil 622. In Figures 64E-64H, the actuator comprises kink 624, which may act as a camming surface, as shown. The kink may also provide tactile feedback to a medical practitioner. In Figures 64I and 64J, the release actuator comprises ball or knob 626 disposed proximal of the actuator's distal end. In Figures 64K and 64L, ball 626 is disposed at the distal end of actuator 112. The ball may act as a camming surface. In Figure 64M, actuator 112 comprises protrusion 628 having proximal camming surface 629. In Figure 64N, the actuator comprises oblong protrusion 430 having friction surface 431. Additional variations of actuator 112 will be apparent.

Referring now to Figures 65, an embodiment of delivery system/deployment tool 100 is described. Figure 65A provides a detail view of multi-lumen catheter 108 and sheath 110. As discussed previously catheter 108 comprises central lumen 109 and a plurality of circumferentially-disposed lumens Lu.

As seen in Figure 65B, actuator 106a is coupled to catheter 108 via proximal extension 602, such that lumen 601 is coaxially disposed within a lumen Lu of the catheter. Release actuator 112 extends through lumens Lu and 601. Actuator 106a is distally attached to the braid of anchor 30 along releasable attachment mechanism 604. For the sake of clarity, a single actuator 106a is shown in Figure 65B, but multiple such actuators preferably are provided, as in Figures 66 described hereinafter.

Figure 65B also illustrates actuator 106b. The actuator extends through a lumen Lu of catheter 108 and through anchor lock element 34 to post 32 (not shown). Unlock actuator 350 is also provided and extends through a lumen Lu to unlock actuator attachment 344 of anchor lock element 34. Anchor lock element 34 illustratively comprises the variation described previously with respect to Figures 46. The element is attached to the braid of anchor 30 along anchor, attachment elements 340. As with actuator 106a, a single anchor lock element 34 and actuator 106b are shown in Figure 65B. This is only for the sake of clarity, and multiple such actuators may be provided, e.g., three actuators.

Referring now to Figures 66, delivery system/deployment tool 100 is shown with a plurality of actuators 106a and actuators 106b for releasable attachment to anchor 30 of apparatus 10. In Figure 66A, anchor actuation elements 106a are coupled to the anchor. In Figure 66B, the elements are decoupled from the anchor.

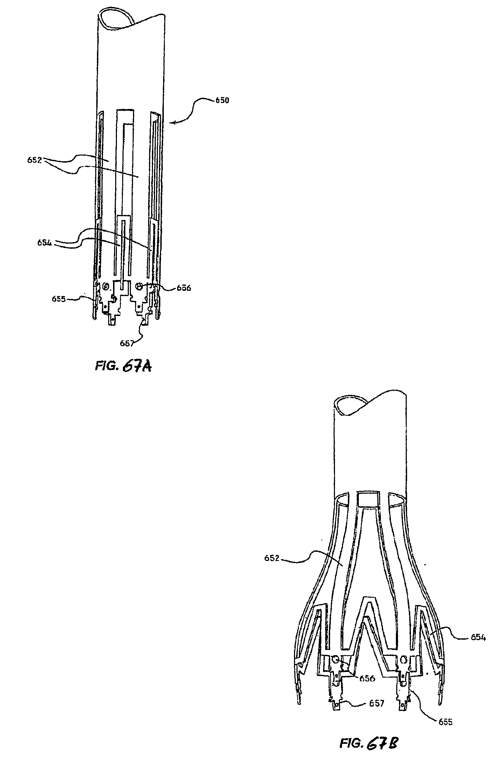

With reference now to Figures 67, a variation of the delivery system/deployment tool of Figures 65 and 66 is described comprising a plurality of arms or actuators that extend from a unitary structure. Unitary structure 650, which may extend from a distal region of multi-lumen shaft 108, is preferably fabricated from a laser-cut tube. Structure 650 comprises a plurality of circumferentially disposed arms 652 that serve as actuators. Expansile elements 654 may be disposed between arms 652 and facilitate constraint of the arms radially outward or inward with respect to other arms as the anchor reshapes. Figure 67A shows the arms in a radially collapsed configuration, and Figures 67B shows the arms in a radially expanded configuration. Wrap portions 655 are adapted to wrap around the proximal portion of an anchor braid. Openings 656 and 657 are formed in wrap portions 655 to engage a release actuator, as described in embodiments above.

Referring now to Figures 68, various ways to connect elements to the braid of anchor 30 of replacement valve apparatus 10 are described. In Figure 68A, a post 32 having a single braid attachment hole 660 is attached to anchor 30 along three separate intersections of the braid via suture S. Figure 68B provides a detail view of one exemplary technique for routing the suture between hole 660 and anchor 30. Figure 68C illustrates a variation of the attachment, wherein post 32 comprises multiple braid attachment holes 660. As will be apparent, elements other than posts 32 may be attached to anchor 30 in the manner described, for example, anchor lock elements 34 may be attached in a similar manner.

As described in more detail in U.S. Patent Appl. Ser. No. 10/746,280, the distal region of anchor 30 may be pulled proximally via a proximally directed force applied to posts 32 via a distal deployment system interface. The distal deployment system interface can be adapted to expand radially during application of a proximally directed force on the distal end of the anchor.

The distal deployment system interface may include control actuators that are controlled, e.g., by control knob 122 of control handle 120. Similarly, the proximal regions of anchor 30 may be pushed distally via a proximal deployment system interface at the proximal end of the anchor. The proximal deployment system interface is adapted to permit deployment system to apply a distally directed force to the proximal end of anchor 30 through, e.g., fingers 106, which are controlled by, e.g., Control knob 124 of control handle 120.

The proximal deployment system interface is further adapted to expand radially during application of a distally directed force on the proximal end of the anchor. Preferably, the proximal deployment system interface is adapted to permit deployment system to apply a distally directed force on the proximal end of the anchor system through a plurality of deployment system fingers or actuators 106. Such expansion optionally may be assisted via inflation of a balloon catheter (not shown) reversibly disposed within apparatus 10, as described in U.S. Patent Appl. Ser. No. 10/746,280.

Once anchor 30 is fully deployed, posts 32 and buckles 34 of anchor 30 may be used to lock and maintain the anchor in the deployed configuration. In one embodiment, the control actuators attached to posts 32 are threaded through buckles 34 so that the proximally directed force exerted on posts 32 by the control actuators during deployment pulls the proximal locking end of posts 32 toward and through buckles 34. Such lock optionally may be selectively reversible to allow for repositioning and/or retrieval of apparatus 10 during or post-deployment. Apparatus 10 may be repositioned or retrieved from the patient until the two-part locking mechanism of posts 32 and buckles 34 of anchor 30 have been actuated. When the lock is selectively reversible, the apparatus may be repositioned and/or retrieved as desired, e.g., even after actuation of the two-part locking mechanism. Once again, further details of this and other anchor locking structures may be found in U.S. Patent Appl. Ser. No. 10/746,280. Locking mechanisms used herein may also include a plurality of levels of locking wherein each level of locking results in a different amount of expansion. For example, the proximal end of the post can have multiple configurations for locking within the buckle wherein each configuration results in a different amount of anchor expansion.

Prior to implantation of replacement valve apparatus described herein, it may be desirable to perform a valvuloplasty on the patient's diseased valve by inserting a balloon into the valve and expanding it using, e.g., saline mixed with a contrast agent. In addition to preparing the valve site for implant, fluoroscopic viewing of the valvuloplasty will help determine the appropriate size of replacement valve implant to use.

Figures 28A-F show further details of anchor 30 of apparatus 10. Figure 28A shows the apparatus in a collapsed configuration, such as for delivery within a sheath or other lumen or for retrieval and recapture into a sheath or other lumen. Figures 28B and 28C show the anchor and valve in an expanded and locked configuration.

As shown in Figure 28C, anchor 30 has three posts and three buckles. As seen in Figure 28C, the three leaflets of replacement valve 20 may be coupled to the three posts 32 also known as valve supports. The posts, unlike the braid, do not collapse or expand.In some embodiments a post 32 has one or more proximal slots 33, at least one proximal hole 36a and at least one distal hole 36b. Leaflet tissue may be passed through slot 33 and sutured in place via suture routed through one or more proximal holes 36a. Other means known in the art for fixing valve leaflets to posts may also be employed.

Figure 69 illustrates an exemplary apparatus for fabricating braided anchors. Such apparatus includes a cylindrical braiding fixture 200. The cylindrical braiding fixture 200 comprises proximal circumference of inner posts 202a separated by a distance x from distal circumference of inner posts 202b. x can be, for example, 10 to 60 mm, more preferably 20 to 50 mm, or more preferably 30 to 40 mm. Optionally, the fixture may also comprise proximal and distal circumferences of outer posts 204a and 204b, respectively. 204a and 204b can be situated about 2-10 mm from 202a and 202b, respectively. Posts 202a/b and 204a/b project from fixture 200 and may be used to route wire, e.g., for forming anchor braid 30. Inner posts 202a and 202b generally facilitate formation of a braid, while outer posts 204a and 204b generally facilitate formation of desired features at the ends of the braid, as described hereinafter with respect to Figures 93-96.

In some embodiments, fixture 200 comprises approximately 6-20 posts, more preferably 8-18 posts, or more preferably 10-16 posts around its circumference, though any alternative number of posts may be provided. Likewise, fixture 200 preferably has a diameter of about 2-40 mm, more preferably 4-30 mm, or more preferably 6-20 mm, though any alternative diameter may be provided. The diameter of fixture 200 preferably is the diameter of the braid in its "at rest" configuration.

Fixture 200 can optionally further comprise circumferential grooves 206 to facilitate interweaving of a first section of wire underneath an adjacent section of wire. The fixture optionally also may comprise localized depressions or holes 208 in addition, or as an alternative, to grooves 206. Depressions 208 may be provided at locations where wire segments cross to act as a visual guide for formation of anchor braid 30, as well as to facilitate the interweaving of a first section of wire beneath an adjacent section of wire.

Referring now to Figures 70A-D, an illustrative method of using fixture 200 to fabricate braided anchors in accordance with the present invention is described. Figure 70A provides a detail view of a proximal front side region of fixture 200 during formation of a braided anchor. Figure 70B shows a detail backside view of a central section of the fixture. Figure 70C shows a full-length frontside view of the fixture and Figure 70D shows the completed braid. In Figures 70, anchor braid 30 is formed from a single strand of wrapped and interwoven wire W. However, it should be understood that anchor braid 30 alternatively may be formed from multiple strands of wire.

As seen in Figure 70A, formation of anchor braid 30 begins with wire W being routed from starting position P near the proximal end of fixture 200 past outer proximal posts 204a and inner proximal posts 202a. Wire W preferably is formed from a superelastic and/or shape-memory material, such as Nitinol. However, alternative wire materials may be utilized, including Cobalt-Chromium, Steel and combinations thereof, as well as additional materials that will be apparent to those of skill in the art.

After passing inner proximal posts 202a, wire W encircles fixture 200 in a helical spiral while extending towards the distal posts, as seen in Figures 70B and 70C. The wire illustratively encircles fixture 200 a full 360° revolution plus one additional post. However, any alternative degree of winding may be provided (e.g., a full 360° plus 2 additional posts, a full 360° plus 3 additional posts, o a number of posts less than a full 360°). As will be apparent to those of skill in the art, altering the degree of winding will alter the expansion characteristics of the resultant braid in ways per se known.

At distal inner posts 202b, wire W forms turn Tu and is rerouted back towards proximal imier posts 202a. It should be noted that wire W can form turn Tu in either inner posts 202 or outer posts 204. Turn Tu forms a closed end of the braid. Additional sets of inner and outer posts are also contemplated. The wire once again encircles fixture 200 in a full 360° helical revolution plus one additional post before reaching the proximal inner posts and being rerouted back towards the distal inner posts. This process is repeated with the wire repetitively interwoven at crossing locations between the proximal and distal posts, e.g., via grooves 206 and/or depressions 208, to define the cells of the braid that will provide anchor 30 with desired characteristics. As seen in Figure 70D, wire W turns both proximally and distally in order to complete formation of the braid.In this embodiment, wire W terminates in the central portion of the braid at T. Termination T may be formed, for example, by welding the wires together, applying a shrink tube about the overlap, using a crimp, braising the wires, etc. Additional techniques will be apparent to those of skill in the art.

When anchor braid 30 is formed from a shape-memory material, the braid may be heat set such that it maintains a desired degree of expansion in an at-rest configuration. The heat set at-rest configuration may comprise, for example, the delivery configuration (e.g., collapsed configuration) of Figure 28A, the deployed configuration (e.g., expanded configuration) of Figures 28B and 28C, or any desired configuration therebetween. In preferred embodiments, the anchor is heat-set in a configuration between the delivery configuration and the deployed configuration. Anchor braid 30 may be heat set while still disposed on fixture 200 to maintain an at-rest configuration as formed on the fixture, which preferably is a configuration between the delivery and deployed configurations. Alternatively, the braid may be heat set after complete or partial removal from the fixture. As yet another alternative, the braid may be initially heat set while still disposed on the fixture, but thereafter may be additionally heat set in a different shape, for example, a more expanded configuration. It is expected that heat setting anchor braid 30 will provide the braid with desired delivery and/or deployment characteristics.

Referring now to Figures 71A-71O, in conjunction with Figures 28C and 92, an anchor braid 30 may be defined by a set of cells that is different than other cells. Such cells may be formed to provide anchor braid 30 with one or more edge features (for either or both the distal and proximal ends). These edge features can, for example, reduce or relieve stress within the braid during delivery and deployment, which in turn may reduce the incidence of anchor material fatigue caused by the pulsatile anchor motion of the anchor site. As will be apparent to those of skill in the art, forming braid 31 from a single strand of wire W (or from multiple strands of wire W that form turns or that are joined together) may lead to stress concentration at turns Tu in the wire where the wire changes direction and extends back towards the opposite end of the braid. Such stress concentration may be most pronounced while the braid is disposed in its extreme configurations, i.e. when the braid is disposed in the collapsed delivery configuration of Figure 28A or the expanded deployed configuration of Figures 28B and 28C.

Stress concentration may increase the rigidity of an anchor braid and/or may impede delivery and deployment, as well as sheathing, of the braid. Thus, in preferred embodiments, a group of cells can be configured to reduce the sheathing force as described herein. Furthermore, to enhance deliverability, stress concentration may require that anchor braid 30 be fabricated from a relatively thin wire W. However, thin wire may not provide anchor braid 30 with adequate radial strength to displace a patient's diseased native heart valve leaflets and/or to anchor apparatus 10 against a patient's anatomy. Conversely, use of a relatively thick wire W may increase stiffness, thereby precluding retrograde delivery of apparatus 10, as well as a risk of kinking at turns in the braid. Thus, in some embodiments, wires varying in thickness may be used, or multiple wires having different thickness may be woven together. Also, wires made from different materials may be used to form an anchor braid.

It may be desirable to reduce stress concentration at the edges of anchor 30 where wire W changes direction and/or to reduce the circumferential stiffness of the anchor braid. The edge characteristics of the anchor may be altered by altering the shape of substantially all anchor braid cells at the anchor's edge (e.g., distal edge and/or proximal edge). Wire turns that control the shape of the edge cells may be formed within anchor braid 30 by routing wire W around optional outer posts 204 of fixture 200 during formation of the braid.

Figure 71A illusfrates a detail view of a standard end turn Tu in an anchor braid resulting in a braid with substantially uniform cell size and shape.

Figure 71B illustrates a turn that has been elongated to lengthen the distance over which forces concentrated in the turn may be distributed, resulting in an anchor braid having edge cells that are longer along the anchor axis than the other cells defined by the braid. This elongated turn feature may be formed by routing the wire of braid about outer posts 204 of fixture 200, and then heat setting the wire.

Figure 71C illustrates an alternative anchor edge cell configuration, wherein the tip of the elongated wire turn has been bent out of a cylindrical shape defined by the braid of anchor braid 30. This may be achieved, for example, via a combination of routing of wire W within fixture 200 and heat setting. The out-of-plane bend of turn Tu in the anchor edge cells in Figure 71C may reduce stress in some configurations, and may also provide a lip for engaging the patient's native valve leaflets to facilitate proper positioning of apparatus 10 during deployment.

In Figure 71D, a W-shaped turn feature has been formed at the wire turn, e.g., by routing the wire of anchor braid 30 about a central inner post 202 and two flanking outer posts 204 of fixture 200. As with the elongated braid cells of Figures 71B and 93C, the W-shape may better distribute stress about turn Tu.

The anchor edge cell configuration in Figure 71E includes a loop formed in braid 31 at the turn, which may be formed by looping wire W around an inner or outer post of fixture 200.

Figure 71F provides another alternative anchor edge cell configuration having a figure-eight shape. Such a shape may be formed, for example, by wrapping wire W about an inner post 202 and an aligned outer post 204 in a figure-eight fashion, and then heat setting the wire in the resultant shape.

In Figure 71G, the edge cells of braid 31 include a heart-shaped configuration, which may be formed by wrapping the wire about an aligned inner and outer post of fixture 200 in the desired manner.

In Figure 71H, the edge cells of braid 31 have an asymmetric loop at turn Tu. The asymmetric loop will affect twisting of braid 31 during expansion and collapse of the braid, in addition to affecting stress concentration.

In Figure 71I, the anchor edge cells have a double-looped turn configuration, e.g. via wrapping about two adjacent inner or outer posts of fixture 200. Additional loops may also be employed. The double loop turn feature may be formed with a smooth transition between the loops, as in Figure 93I, or may be heat set with a more discontinuous shape, as in Figure 71J.

Figure 71K illusfrates that the edge cells of braid 31 may have multiple different configurations about the anchor's circumference. For example, the anchor edge cells shown in Figure 71K have extended length cells as in Figure 71B disposed adjacent to standard size edge cells, as in Figure 71A.

The anchor edge cells of Figure 71L have an extended turn configuration having an extended loop.

The anchor edge cells shown in Figure 71M have an alternative extended configuration with a specified heat set profile. Finally, the anchor edge cells shown in Figure 71N that overlap or are interwoven to be coupled to one another.

In preferred embodiments, the edge cells may be wrapped using wire, string, or sutures, at a location where the wire overlaps after an end turn as is illustrated in Figure 71O. This tied-end turn feature prevents cells from interlocking with each other during deployment.

The edge cell configuration of Figure 71 may be heat set independently of the rest of the braid. The anchor edge cell configurations of Figures 71 are provided only for the sake of illustration and should in no way be construed as limiting. Additional turn features within the scope of the present invention will apparent to those of skill in the art in view of Figures 71. Furthermore, combinations of any such turn features may be provided to achieve desired characteristics of anchor braid 30.

Referring now to Figures 72A-E, additional configurations for reducing stress concentration and/or circumferential stiffness of anchor braid 30 are illustrated. Such configurations can be used independently or in conjunction with other configurations disclosed herein. Such configurations are preferably used at the anchor's edges to locally reduce the cross-sectional area of substantially all cells or all cells in the anchor braid's edge (e.g., proximal and/or distal). As seen in Figures 72A and 72B, turns Tu in wire W typically may have a substantially continuous (e.g., round) cross-sectional profile. As seen in Figure 72C, modifying the edge cell configuration by locally reducing the thickness or cross-sectional area of wire W at turn(s) Tu will reduce stress concentration within the wire at the turns and facilitate collapse and/or expansion of anchor braid 30 from the delivery to the deployed configurations. Furthermore, it is expected that such localized reduction in thickness or cross-sectional area will reduce a risk of kinking, fatigue or other failure at turns Tu.

Localized reduction may be achieved via a localized etching and/or electropolishing process. Alternatively or additionally, localized grinding of the turns may be utilized. Additional processing techniques will be apparent to those of skill in the art. As seen in Figures 72D-72E, wire W may, for example, comprise an oval or rectangular cross-sectional profile, respectively, after localized reduction. The wire alternatively may comprise a round profile of reduced cross-sectional area (not shown). Additional profiles will be apparent. Localized reduction can take place at any time (e.g., before or after a braid is woven). Preferably, localized reduction occurs after weaving. However, in some embodiments, a wire of a given length may be etched or ground at preset segments and subsequently woven.

Referring now to Figures 73A- J, instead of terminating the beginning and end of wire W of braid 31 at an overlap within the braid, as discussed previously, the two ends of the wire may be terminated at the anchor's edge. Likewise, when braid 31 is fabricated from multiple wires W, the wires (or a subset of the wires) optionally may be joined together or terminated at turn(s) of the braid. In Figure 73A, wire termination T at the ends of wirc(s) W comprises a hinged termination with hinge post 38. In Figure 73B termination T comprises a clipped or crimped termination with end cap 39. In Figure 73C, cap 39 is wrapped about the ends of wire W to form wrapped termination T.

In Figure 73D, cap 39 is placed over the wire ends, which are then bent to provide a swivel termination. In Figure 73E, the wire ends are potted within cap 39 at termination T. In Figure 73F, cap 39 is swaged about the wire ends. In Figure 73G, the wire ends are welded or glued together.In Figure 73G, the wire ends are spot welded together. Alternatively, the wire ends may be braised to form termination T, as in Figure 73H. As yet another alternative, cap 39 may be placed about the wire ends, and kinks K may be formed in wire W to provide the ends of the wire with an 'over-center' bias that maintains termination T, e.g., swivel termination T. Additional terminations will be apparent to those of skill in the art.

The anchors described herein can be, for example, radially symmetrical, bilaterally symmetrical, or asymmetrical. A radially symmetrical anchor is one for which symmetry exists across any diameter. A bilaterally symmetrical anchor is one for which symmetry exists across a finite number if diameters). An asymmetrical anchor is one for which there exists no diameter across which a symmetry may be found. Figure 28B illustrates one embodiment of a radially symmetrical anchor. Figure 74A illustrates one embodiment of a bilaterally symmetrical anchor. Figure 74B illusfrates two embodiments (side and top views) of asymmetrical anchors. The benefits of bilaterally symmetrical an asymmetrical anchors is their ability to avoid interfering with anatomical features, such as, for example the coronary ostial and/or mitral valve. Thus, in preferred embodiments, a braided anchor includes a region adapted to prevent expansion of the anchor into the mitral valve, as is illustrated in Figure 74A.

In preferred embodiments, the anchor includes a leaflet engagement element and/or a seal inverting element situated on its proximal end. The leaflet engagement element is adapted for engaging the native leaflets of the patient's heart, or more preferably the proximal edge and/or the commissural attachments of the native leaflets. The leaflet engagement v element need not extend all the way into the pocket or the distal end of the native leaflet. Preferred embodiments of the apparatus herein are depicted in Figures 32-34, 49, 50, 93 and 98-109, which are discussed in more detail below.

Figure 75 provides a detail view of a front side region of anchor braid 30 with closed end turns Tu. Anchor braid 30 includes various cells, some having an end turn (Tu). End turns can serve various functions. For example, end turns can be configured to reduce the sheathing force, to reduce stress within the braid during delivery and deployment, to prevent distal migration during expansion of the anchor, and/or to positively register the anchor against the native valve during deployment. In preferred embodiments, an end turn feature functions to prevent distal migration and to register the anchor by engaging the native leaflets. In preferred embodiments, the proximal end of an anchor comprises embodiments (Tu).

Figures 71A-71N provide multiple examples of edge cells having end turn feature. The end turn features disclosed and others known in the art may be used as leaflet engagement elements to engage the native heart leaflets with the anchor. The leaflet engagement elements are preferably integral with the anchor, or more preferably part of a braided anchor. The end turn features can occur at the proximal end, the distal end, or both proximal and distal ends of the anchor. For details re the end turn features, it is referred to the description of Figures 71A-N.

The anchor and any of its features may be heat set at different configurations. For example, the anchor may be heat set ay its "at rest" configuration such that upon unsheathing it expands radially. The end turn features/leaflet engagement elements may be heat set at a different "at rest" configuration than the rest of the anchor. In preferred embodiment, end turn features are heat set to "flower" and then "evert" upon unsheathing.

The end turn features of Figures 71 are provided only for the sake of illustration and should in no way be construed as limiting. Additional turn features within the scope of the present invention will apparent to those of skill in the art in view of Figures 71. Furthermore, combinations of any such end turn features may be provided to achieve the desired characteristics of anchor 30. Additional configurations for reducing and/or circumferential stiffness of an anchor braid and/or leaflet engagement elements are illustrated in Figure 72.

With reference now to Figures 76A-F, a method of endovascularly replacing a patient's diseased aortic valve is provided. The method involves endovascularly delivering an anchor/valve apparatus and properly positioning such apparatus via positive registration with the patient's native valve leaflets. Registration with the native valve leaflet preferably occurs using the leaflet engagement elements.

In Figure 76A, modified delivery system 100' delivers apparatus 10 to diseased aortic valve AV within sheath 110. Apparatus 10 is delivered in a collapsed delivery configuration.

As seen in Figures 76B and 76C, apparatus 10 is deployed from lumen 112 of sheath 110, for example, under fluoroscopic guidance. Sheath 110 includes at its distal end leaflet engagement elements 120. Upon deployment, anchor 30 of apparatus 10 dynamically self-expands to a partially deployed configuration. This causes elements 60 to also dynamically expand, as well as membrane filter (or braid) 61 A and leaflet engagement elements 120. As when deployed via delivery system 100, deployment of apparatus 10 via delivery system 100' is fully reversible until locks 40 have been actuated.

Thus, delivery system 100' comprises leaflet engagement element 120, which preferably self-expands along with anchor 30. In preferred embodiments, the distal end of leaflet engagement elements 120 expands a greater radial distance than anchor 30. Moreover, engagement elements 120 maybe disposed between elements 60 of delivery system 100' and lip region 32 of anchor 30. However, leaflet engagement elements 120 may also be disposed on the proximal end of an anchor (as is illustrated in Figure 77). Leaflet engagement elements 120 releasably engage the anchor. As seen in Figure 76C, the leaflet engagement elements 120 are initially deployed proximal of the patient's native valve leaflets L. Apparatus 10 and element 120 then may be advanced/dynamically repositioned until engagement element positively registers against the leaflets, thereby ensuring proper positioning of apparatus 10. The leaflet engagement element engages with the proximal edges of the native valve leaflets and/or the commissural attachments. The leaflet engagement element need not extend all the way to the distal edge of the native leaflets (the leaflet pockets). In preferred embodiments, a leaflet engagement element length is less than about 20 mm, more preferably less than about 15 mm, or more preferably less than about 10 mm. Once leaflet engagement element 120 is registered against the native valve leaflets and/or commissural attachments, apparatus 10 deploys substantially distal to the coronary ostia of the heart.

In any of the embodiments herein, delivery system 100' can include filter structure 61 A (e.g., filter membrane or braid) as part of push elements 60 to act as an embolic protection element. Emboli can be generated during manipulation and placement of anchor from either diseased native leaflet or surrounding aortic tissue and can cause blockage. Arrows 61B in Figure 76C show blood flow through filter structure 61A where blood is allowed to flow but emboli is frapped in the delivery system and removed with it at the end of the procedure.

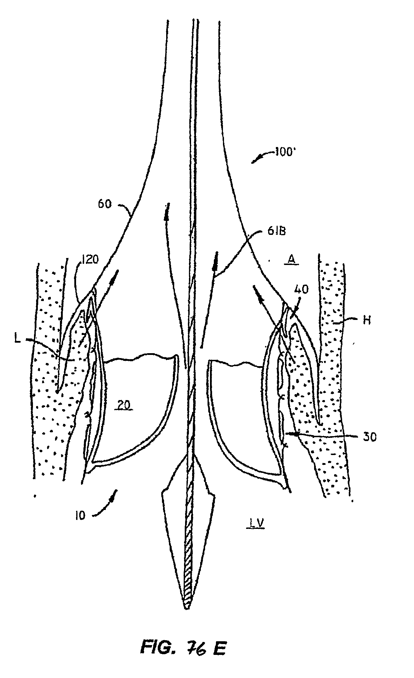

Active foreshortening may be imposed upon anchor 30 while element 120 is disposed proximal of the leaflets, as is illustrated in Figure 76D. Active foreshortening can be accomplished by actuating distal anchor actuation elements (e.g., elements 50) and/or proximal anchor actuation elements (e.g., elements 60). Upon positive registration of element 120 against leaflets L, element 120 precludes further distal migration of apparatus 10 during additional foreshortening, thereby reducing a risk of improperly positioning the apparatus. Figure 76E details engagement of element 120 against the native leaflets.

As seen in Figure 76F, once apparatus 10 is fully deployed, anchor 30 may be locked (reversibly or irreversibly). Subsequently, structure 61A leaflet engagement, elements 120, elements 50 and/or elements 60 may be decoupled from the apparatus, and delivery system 100' may be removed from the patient, thereby completing the procedure.

Figure 77 illustrates an alternative embodiment of the apparatus of Figures 76A-F described above, wherein leaflet engagement elements 120 are coupled to anchor 30 of apparatus 10' rather than to delivery system 100. In the embodiment illustrated in Figure 77, leaflet engagement elements 120 remain implanted near the patient's native heart valve after the deployment of apparatus 10' and removal of delivery system 100. Leaflets L may be sandwiched between the proximal region of anchor 30 and leaflet engagement element 120 in the fully deployed configuration. In this manner, element 120 positively registers apparatus 10' relative to the leaflets L and precludes distal migration of the apparatus over time.

Figures 78A-78C illustrate another embodiment for endovascularly delivering an apparatus of the present invention. In Figure 78A, a catheter 600 is delivered percutaneously in a retrograde fashion to the aortic valve. The catheter passes through the native aortic valve before an operator actuates the unseathing of the anchor/valve apparatus. As the sheathing catheter is pulled proximally out of the native valve, anchor 30 and replacement valve 20 become unsheathed. Immediately the portion of the unsheathed anchor 30 dynamically self-expands to its "at rest" position, and replacement valve 20 within the anchor regains an uncollapsed structure, allowing it to begin to function. In preferred embodiments in its "at rest" position, anchor 30 presses against the native leaflets limiting blood from flowing in between the anchor and leaflet. Also, in preferred embodiments, anchor 30 portions relatively adjacent to the valve is externally covered by a seal 60, more preferably the entire exterior contour of anchor 30 excluding the leaflet engagement elements is externally covered by a seal, or more preferably the entire contour of anchor 30 including the external face of the leaflet engagement elements is externally covered by a seal. A seal can be composed of any material that prevents or limits the flow of blood through the anchor. In preferred embodiments, a seal is composed of a thin, elastic polymer or any other type of fabric. The seal can be attached by any means known in the art to the anchor and, in some embodiments, to the distal end of the valve. In preferred embodiments, a seal is attached to the anchor by suturing.

In Figure 78B, as the catheter is further pulled proximally, the proximal end of anchor 30 and fingers 50 are unsheathed. In this embodiment, it is possible to visualize that the seal covers the entire contour of the anchor including the external face of the leaflet engagement element 70. As soon as the proximal end of the anchor is exposed, it also dynamically expands. Furthermore, when fingers 50 become exposed, replacement valve 20 begins to function permitting blood to flow through replacement valve 20, between fingers 50, and around the catheter 600. This also permits blood to flow into the coronary ostias. In other embodiments where the seal does not cover the proximal end of the anchor, the replacement valve can begin to function as soon as the unsealed portion of the anchor is unsheathed. This causes the leaflet engagement elements 70 to radially expand to their heat set position and engage with the native heart leaflets.

Next, Figure 78C, as the apparatus is actively foreshortened using proximal (e.g., fingers) and/or distal actuators (e.g., elements 55), the leaflet engagement elements positively register with the native valve leaflets. Foreshortening can cause seal 60 to bunch up and create pleats. These pleats can then fill pockets thereby improving the paravalvular seal. In preferred embodiments, wherein the leaflet engagement elements are covered with a seal, at least a portion of the seal is also positioned between the native valve leaflets and the aortic wall. Once the anchor is fully compressed within the aortic valve, the anchor is locked, the fingers and post mandrels are disengaged, and the seal is adapted to further limit blood flow around the replacement valve. The catheter is subsequently'withdrawn, leaving behind valve 20, seal 60 and anchor 30. When fully deployed, the anchor is substantially distal to the coronary ostia of the patient such that it will not interfere with blood flow through the ostia.

Figures 89A-79B illustrate an embodiment wherein only a distal portion anchor 30 is covered by seal 60 and wherein anchor 30 is only partially deployed since the blood can escape through the proximal end of the anchor braid. As anchor 30 in this embodiment is unsheathed, it presses against the native valve leaflets. At this point replacement valve 20 is functional even though anchor 30 is not fully deployed since blood can escape through the proximal end of the anchor braid. This allows blood to flow through replacement valve 20 and out of holes in the distal end of anchor 30 during systole (Figure 79A) while preventing backflow during diastole (Figure 79B).

Figures 80A-80B illustrate a similar embodiment wherein seal 60 around anchor 30 surrounds the entire contour of anchor 30. In this embodiment, valve 20 does not become functional until both anchor 30 and a portion of fingers 50 are unsheathed. As soon as a portion of fingers 50 is unsheathed, replacement valve 20 is fully functional. This allows blood to flow through replacement valve 20 and anchor 30, out of fingers 50, and around catheter 60 into the aorta and coronary ostias during systole. Similarly, during diastole, replacement valve 20 closes preventing blood backflow from entering the chamber.

In any of the embodiments herein the anchor is preferably a self-expanding anchor braid. Anchor braid of the present invention can be made from one or more wires, more preferably 2-20 wires, more preferably 3-15 wires, or more preferably 4-10 wires. Moreover, the density of the braid can be modified by various forms of weave used.

## Claims

1. Apparatus (10, 10', 10", 450) for replacing a native aortic valve, the apparatus (10, 10', 10", 450) comprising:
an anchor (30, 30', 300, 350, 470) comprising an expandable braid with closed ends, the anchor (30, 30', 300, 350, 470) being adapted to be endovascularly delivered and secured at an anchor site within the native aortic valve, and
a replacement valve (20, 354, 460) configured to be secured within the anchor (30, 30', 300, 350, 470),
wherein the anchor (30, 30', 300, 350, 470) comprises a proximal deployment system interface at a proximal end of the anchor (30, 30', 300, 350, 470), the proximal deployment system interface being adapted to permit a deployment system to apply a distally directed force on the proximal end of the anchor (30, 30', 300, 350, 470), and
wherein the proximal deployment system interface is further adapted to expand radially during application of a distally directed force on the proximal end of the anchor (30, 30', 300, 350, 470).

2. The apparatus of claim 1, wherein the proximal deployment system interface is further adapted to permit a deployment system to apply a distally directed force on the proximal end of the anchor (30, 30', 300, 350, 470) through a plurality of deployment system fingers (106a).

3. The apparatus of claim 1 or 2, wherein the anchor (30, 30', 300, 350, 470) comprises a distal deployment system interface disposed at a distal end of the anchor (30, 30', 300, 350, 470), the distal deployment system interface being adapted to permit a deployment system to apply a proximally directed force on the distal end of the anchor (30, 30', 300, 350, 470).

4. The apparatus of claim 3, wherein the distal deployment system interface is further adapted to expand radially during application of a proximally directed force on the distal end of the anchor (30, 30', 300, 350, 470).

5. The apparatus of claim 3 or 4, wherein the distal deployment system interface is further adapted to permit a deployment system to apply a proximally directed force on the distal end of the anchor (30, 30', 300, 350, 470) without passing any portion of a deployment system through a center opening of the replacement valve (20, 354, 460).

6. A deployment system (100, 100', 100", 410) and an apparatus (10, 10', 10", 450) for replacing a native aortic valve, the apparatus (10, 10', 10", 450) comprising:
an anchor (30, 30', 300, 350, 470) comprising an expandable braid with closed ends, the anchor (30, 30', 300, 350, 470) being adapted to be endovascularly delivered and secured at an anchor site within the native aortic valve, and
a replacement valve (20, 354, 460) configured to be secured within the anchor (30, 30', 300, 350, 470),
wherein the anchor (30, 30', 300, 350, 470) comprises a proximal deployment system interface at a proximal end of the anchor (30, 30', 300, 350, 470), the proximal deployment system interface being adapted to permit the deployment system to apply a distally directed force on the proximal end of the anchor (30, 30', 300, 350, 470), and wherein the proximal deployment system interface is further adapted to expand radially during application of a distally directed force on the proximal end of the anchor (30, 30', 300, 350, 470).

7. The deployment system and apparatus of claim 6, wherein the proximal deployment system interface is further adapted to permit the deployment system to apply a distally directed force on the proximal end of the anchor (30, 30', 300, 350, 470) through a plurality of deployment system fingers (106a).

8. The deployment system and apparatus of claim 6 or 7, wherein the anchor (30, 30', 300, 350, 470) comprises a distal deployment system interface disposed at a distal end of the anchor (30, 30', 300, 350, 470), the distal deployment system interface being adapted to permit the deployment system to apply a proximally directed force on the distal end of the anchor (30, 30', 300, 350, 470).

9. The deployment system and apparatus of claim 8, wherein the distal deployment system interface is further adapted to expand radially during application of a proximally directed force on the distal end of the anchor (30, 30', 300, 350, 470).

10. The deployment system and apparatus of claim 8 or 9, wherein the distal deployment system interface is further adapted to permit the deployment system to apply a proximally directed force on the distal end of the anchor (30, 30', 300, 350, 470) without passing any portion of a deployment system through a center opening of the replacement valve (20, 354, 460).

## Patentansprüche

1. Gerät (10, 10', 10", 450) zum Ersetzen einer nativen Aortenklappen, wobei das Gerät (10, 10', 10", 450) umfasst:
eine Befestigung (30, 30', 300, 350, 470), die ein expandierbares Geflecht mit geschlossenen Enden umfasst, wobei die Befestigung (30, 30', 300, 350, 470) angepasst ist, endovaskulär an eine Befestigungsstelle innerhalb der nativen Aortenklappe gebracht und dort befestigt zu werden, und
eine Ersatzklappe (20, 354, 460), ausgelegt, um innerhalb der Befestigung (30, 30', 300, 350, 470) befestigt zu werden,
wobei die Befestigung (30, 30', 300, 350, 470) eine proximale Einsetzsystem-Schnittstelle am proximalen Ende der Befestigung (30, 30', 300, 350, 470) umfasst, wobei die proximale Einsetzsystem-Schnittstelle angepasst ist, um einem Einsetzsystem zu ermöglichen eine distal gerichtete Kraft auf das proximale Ende der Befestigung (30, 30', 300, 350, 470) auszuüben, und
wobei die proximale Einsetzsystem-Schnittstelle außerdem angepasst ist, während des Ausübens einer distal gerichteten Kraft auf das proximale Ende der Befestigung (30, 30', 300, 350, 470) radial zu expandieren.

2. Gerät nach Anspruch 1, wobei die proximale Einsetzsystem-Schnittstelle außerdem angepasst ist, einem Einsetzsystem zu ermöglichen eine distal gerichtete Kraft auf das proximale Ende der Befestigung (30, 30', 300, 350, 470) über eine Mehrzahl an Einsetzsystem-Finger (106a) auszuüben.

3. Gerät nach Anspruch 1 oder 2, wobei die Befestigung (30, 30', 300, 350, 470) eine distale Einsetzsystem-Schnittstelle angeordnet an einem distalen Ende der Befestigung (30, 30', 300, 350, 470) umfasst, wobei die distale Einsetzsystem-Schnittstelle angepasst ist, um einem Einsetzsystem zu ermöglichen eine proximal gerichtete Kraft auf das distale Ende der Befestigung (30, 30', 300, 350, 470) auszuüben.

4. Gerät nach Anspruch 3, wobei die distale Einsetzsystem-Schnittstelle außerdem angepasst ist, während des Ausübens einer proximal gerichteten Kraft auf das distale Ende der Befestigung (30, 30', 300, 350, 470) radial zu expandieren.

5. Gerät nach Anspruch 3 oder 4, wobei die distale Einsetzsystem-Schnittstelle außerdem angepasst ist, einem Einsetzsystem zu ermöglichen eine proximal gerichtete Kraft auf das distale Ende der Befestigung (30, 30', 300, 350, 470) auszuüben, ohne dabei irgendein Teil des Einsetzsystems durch eine mittige Öffnung der Ersatzklappe (20, 354, 460) zu führen.

6. Einsetzsystem (100, 100', 100", 410) und Gerät (10, 10', 10", 450) zum Ersetzen einer nativen Aortenklappe, wobei das Gerät (10, 10', 10", 450) umfasst:
eine Befestigung (30, 30', 300, 350, 470), die ein expandierbares Geflecht mit geschlossenen Enden umfasst, wobei die Befestigung (30, 30', 300, 350, 470) angepasst ist, endovaskulär an eine Befestigungsstelle innerhalb der nativen Aortenklappe gebracht und dort befestigt zu werden, und
eine Ersatzklappe (20, 354, 460), ausgelegt, um innerhalb der Befestigung (30, 30', 300, 350, 470) befestigt zu werden,
wobei die Befestigung (30, 30', 300, 350, 470) eine proximale Einsetzsystem-Schnittstelle am proximalen Ende der Befestigung (30, 30', 300, 350, 470) umfasst, wobei die proximale Einsetzsystem-Schnittstelle angepasst ist, um einem Einsetzsystem zu ermöglichen eine distal gerichtete Kraft auf das proximale Ende der Befestigung (30, 30', 300, 350, 470) auszuüben, und wobei die proximale Einsetzsystem-Schnittstelle außerdem angepasst ist, während des Ausübens einer distal gerichteten Kraft auf das proximale Ende der Befestigung (30, 30', 300, 350, 470) radial zu expandieren.

7. Einsetzsystem und Gerät nach Anspruch 6, wobei die proximale Einsetzsystem-Schnittstelle außerdem angepasst ist, dem Einsetzsystem zu ermöglichen eine distal gerichtete Kraft auf das proximale Ende der Befestigung (30, 30', 300, 350, 470) über eine Mehrzahl an Einsetzsystem-Finger (106a) auszuüben.

8. Einsetzsystem und Gerät nach Anspruch 6 oder 7, wobei die Befestigung (30, 30', 300, 350, 470) eine distale Einsetzsystem-Schnittstelle angeordnet an einem distalen Ende der Befestigung (30, 30', 300, 350, 470) umfasst, wobei die distale Einsetzsystem-Schnittstelle angepasst ist, um einem Einsetzsystem zu ermöglichen eine proximal gerichtete Kraft auf das distale Ende der Befestigung (30, 30', 300, 350, 470) auszuüben.

9. Einsetzsystem und Gerät nach Anspruch 8, wobei die distale Einsetzsystem-Schnittstelle außerdem angepasst ist, während des Ausübens einer proximal gerichteten Kraft auf das distale Ende der Befestigung (30, 30', 300, 350, 470) radial zu expandieren.

10. Einsetzsystem und Gerät nach Anspruch 8 oder 9, wobei die distale Einsetzsystem-Schnittstelle außerdem angepasst ist, einem Einsetzsystem zu ermöglichen eine proximal gerichtete Kraft auf das distale Ende der Befestigung (30, 30', 300, 350, 470) auszuüben, ohne dabei irgendein Teil des Einsetzsystems durch eine mittige Öffnung der Ersatzklappe (20, 354, 460) zu führen.

## Revendications

1. Dispositif (10, 10', 10", 450) pour remplacer une valvule aortique native, le dispositif (10, 10', 10", 450) comprenant:
un ancrage (30, 30', 300, 350, 470) comprenant une tresse expansible présentant des extrémités fermées, l'ancrage (30, 30', 300, 350, 470) étant adapté pour être délivré et fixé de façon endovasculaire à un site d'ancrage à l'intérieur de la valvule aortique native, et
une valvule de remplacement (20, 354, 460) configurée de manière à être fixée à l'intérieur de l'ancrage (30, 30', 300, 350, 470),
dans lequel l'ancrage (30, 30', 300, 350, 470) comprend une interface de système de déploiement proximale à une extrémité proximale de l'ancrage (30, 30', 300, 350, 470), l'interface de système de déploiement proximale étant adaptée pour permettre à un système de déploiement d'appliquer une force orientée de façon distale sur l'extrémité proximale de l'ancrage (30, 30', 300, 350, 470), et
dans lequel l'interface de système de déploiement proximale est en outre adaptée pour s'expanser radialement pendant l'application d'une force orientée de façon distale sur l'extrémité proximale de l'ancrage (30, 30', 300, 350, 470).

2. Dispositif selon la revendication 1, dans lequel l'interface de système de déploiement proximale est en outre adaptée pour permettre à un système de déploiement d'appliquer une force orientée de façon distale sur l'extrémité proximale de l'ancrage (30, 30', 300, 350, 470) par l'intermédiaire d'une pluralité de doigts de système de déploiement (106a).

3. Dispositif selon la revendication 1 ou 2, dans lequel l'ancrage (30, 30', 300, 350, 470) présente une interface de système de déploiement distale qui est disposée à une extrémité distale de l'ancrage (30, 30', 300, 350, 470), l'interface de système de déploiement distale étant adaptée pour permettre à un système de déploiement d'appliquer une force orientée de façon proximale sur l'extrémité distale de l'ancrage (30, 30', 300, 350, 470).

4. Dispositif selon la revendication 3, dans lequel l'interface de système de déploiement distale est en outre adaptée pour s'expanser radialement pendant l'application d'une force orientée de façon proximale sur l'extrémité distale de l'ancrage (30, 30', 300, 350, 470).

5. Dispositif selon la revendication 3 ou 4, dans lequel l'interface de système de déploiement distale est en outre adaptée pour permettre à un système de déploiement d'appliquer une force orientée de façon proximale sur l'extrémité distale de l'ancrage (30, 30', 300, 350, 470) en ne faisant passer aucune partie d'un système de déploiement à travers une ouverture centrale de la valvule de remplacement (20, 354, 460).

6. Système de déploiement (100, 100', 100", 410) et dispositif (10, 10', 10", 450) pour remplacer une valvule aortique native, le dispositif (10, 10', 10", 450) comprenant:
un ancrage (30, 30', 300, 350, 470) comprenant une tresse expansible présentant des extrémités fermées, l'ancrage (30, 30', 300, 350, 470) étant adapté pour être délivré et fixé de façon endovasculaire à un site d'ancrage à l'intérieur de la valvule aortique native, et
une valvule de remplacement (20, 354, 460) configurée de manière à être fixée à l'intérieur de l'ancrage (30, 30', 300, 350, 470),
dans lequel l'ancrage (30, 30', 300, 350, 470) comprend une interface de système de déploiement proximale à une extrémité proximale de l'ancrage (30, 30', 300, 350, 470), l'interface de système de déploiement proximale étant adaptée pour permettre au système de déploiement d'appliquer une force orientée de façon distale sur l'extrémité proximale de l'ancrage (30, 30', 300, 350, 470), et
dans lequel l'interface de système de déploiement proximale est en outre adaptée pour s'expanser radialement pendant l'application d'une force orientée de façon distale sur l'extrémité proximale de l'ancrage (30, 30', 300, 350, 470).

7. Système de déploiement et dispositif selon la revendication 6, dans lesquels l'interface de système de déploiement proximale est en outre adaptée pour permettre au système de déploiement d'appliquer une force orientée de façon distale sur l'extrémité proximale de l'ancrage (30, 30', 300, 350, 470) par l'intermédiaire d'une pluralité de doigts de système de déploiement (106a).

8. Système de déploiement et dispositif selon la revendication 6 ou 7, dans lesquels l'ancrage (30, 30', 300, 350, 470) présente une interface de système de déploiement distale qui est disposée à une extrémité distale de l'ancrage (30, 30', 300, 350, 470), l'interface de système de déploiement distale étant adaptée pour permettre au système de déploiement d'appliquer une force orientée de façon proximale sur l'extrémité distale de l'ancrage (30, 30', 300, 350, 470).

9. Système de déploiement et dispositif selon la revendication 8, dans lesquels l'interface de système de déploiement distale est en outre adaptée pour s'expanser radialement pendant l'application d'une force orientée de façon proximale sur l'extrémité distale de l'ancrage (30, 30', 300, 350, 470).

10. Système de déploiement et dispositif selon la revendication 8 ou 9, dans lesquels l'interface de système de déploiement distale est en outre adaptée pour permettre au système de déploiement d'appliquer une force orientée de façon proximale sur l'extrémité distale de l'ancrage (30, 30', 300, 350, 470) en ne faisant passer aucune partie d'un système de déploiement à travers une ouverture centrale de la valvule de remplacement (20, 354, 460).
